# EUROPEAN PATENT SPECIFICATION

(11) **EP 2 814 972 B1**
(45) Date of publication and mention of the grant of the patent: **22.04.2020**
(21) Application number: 12869541.8
(22) Date of filing: 24.02.2012
(51) Int. Cl.: C12P 7/64, C12N 9/10, C12N 9/16

(54) **ENHANCED PRODUCTION OF FATTY ACIDS AND FATTY ACID DERIVATIVES BY RECOMBINANT MICROORGANISMS**
ERHÖHTE PRODUKTION VON FETTSÄUREN UND FETTSÄUREDERIVATEN DURCH REKOMBINANTE MIKROORGANISMEN
PRODUCTION AMÉLIORÉE D'ACIDES GRAS ET DE DÉRIVÉS D'ACIDES GRAS PAR DES MICRO-ORGANISMES RECOMBINÉS

(43) Date of publication of application: 24.12.2014
(73) Proprietor: ExxonMobil Research and Engineering Company, Annandale, NJ 08801-0900 (US)
(72) Inventor: SESHADRI, Rekha, San Diego, CA 92119 (US); KIT, Jennie, Lau, Encinitas, CA 92024 (US); BAUMAN, Nicholas, San Diego, CA 92103 (US); BROWN, Robert, Christopher, San Diego, CA 92117 (US)
(74) Representative: Abel & Imray
(86) International application number: PCT/US2012/026566
(87) International publication number: WO 2013/126076

(56) References cited:
- WO-A1-95/27791
- US-A1- 2011 250 659
- US-B2- 7 537 920
- US-B2- 8 048 654
- WIBERG EVA ET AL: "The distribution of caprylate, caprate and laurate in lipids from developing and mature seeds of transgenic Brassica napus L", PLANTA, SPRINGER VERLAG, DE, vol. 212, no. 1, 1 December 2000 (2000-12-01), pages 33-40, XP002420089, ISSN: 0032-0935, DOI: 10.1007/S004250000361
- SNYDER C L ET AL: "Acyltransferase action in the modification of seed oil biosynthesis", NEW BIOTECHNOLOGY, ELSEVIER BV, NL, vol. 26, no. 1-2, 1 October 2009 (2009-10-01), pages 11-16, XP026676318, ISSN: 1871-6784, DOI: 10.1016/J.NBT.2009.05.005 [retrieved on 2009-06-06]
- STAMATIA BELLOU ET AL: "Microalgal lipids biochemistry and biotechnological perspectives", BIOTECHNOLOGY ADVANCES, vol. 32, no. 8, 1 December 2014 (2014-12-01), pages 1476-1493, XP055174502, ISSN: 0734-9750, DOI: 10.1016/j.biotechadv.2014.10.003
- JILLIAN L. BLATTI ET AL: "Engineering fatty acid biosynthesis in microalgae for sustainable biodiesel", CURRENT OPINION IN CHEMICAL BIOLOGY, vol. 17, no. 3, 1 June 2013 (2013-06-01), pages 496-505, XP055229155, GB ISSN: 1367-5931, DOI: 10.1016/j.cbpa.2013.04.007
- WEIER ET AL.: 'Characterisation of acyltransferases from Synechocystis sp. PCC6803.' BIOCHEM. BIOPHYS. RES. COMM. vol. 334, 14 July 2005, pages 1127 - 1134, XP005001505
- OKAZAKI ET AL.: 'The significance of C16 fatty acids in the sn-2 positions of glycerolipids in the photosynthetic growth of Synechocystis sp. PCC6803.' PLANT PHYSIOLOGY vol. 141, June 2006, pages 546 - 556, XP055082219
- SHINDOU ET AL.: 'Acyl-CoA:Lysophospholipid Acyltransferases.' J. BIOL. CHEM. 2009 vol. 284, no. 1, 02 January 2009, pages 1 - 5, XP055082228
- KNUTZON DEBORAH S ET AL: "Lysophosphatidic acid acyltransferase from coconut endosperm mediates the insertion of laurate at the sn-2 position of triacylglycerols in lauric rapeseed oil and can increase total laurate levels", July 1999 (1999-07), PLANT PHYSIOLOGY (ROCKVILLE), VOL. 120, NR. 3, PAGE(S) 739-746 ISSN: 0032-0889

## Description

### REFERENCE TO A SEQUENCE LISTING

This application contains references to amino acid sequences and/or nucleic acid sequences which have been submitted concurrently herewith as the sequence listing text file "60930641_1.txt", file size 126 KiloBytes (KB), created on February 24, 2012.

### FIELD OF THE INVENTION

The present invention relates to the fields of bioengineering, metabolic biochemistry, and molecular biology. In particular, the invention relates to the production of fatty acid products in a recombinant microorganism or host cell engineered to reduce or minimize feedback inhibition of fatty acid synthesis enzymes and to methods of producing fatty acid products using such recombinant microorganisms or host cells.

### BACKGROUND OF THE INVENTION

Producing renewable sources for a variety of fuels and chemicals is of great importance to a world with increasing demand for such products. While petroleum is a product of decayed plant and other matter that has been incubated beneath the earth's surface for millions of years, some efforts today focus on the direct use of plants and other organisms to generate, *e.g.,* lipids, which can include fatty acids and derivatives thereof, for use in the fuel and chemical industries. Fatty acids are composed of long alkyl chains, similar to petroleum, and are a primary metabolite used by cells for both chemical and energy storage functions. Improving the scalability, controllability, and cost-effectiveness of producing fatty acids and fatty acid derivatives (collectively "fatty acid products") would be beneficial to the development of renewable energy and chemical sources.

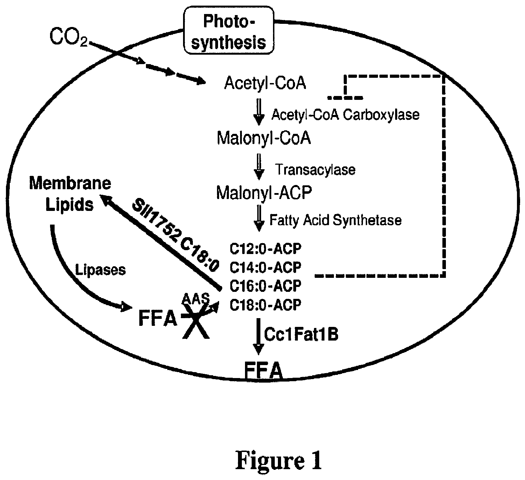

Fatty acid synthesis in a cell is a repeating cycle where malonyl-acyl carrier protein (malonyl-ACP) is condensed with a carbon substrate to form a growing chain of acyl-ACP. Fatty acid production by engineered cyanobacteria has been described in U.S. Appl. Pub. No. 2009/0298143, which discloses introducing a non-native gene encoding a fatty-acyl-ACP thioesterase to release fatty acids from ACP. However, as with many cellular processes, feedback inhibition by intermediates and/or various end-products may limit production of fatty acids. For example, feedback inhibition of key fatty acid synthesis enzymes, such as ACCase, FabH, and FabI, is mediated by medium- to long-chain acyl-ACPs. *See* Handke et al., Metabolic Eng. 13:28-37 (2011); Figure 1 (dashed line).

### SUMMARY OF THE INVENTION

The present invention describes additional genetic modifications for improving fatty acid product yields in a recombinant cyanobacterium engineered to express a non-native gene encoding a thioesterase. Such modifications include expression of an additional non-native gene encoding lysophosphatidic acid acyltransferase (LPAAT), which uses acyl-ACP or acyl-CoA substrates to acylate the sn-2 hydroxyl group of lysophosphatidic acid to form phosphatidic acid, a precursor of membrane lipids. The LPAAT preferably has a different acyl chain length substrate preference than the acyl chain length substrate preference of the thioesterase produced by expression of a non-native gene by the recombinant microorganism. Additional modifications of the host microorganism can optionally include attenuation of an acyl-ACP synthetase gene or an acyl-CoA synthetase gene, which participate in recycling and degradation, respectively, of fatty acids, and/or overexpression of one or more polypeptides having lipolytic activity (*e.g.*, lipases) that can release fatty acids from lipids (*see e.g.,* Figure 1).

In one aspect the invention provides a recombinant cyanobacterium transformed with a non-native nucleic acid sequence encoding an acyl-ACP thioesterase derived from a higher plant operably linked to a heterologous promoter and a non-native nucleic acid sequence encoding a lysophosphatidic acid acyltransferase (LPAAT) derived from a cyanobacterium operably linked to a heterologous promoter, wherein the cyanobacterium produces at least one free fatty acid or at least one fatty acid derivative, and wherein the LPAAT has at least 85% identity to SEQ ID NO:2, and the thioesterase has at least 85% identity to SEQ ID NO:44. A fatty acid product can be, for example, a free fatty acid, a fatty aldehyde, a fatty alcohol, a fatty acid ester, a wax ester, an alkane, or an alkene.

The recombinant cyanobacterium that includes a non-native thioesterase gene further includes a non-native nucleic acid molecule that includes a sequence encoding an LPAAT, where the LPAAT-encoding sequence can be homologous (originating from the same species) or heterologous (originating from a different species) with respect to the host cyanobacterium. For example, a non-native LPAAT gene from the same or a different species, operably linked to a heterologous promoter, can be introduced into the host cyanobacterium, or alternatively, a homologous or heterologous LPAAT gene can be introduced into the host cyanobacterium such that the LPAAT gene recombines into the host genome and becomes operably linked to a promoter that is endogenous to the host. Further alternatively, a recombinant cyanobacterium that includes a non-native LPAAT gene can comprise an endogenous LPAAT gene residing in the genome of the host cyanobacterium operably linked to a heterologous promoter introduced into the cyanobacterium and integrated into the genome, for example, by homologous recombination. A non-native nucleic acid molecule that includes an LPAAT gene in a cyanobacterium provided herein is derived from a cyanobacterium.

The recombinant cyanobacterium comprises at least one non-native LPAAT gene and at least one non-native thioesterase gene that can cleave an acyl thioester substrate, in which the LPAAT and the thioesterase preferably have different acyl chain length substrate specificities. For example, the recombinant cyanobacterium can include a non-native nucleic acid molecule encoding an LPAAT that has a substrate preference for one or more long chain acyl substrates (*e.g.,* C16 and/or C18 acyl-ACP or acyl-CoA) and a non-native gene encoding a thioesterase that has a substrate preference for one or more medium chain acyl substrates (*e.g*., C8, C10, C12, and/or C14 acyl-ACP substrates). Alternatively, the recombinant cyanobacterium can include a non-native nucleic acid molecule encoding an LPAAT that has a substrate preference for one or more medium chain acyl substrates (*e.g*., C8, C10, C12, and/or C14 acyl-ACP substrates) and a non-native gene encoding a thioesterase that has a substrate preference for one or more long chain acyl substrates (*e.g*., C16 and/or C18 acyl-ACP or acyl-CoA). In another example, the recombinant cyanobacterium can include a non-native gene encoding an LPAAT that has a substrate preference for a C16 acyl substrate (*e.g*., C16 acyl-ACP and/or C16 acyl-CoA) and a non-native gene encoding a thioesterase that has a substrate preference for one or more of a C8, C10, C12, or C14 acyl substrate (*e.g*., C8, C10, C12, and/or C14 acyl-ACP). Alternatively, the recombinant cyanobacterium can include a non-native gene encoding an LPAAT that has a substrate preference for C18 acyl substrates (*e.g*., C18 acyl-ACP and/or C18 acyl-CoA) and a non-native gene encoding a thioesterase that has a substrate preference for one or more of a C8, C10, C12, C14, or C16 acyl substrate (*e.g*., C8, C10, C12, C14, and/or C16 acyl-ACP). In further non-limiting examples, a recombinant cyanobacterium can include a non-native gene encoding an LPAAT that has a substrate preference for a C18 acyl substrate and a non-native gene encoding a thioesterase that has a substrate preference for a C16 acyl substrate; a recombinant cyanobacterium can include a non-native gene encoding an LPAAT that has a substrate preference for a C18 acyl substrate and a non-native gene encoding a thioesterase gene that has a substrate preference for C14 and C16 acyl substrates; or a recombinant cyanobacterium can include a non-native gene encoding an LPAAT that has a substrate preference for a C18 acyl substrate and a non-native gene encoding a thioesterase that has a substrate preference for C12, C14, and C16 acyl substrates. In further examples, a cyanobacterium can include a non-native gene encoding an LPAAT that has a substrate preference for a C16 acyl substrate and a non-native gene encoding a thioesterase that has a substrate preference for a C14 acyl substrate; a cyanobacteium can include a non-native gene encoding an LPAAT that has a substrate preference for a C16 acyl substrate and a non-native gene encoding a thioesterase that has a substrate preference for a C12 acyl substrate, and a cyanobacterium can include a non-native gene encoding an LPAAT that has a substrate preference for a C16 acyl substrate and a non-native gene encoding a thioesterase that has a substrate preference for C12 and C14 acyl substrates.

A recombinant cyanobacterium includes a non-native nucleic acid molecule that has at least 85%, for example at least 90%, at least 95%, at least 96%, at least 97%, at least 98%, at least 99%, or about 100% identity to SEQ ID NO:2.

The heterologous promoter can be a regulatable promoter, and can optionally be an inducible promoter. For example, the promoter can be induced by light, temperature, or the addition or removal of a compound to the growth media, such as for example, a sugar or sugar analogue, a hormone, a salt, a metal, *etc.* In particular examples a nucleic acid sequence encoding a thioesterase gene expressed by recombinant cyanobacterium is operably linked to a heterologous promoter that can be, for example, regulatable, and can optionally be inducible. Additionally, an LPAAT-encoding nucleic acid sequence and a thioesterase-encoding nucleic acid sequence can be operably linked to different copies of the same promoter, or to different promoters regulated by the same or different conditions or compound(s). Alternatively, an LPAAT-encoding nucleic acid sequence and a thioesterase-encoding nucleic acid sequence can be operably linked to the same promoter, for example, the LPAAT-encoding nucleic acid sequence and the thioesterase-encoding sequence can be organized as an operon.

Additionally, a recombinant cyanobacterium can optionally further comprise an attenuated or disrupted acyl-ACP synthetase gene or acyl-CoA synthetase gene. For example, the endogenous acyl-ACP synthetase gene or acyl-CoA synthetase gene can be targeted by antisense RNA, one or more micro RNAs, an siRNA (*e.g*., generated by a shRNA construct), or one or more ribozymes, to reduce expression of the acyl-ACP synthetase gene or acyl-CoA synthetase gene. Alternatively, the endogenous acyl-ACP synthetase gene or acyl-CoA synthetase gene can be interrupted, truncated, or deleted, for example, by insertional mutagenesis, meganuclease genome modification, and/or homologous recombination.

The recombinant cyanobacterium that includes a non-native LPAAT gene and a non-native thioesterase gene can further optionally include a non-native gene encoding a polypeptide having lipolytic activity, which can be, for example, a lipase, an esterase, a cutinase, or an amidase. Additionally or alternatively, the recombinant cyanobacterium can have an attenuated gene that encodes an acyl-ACP synthetase, an acyl-CoA synthetase, or an acyl-CoA oxidase.

Further additionally or alternatively, a recombinant cyanobacterium that includes a non-native LPAAT gene and a non-native thioesterase gene can further optionally include one or more non-native genes that encode enzymes for producing fatty acid derivatives such as fatty aldehydes, fatty alcohols, fatty acid esters, wax esters, alkanes, and alkenes. Non-limiting examples of such enzymes are aldehyde-forming acyl-ACP reductases, aldehyde-forming acyl-CoA reductases, carboxylic acid reductases, aldehyde-forming fatty acid reductases, alcohol-forming acyl-ACP reductases, alcohol-forming acyl-CoA reductases, acyltransferases, wax synthases, fatty aldehyde decarbonylases, and fatty acid decarboxylases.

The recombinant cyanobacterium that includes a non-native LPAAT gene and a non-native thioesterase gene can produce at least 10% more of a fatty acid product that a cyanobacterium identical in all respects except that it lacks a non-native nucleic acid molecule that comprises a nucleic acid sequence encoding an LPAAT. For example, a recombinant cyanobacterium can produce at least 20%, at least 30%, at least 40%, at least 50%, at least 60%, at least 70%, at least 80%, at least 90%, at least 100%, at least 150%, at least 200%, at least 400%, at least 600%, at least 800%, or at least 1000% more than the amount of the fatty acid product produced by an otherwise identical cyanobacterium not including the non-native nucleic acid molecule that comprises a nucleic acid sequence encoding an LPAAT cultured under identical conditions.

The recombinant cyanobacterium can be a cyanobacterium, for example, of the genus *Agmenellum, Anabaena, Anabaenopsis, Anacystis, Aphanizomenon, Arthrospira, Asterocapsa, Borzia, Calothrix, Chamaesiphon, Chlorogloeopsis, Chroococcidiopsis, Chroococcus, Crinalium, Cyanobacterium, Cyanobium, Cyanocystis, Cyanospira, Cyanothece, Cylindrospermopsis, Cylindrospermum, Dactylococcopsis, Dermocarpella, Fischerella, Fremyella, Geitleria, Geitlerinema, Gloeobacter, Gloeocapsa, Gloeothece, Halospirulina, Iyengariella, Leptolyngbya, Limnothrix, Lyngbya, Microcoleus, Microcystis, Myxosarcina, Nodularia, Nostoc, Nostochopsis, Oscillatoria, Phormidium, Planktothrix, Pleurocapsa, Prochlorococcus, Prochloron, Prochlorothrix, Pseudanabaena, Rivularia, Schizothrix, Scytonema, Spirulina, Stanieria, Starria, Stigonema, Symploca, Synechococcus, Synechocystis, Thermosynechococcus, Tolypothrix, Trichodesmium, Tychonema* or *Xenococcus.*

Also provided herein are methods for producing a fatty acid product, where the method includes culturing any of the recombinant cyanobacterium of the invention under conditions in which at least one fatty acid product is produced. The at least one fatty acid product is one or more of a free fatty acid, a fatty aldehyde, a fatty alcohol, a fatty acid ester, a wax ester, or a hydrocarbon, and wherein the fatty acid product is secreted into the medium. In some examples, the recombinant cyanobacterium produces at least one C₈ to C₂₄ free fatty acid, such as at least one C₁₂ to C₁₈ free fatty acid, such as, for example, a C₁₂ to C₁₆ free fatty acid. Alternatively or in addition, the fatty acid or fatty acid derivative produced using the methods provided herein comprises at least one C₈ to C₂₄ fatty alcohol or fatty aldehyde, such as at least one C₁₂ to C₁₈ fatty alcohol or fatty aldehyde, such as a C₁₂ to C₁₆ fatty alcohol or fatty aldehyde; or at least one wax ester having an A chain of C₈ to C₂₄ or C₁₂ to C₁₈, or for example, an A chain of C₁₂ to C₁₆, and a B chain of C₈ to C₂₄ or C₁₂ to C₁₈, or for example, a B chain of C₁₂ to C_{16;} or at least one C₁₁ to C₂₃ alkane or alkene, such as a C₁₁ to C₁₅ alkane or alkene. The method can further include isolating a fatty acid product from the cyanobacterium, the culture medium, or a combination thereof. Additionally, the recombinant cyanobacterium may secrete at least a portion of the fatty acid product into the culture medium, and optionally all or a portion of the secreted product can be isolated from the culture medium.

In various methods, the amount of fatty acid product produced by the recombinant cyanobacterium can be at least 10% greater, at least 20% greater, at least 30% greater, at least 40% greater, at least 50% greater, at least 60% greater, at least 70% greater, at least 80% greater, at least 90% greater, or at least 100% greater than the amount produced by an otherwise identical cyanobacterium lacking the non-native nucleic sequence encoding an LPAAT that is cultured under the same conditions.

Further disclosed is a fatty acid product made using the methods of the invention. The fatty acid product can be, for example, a fatty acid, a fatty alcohol, a fatty aldehyde, an alkane, an alkene, a fatty acid ester, or a wax ester. The fatty acid product can have at least one carbon chain having a length of C₇ to C₂₄, and in some preferred embodiments, the fatty acid product includes one or more free fatty acids, fatty aldehydes, fatty alcohols, alkanes, or alkenes having C₁₂ to C₁₈ chain lengths, for example, chain lengths of C₁₂ to C₁₆, or one or more fatty acid esters having one or both of an A chain and a B chain of C₈ to C₂₄, or of C₁₂ to C₁₈, for example, C₁₂ to C₁₆. For example, a wax ester produced using the methods provided herein can have an A and a B chain of C₈ to C₂₄, or of C₁₂ to C₁₈, or in some examples of C₁₂ to C₁₆.

### BRIEF DESCRIPTION OF THE DRAWINGS

Figure 1 is a schematic depicting genetic modifications proposed to relieve feedback inhibition of fatty acid biosynthesis by reducing the pool of C18-ACP. Abbreviations are FFA: free fatty acid, AAS: acyl-ACP synthase, Cc1FatB1: *Cuphea carthagenensis* acyl-ACP thioesterase with a C12, C14, and C16 substrate preference, and Sll1752 C18:0: LPAAT of *Synechocystis* sp. 6803 with a C18 acyl substrate preference.
Figure 2 is a schematic representation of an example of an integration vector that includes a nucleic acid sequence encoding an LPAAT (sll1752).
Figure 3 is a schematic representation of an example of an integration vector that includes a nucleic acid sequence encoding an acyl-ACP thioesterase (Cc1FatB1).
Figure 4 is a schematic representation of an example of a vector for disrupting a gene encoding an acyl-ACP synthetase (slr1609).
Figure 5 is a graph depicting non-normalized total free fatty acid production (µg/mL) of *Synechocystis* sp. 6803 strains comprising (left to right) the Cc1FatB1 acyl-ACP thioesterase gene (Control 1A), the recombinant sll1752 gene encoding a C18:0 LPAAT (sll1752), an attenuated acyl-ACP synthetase gene (AASKO), followed by three separate clones (Patch 3, Patch 5, Patch 6) containing the Cc1FatB1 acyl-ACP thioesterase gene in combination with the inducible sll1752 gene (1A / sll1752), and finally the sll1752 LPAAT gene in combination with the attenuated acyl-ACP synthetase gene (AASKO).

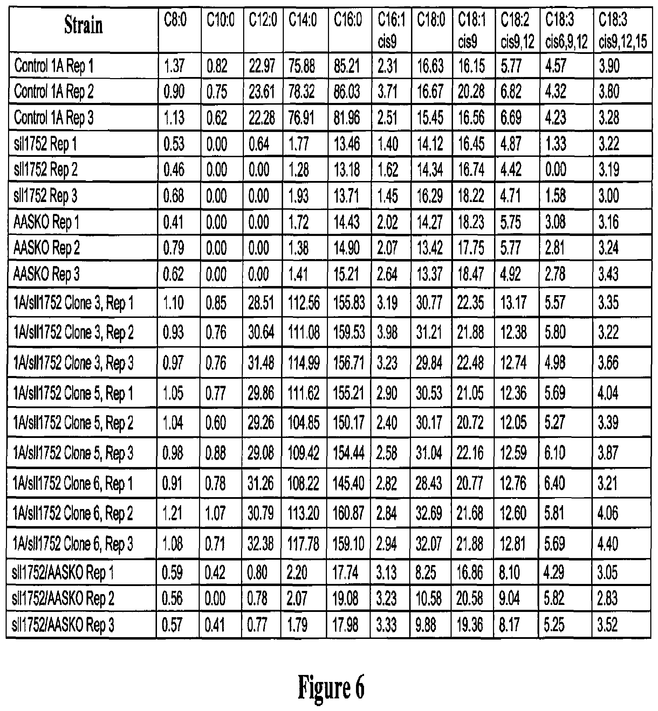

Figure 6 is a table providing the amounts (µg/mL) of free fatty acids of particular chain lengths and degree of saturation produced by *Synechocystis* sp. PCC 6803 strains comprising the Cc1FatB1 acyl-ACP thioesterase gene (Control 1A), the recombinant sll1752 gene encoding a C18:0 LPAAT (sll1752), an attenuated acyl-ACP synthetase gene (AASKO), three separate clones (Clone 3, Clone 5, Clone 6) containing the Cc1FatB1 acyl-ACP thioesterase gene as well as the recombinant sll1752 gene (1A / sll1752), and the recombinant sll1752 gene in combination with the attenuated acyl-ACP synthetase gene (sll1752 / AASKO).

### DETAILED DESCRIPTION OF THE INVENTION

### Definitions

Unless defined otherwise, all technical and scientific terms used herein have the same meaning as commonly understood by one of ordinary skill in the art to which this invention belongs. In case of conflict, the present application including the definitions will control. Unless otherwise required by context, singular terms shall include pluralities and plural terms shall include the singular.

Although methods and materials similar or equivalent to those described herein can be used in practice or testing of the present invention, suitable methods and materials are described below. The materials, methods and examples are illustrative only and are not intended to be limiting. Other features and advantages of the invention will be apparent from the detailed description and from the claims.

To facilitate an understanding of the present invention, a number of terms and phrases are defined below.

As used in the present disclosure and claims, the singular forms "a," "an," and "the" include plural forms unless the context clearly dictates otherwise.

Wherever embodiments are described herein with the language "comprising," otherwise analogous embodiments described in terms of "consisting of' and/or "consisting essentially of' are also provided.

The term "and/or" as used in a phrase such as "A and/or B" herein is intended to include "A and B", "A or B", "A", and "B".

The term "gene" is used broadly to refer to any segment of nucleic acid molecule (typically DNA, but optionally RNA) encoding a protein or expressed RNA. Thus, genes include sequences encoding expressed RNA (which can include polypeptide coding sequences). Genes may further comprise the regulatory sequences required for their expression. Genes can be obtained from a variety of sources, including cloning from a source of interest or synthesizing from known or predicted sequence information, and may include sequences designed to have desired parameters.

The term "nucleic acid" or "nucleic acid molecule" refers to, *e.g.,* DNA or RNA (*e.g.,* mRNA). The nucleic acid molecules can be double-stranded or single-stranded; single stranded RNA or DNA can be the coding (sense) strand or the non-coding (antisense) strand.

A nucleic acid molecule may be "derived from" an indicated source, which includes the isolation (in whole or in part) of a nucleic acid segment from an indicated source or the purification of a polypeptide from an indicated source. A nucleic acid molecule may also be derived from an indicated source by, for example, direct cloning, PCR amplification, or artificial synthesis from the indicated polynucleotide source or based on a sequence associated with the indicated polynucleotide source. Genes or nucleic acid molecules derived from a particular source or species also include genes or nucleic acid molecules having sequence modifications with respect to the source nucleic acid molecules. For example, a gene or nucleic acid molecule derived from a source (*e.g.,* a particular referenced gene) can incur one or more mutations with respect to the source gene or nucleic acid molecule that are unintended or that are deliberately introduced, and if one or more mutations, including substitutions, deletions, or insertions, are deliberately introduced the sequence alterations can be introduced by random or targeted mutation of cells or nucleic acids, by amplification or other molecular biology techniques, or by chemical synthesis. A gene or nucleic acid molecule that is derived from a referenced gene or nucleic acid molecule that encodes a functional RNA or polypeptide can encode a functional RNA or polypeptide having at least 70%, at least 75%, at least 80%, at least 85%, at least 90%, at least 95%, at least 96%, at least 97%, at least 98%, or at least 99% sequence identity with the referenced or source functional RNA or polypeptide, or to a functional fragment thereof. For example, a gene or nucleic acid molecule that is derived from a referenced gene or nucleic acid molecule that encodes a functional RNA or polypeptide can encode a functional RNA or polypeptide having at least 85%, at least 90%, at least 95%, at least 96%, at least 97%, at least 98%, or at least 99% sequence identity with the referenced or source functional RNA or polypeptide, or to a functional fragment thereof.

As used herein, an "isolated" nucleic acid or protein is removed from its natural milieu or the context in which the nucleic acid or protein exists in nature. For example, an isolated protein or nucleic acid molecule is removed from the cell or organism with which it is associated in its native or natural environment. An isolated nucleic acid or protein can be, in some instances, partially or substantially purified, but no particular level of purification is required for isolation. Thus, for example, an isolated nucleic acid molecule can be a nucleic acid sequence that has been excised from the chromosome, genome, or episome that it is integrated into in nature.

A "purified" nucleic acid molecule or nucleotide sequence, or protein or polypeptide sequence, is substantially free of cellular material and cellular components. The purified nucleic acid molecule or protein may be free of chemicals beyond buffer or solvent, for example. "Substantially free" is not intended to mean that other components beyond the novel nucleic acid molecules are undetectable. In some circumstances "substantially free" may mean that the nucleic acid molecule or nucleotide sequence is free of at least 95% (w/w) of cellular material and components.

The terms "naturally-occurring" and "wild-type" refer to a form found in nature. For example, a naturally occurring or wild-type nucleic acid molecule, nucleotide sequence or protein may be present in and isolated from a natural source, and is not intentionally modified by human manipulation.

As used herein "attenuated" means reduced in amount, degree, intensity, or strength. Attenuated gene expression may refer to a significantly reduced amount and/or rate of transcription of the gene in question, or of translation, folding, or assembly of the encoded protein. As nonlimiting examples, an attenuated gene may be a mutated or disrupted gene (*e.g.,* a gene disrupted by partial or total deletion, or insertional mutation) or having decreased expression due to alteration of gene regulatory sequences.

"Exogenous nucleic acid molecule" or "exogenous gene" refers to a nucleic acid molecule or gene that has been introduced ("transformed") into a cell. A transformed cell may be referred to as a recombinant cell, into which additional exogenous gene(s) may be introduced. A descendent of a cell transformed with a nucleic acid molecule is also referred to as "transformed" if it has inherited the exogenous nucleic acid molecule. The exogenous gene may be from a different species (and so "heterologous"), or from the same species (and so "homologous"), relative to the cell being transformed. An "endogenous" nucleic acid molecule, gene or protein is a native nucleic acid molecule, gene or protein as it occurs in, or is naturally produced by, the host.

The term "native" is used herein to refer to nucleic acid sequences or amino acid sequences as they naturally occur in the host. The term "non-native" is used herein to refer to nucleic acid sequences or amino acid sequences that do not occur naturally in the host. A nucleic acid sequence or amino acid sequence that has been removed from a host cell, subjected to laboratory manipulation, and introduced or reintroduced into a host cell is considered "non-native." Synthetic or partially synthetic genes introduced into a host cell are "non-native." Non-native genes further include genes endogenous to the host microorganism operably linked to one or more heterologous regulatory sequences that have been recombined into the host genome.

A "recombinant" or "engineered" nucleic acid molecule is a nucleic acid molecule that has been altered through human manipulation. As non-limiting examples, a recombinant nucleic acid molecule that: 1) has been synthesized or modified *in vitro,* for example, using chemical or enzymatic techniques (for example, by use of chemical nucleic acid synthesis, or by use of enzymes for the replication, polymerization, digestion (exonucleolytic or endonucleolytic), ligation, reverse transcription, transcription, base modification (including, e.g., methylation), integration or recombination (including homologous and site-specific recombination)) of nucleic acid molecules; 2) includes conjoined nucleotide sequences that are not conjoined in nature, 3) has been engineered using molecular cloning techniques such that it lacks one or more nucleotides with respect to the naturally occurring nucleic acid molecule sequence, and/or 4) has been manipulated using molecular cloning techniques such that it has one or more sequence changes or rearrangements with respect to the naturally occurring nucleic acid sequence. As non-limiting examples, a cDNA is a recombinant DNA molecule, as is any nucleic acid molecule that has been generated by in vitro polymerase reaction(s), or to which linkers have been attached, or that has been integrated into a vector, such as a cloning vector or expression vector.

The term "recombinant protein" as used herein refers to a protein produced by genetic engineering.

When applied to organisms, the term recombinant, engineered, or genetically engineered refers to organisms that have been manipulated by introduction of a heterologous or recombinant nucleic acid sequence into the organism, and includes gene knockouts, targeted mutations and gene replacement, promoter replacement, deletion, or insertion, as well as introduction of transgenes into the organism. Recombinant or genetically engineered organisms can also be organisms into which constructs for gene "knock down" have been introduced. Such constructs include, but are not limited to, RNAi, microRNA, shRNA, antisense, and ribozyme constructs. Also included are organisms whose genomes have been altered by the activity of meganucleases or zinc finger nucleases. The heterologous or recombinant nucleic acid molecule can be integrated into the recombinant/genetically engineered organism's genome or in other instances are not integrated into the recombinant/genetically engineered organism's genome. As used herein, "recombinant microorganism" or "recombinant host cell" includes progeny or derivatives of the recombinant microorganisms of the invention. Because certain modifications may occur in succeeding generations due to either mutation or environmental influences, such progeny or derivatives may not, in fact, be identical to the parent cell, but are still included within the scope of the term as used herein.

The term "promoter" refers to a nucleic acid sequence capable of binding RNA polymerase in a cell and initiating transcription of a downstream (3' direction) coding sequence. A promoter includes the minimum number of bases or elements necessary to initiate transcription at levels detectable above background. A promoter can include a transcription initiation site as well as protein binding domains (consensus sequences) responsible for the binding of RNA polymerase. Eukaryotic promoters often, but not always, contain "TATA" boxes and "CAT" boxes. Prokaryotic promoters may contain -10 and -35 prokaryotic promoter consensus sequences. A large number of promoters, including constitutive, inducible and repressible promoters, from a variety of different sources are well known in the art. Representative sources include for example, viral, mammalian, insect, plant, yeast, and bacterial cell types, and suitable promoters from these sources are readily available, or can be made synthetically, based on sequences publicly available on line or, for example, from depositories such as the ATCC as well as other commercial or individual sources. Promoters can be unidirectional (*i.e*., initiate transcription in one direction) or bi-directional (*i.e*., initiate transcription in both directions off of opposite strands). A promoter may be a constitutive promoter, a repressible promoter, or an inducible promoter. Non-limiting examples of promoters include, for example, the T7 promoter, the cytomegalovirus (CMV) promoter, the SV40 promoter, and the RSV promoter. Examples of inducible promoters include the lac promoter, the pBAD (araA) promoter, the Tet promoter (U.S. Pat. Nos. 5,464,758 and 5,814,618), and the Ecdysone promoter (No et al., Proc. Natl. Acad. Sci. (1996) 93 (8): 3346-3351).

The term "heterologous" when used in reference to a polynucleotide, a gene, a nucleic acid, a polypeptide, or an enzyme refers to a polynucleotide, gene, a nucleic acid, polypeptide, or an enzyme not naturally found in the host organism. When referring to a gene regulatory sequence or to an auxiliary nucleic acid sequence used for maintaining or manipulating a gene sequence (*e.g*. a 5' untranslated region, 3' untranslated region, poly A addition sequence, intron sequence, splice site, ribosome binding site, internal ribosome entry sequence, genome homology region, recombination site, *etc.*)*,* "heterologous" means that the regulatory sequence or auxiliary sequence is from a different source than the gene with which the regulatory or auxiliary nucleic acid sequence is juxtaposed in a construct, genome, chromosome or episome. Thus, a promoter operably linked to a gene to which it is not operably linked to in its natural state (*i.e.* in the genome of a non-genetically engineered organism) is referred to herein as a "heterologous promoter," even though the promoter may be derived from the same species (or, in some cases, the same organism) as the gene to which it is linked.

As used herein, the term "protein" or "polypeptide" is intended to encompass a singular "polypeptide" as well as plural "polypeptides," and refers to a molecule composed of monomers (amino acids) linearly linked by amide bonds (also known as peptide bonds). The term "polypeptide" refers to any chain or chains of two or more amino acids, and does not refer to a specific length of the product. Thus, peptides, dipeptides, tripeptides, oligopeptides, "protein," "amino acid chain," or any other term used to refer to a chain or chains of two or more amino acids, are included within the definition of "polypeptide," and the term "polypeptide" can be used instead of, or interchangeably with any of these terms.

As used herein, the terms "percent identity" or "homology" with respect to nucleic acid or polypeptide sequences are defined as the percentage of nucleotide or amino acid residues in the candidate sequence that are identical with the known polypeptides, after aligning the sequences for maximum percent identity and introducing gaps, if necessary, to achieve the maximum percent homology. N-terminal or C-terminal insertion or deletions shall not be construed as affecting homology, and internal deletions and/or insertions into the polypeptide sequence of less than about 30, less than about 20, or less than about 10 amino acid residues shall not be construed as affecting homology.

Homology or identity at the nucleotide or amino acid sequence level can be determined by BLAST (Basic Local Alignment Search Tool) analysis using the algorithm employed by the programs blastp, blastn, blastx, tblastn, and tblastx (Altschul (1997), Nucleic Acids Res. 25, 3389-3402, and Karlin (1990), Proc. Natl. Acad. Sci. USA 87, 2264-2268), which are tailored for sequence similarity searching. The approach used by the BLAST program is to first consider similar segments, with and without gaps, between a query sequence and a database sequence, then to evaluate the statistical significance of all matches that are identified, and finally to summarize only those matches which satisfy a preselected threshold of significance. For a discussion of basic issues in similarity searching of sequence databases, see Altschul (1994), Nature Genetics 6, 119-129. The search parameters for histogram, descriptions, alignments, expect (*i.e.,* the statistical significance threshold for reporting matches against database sequences), cutoff, matrix, and filter (low complexity) can be at the default settings. The default scoring matrix used by blastp, blastx, tblastn, and tblastx is the BLOSUM62 matrix (Henikoff (1992), Proc. Natl. Acad. Sci. USA 89, 10915-10919), recommended for query sequences over 85 in length (nucleotide bases or amino acids).

For blastn, designed for comparing nucleotide sequences, the scoring matrix is set by the ratios of M (*i.e.,* the reward score for a pair of matching residues) to N (*i.e.,* the penalty score for mismatching residues), wherein the default values for M and N can be +5 and -4, respectively. Four blastn parameters can be adjusted as follows: Q=10 (gap creation penalty); R=10 (gap extension penalty); wink=1 (generates word hits at every winkth position along the query); and gapw=16 (sets the window width within which gapped alignments are generated). The equivalent Blastp parameter settings for comparison of amino acid sequences can be: Q=9; R=2; wink=1; and gapw=32. A Bestfit comparison between sequences, available in the GCG package version 10.0, can use DNA parameters GAP=50 (gap creation penalty) and LEN=3 (gap extension penalty), and the equivalent settings in protein comparisons can be GAP=8 and LEN=2.

Thus, when referring to the polypeptide or nucleic acid sequences of the present invention, included are sequence identities of at least 65%, 70%, 75%, 80%, or 85%, for example at least 86%, at least 87%, at least 88%, at least 89%, at least 90%, at least 91%, at least 92%, at least 93%, at least 94%, at least 95%, at least 96%, at least 97%, at least 98%, at least 99%, or about 100% sequence identity with the full-length polypeptide or nucleic acid sequence, or to fragments thereof comprising a consecutive sequence of at least 50, at least 75, at least 100, at least 125, at least 150 or more amino acid residues of the entire protein; variants of such sequences, *e.g.,* wherein at least one amino acid residue has been inserted N- and/or C-terminal to, and/or within, the disclosed sequence(s) which contain(s) the insertion and substitution. Contemplated variants can additionally or alternately include those containing predetermined mutations by, *e.g.,* homologous recombination or site-directed or PCR mutagenesis, and the corresponding polypeptides or nucleic acids of other species, including, but not limited to, those described herein, the alleles or other naturally occurring variants of the family of polypeptides or nucleic acids which contain an insertion and substitution; and/or derivatives wherein the polypeptide has been covalently modified by substitution, chemical, enzymatic, or other appropriate means with a moiety other than a naturally occurring amino acid which contains the insertion and substitution (for example, a detectable moiety such as an enzyme).

As used herein, the phrase "conservative amino acid substitution" or "conservative mutation" refers to the replacement of one amino acid by another amino acid with a common property. A functional way to define common properties between individual amino acids is to analyze the normalized frequencies of amino acid changes between corresponding proteins of homologous organisms (Schulz (1979) Principles of Protein Structure, Springer-Verlag). According to such analyses, groups of amino acids can be defined where amino acids within a group exchange preferentially with each other, and therefore resemble each other most in their impact on the overall protein structure (Schulz (1979) Principles of Protein Structure, Springer-Verlag). Examples of amino acid groups defined in this manner can include: a "charged/polar group" including Glu, Asp, Asn, Gln, Lys, Arg, and His; an "aromatic or cyclic group" including Pro, Phe, Tyr, and Trp; and an "aliphatic group" including Gly, Ala, Val, Leu, Ile, Met, Ser, Thr, and Cys. Within each group, subgroups can also be identified. For example, the group of charged/polar amino acids can be sub-divided into sub-groups including: the "positively-charged sub-group" comprising Lys, Arg and His; the "negatively-charged sub-group" comprising Glu and Asp; and the "polar sub-group" comprising Asn and Gln. In another example, the aromatic or cyclic group can be sub-divided into sub-groups including: the "nitrogen ring sub-group" comprising Pro, His, and Trp; and the "phenyl sub-group" comprising Phe and Tyr. In another further example, the aliphatic group can be sub-divided into sub-groups including: the "large aliphatic non-polar sub-group" comprising Val, Leu, and Ile; the "aliphatic slightly-polar sub-group" comprising Met, Ser, Thr, and Cys; and the "small-residue sub-group" comprising Gly and Ala. Examples of conservative mutations include amino acid substitutions of amino acids within the sub-groups above, such as, but not limited to: Lys for Arg or vice versa, such that a positive charge can be maintained; Glu for Asp or vice versa, such that a negative charge can be maintained; Ser for Thr or vice versa, such that a free -OH can be maintained; and Gln for Asn or vice versa, such that a free -NH2 can be maintained.

As used herein, "expression" includes the expression of a gene at least at the level of RNA production, and an "expression product" includes the resultant product, *e.g.,* a polypeptide or functional RNA (*e.g.,* a ribosomal RNA, a tRNA, an antisense RNA, a micro RNA, an shRNA, a ribozyme, *etc.*)*,* of an expressed gene. The term "increased expression" includes an alteration in gene expression to facilitate increased mRNA production and/or increased polypeptide expression. "Increased production" includes an increase in the amount of polypeptide expression, in the level of the enzymatic activity of a polypeptide, or a combination of both, as compared to the native production or enzymatic activity of the polypeptide.

The term "secreted" includes movement of polypeptides or fatty acid products produced by the recombinant microorganisms or methods of the invention to the periplasmic space or extracellular milieu. "Increased secretion" includes secretion in excess of the naturally-occurring amount of secretion, *e.g.,* that is at least 1%, 2%, 3%, 4%, 5%, 6%, 7%, 8%, 9%, or 10%, or at least 20%, 30%, 40%, 50%, 60%, 70%, 80%, 90%, 100%, 200%, 300%, 400%, 500%, 600%, 700%, 800%, 900%, 1000%, or more, as compared to the naturally-occurring level of secretion.

Embodiments of the present invention provide for the "insertion," *e.g.,* the addition, integration, incorporation, or introduction, the activation, or up-regulation of certain nucleic acid molecules or particular polynucleotide sequences, with or without additional regulatory sequences, within microorganisms or host cells in order to affect the activity, such as the expression of an enzyme, of certain nucleic acid molecules or particular polynucleotide sequences. In certain embodiments, a microorganism of interest may be engineered by site directed homologous recombination to insert a particular gene of interest or a promoter that affects the expression of a particular gene or set of genes.

Embodiments of the present invention provide recombinant microorganisms in which the nucleic acid molecules or particular polynucleotide sequences are partially, substantially, or completely deleted, silenced, inactivated, or down-regulated in order to affect the activity for which they encode, such as the expression of an enzyme. Genes can be partially, substantially, or completely deleted, silenced, inactivated, or down-regulated by insertion of nucleic acid sequences that disrupt the function and/or expression of the gene (*e.g.,* P1 transduction or other methods known in the art). The terms "eliminate," "elimination," and "knockout" can be used interchangeably with the terms "deletion," "partial deletion," "substantial deletion," or "complete deletion." In certain embodiments, a microorganism of interest may be engineered by site directed homologous recombination to knockout a particular gene of interest. In still other embodiments, RNAi or antisense DNA (asDNA) may be used to partially, substantially, or completely silence, inactivate, or down-regulate a particular gene of interest.

These insertions, deletions, or other modifications of certain nucleic acid molecules or particular polynucleotide sequences may be understood to encompass "genetic modification(s)" or "transformation(s)" such that the resulting strains of the microorganisms or host cells may be understood to be "genetically modified" or "transformed."

As used herein, "up-regulated" or "up-regulation" includes an increase in expression of a gene or nucleic acid molecule of interest or the activity of an enzyme, *e.g.,* an increase in gene expression or enzymatic activity as compared to the expression or activity in an otherwise identical gene or enzyme that has not been up-regulated.

As used herein, "down-regulated" or "down-regulation" includes a decrease in expression of a gene or nucleic acid molecule of interest or the activity of an enzyme, *e.g.,* a decrease in gene expression or enzymatic activity as compared to the expression or activity in an otherwise identical gene or enzyme that has not been down-regulated.

The term "Pfam" refers to a large collection of protein domains and protein families maintained by the Pfam Consortium and available at several sponsored world wide web sites, including: pfam.sanger.ac.uk/ (Welcome Trust, Sanger Institute); pfam.sbc.su.se/ (Stockholm Bioinformatics Center); pfam.janelia.org/ (Janelia Farm, Howard Hughes Medical Institute); pfam.jouy.inra.fr/ (Institut national de la Recherche Agronomique); and pfam.ccbb.re.kr. The latest release of Pfam is Pfam 26.0 (November 2011) based on the UniProt protein database release 15.6, a composite of Swiss-Prot release 57.6 and TrEMBL release 40.6. Pfam domains and families are identified using multiple sequence alignments and hidden Markov models (HMMs). Pfam-A family or domain assignments, are high quality assignments generated by a curated seed alignment using representative members of a protein family and profile hidden Markov models based on the seed alignment. (Unless otherwise specified, matches of a queried protein to a Pfam domain or family are Pfam-A matches.) All identified sequences belonging to the family are then used to automatically generate a full alignment for the family (Sonnhammer (1998) Nucleic Acids Research 26, 320-322; Bateman (2000) Nucleic Acids Research 26, 263-266; Bateman (2004) Nucleic Acids Research 32, Database Issue, D138-D141; Finn (2006) Nucleic Acids Research Database Issue 34, D247-251; Finn (2010) Nucleic Acids Research Database Issue 38, D211-222). By accessing the Pfam database, for example, using any of the above-reference websites, protein sequences can be queried against the HMMs using HMMER homology search software (*e.g*., HMMER2, HMMER3, or a higher version, hmmer.janelia.org/). Significant matches that identify a queried protein as being in a Pfam family (or as having a particular Pfam domain) are those in which the bit score is greater than or equal to the gathering threshold for the Pfam domain. Expectation values (e values) can also be used as a criterion for inclusion of a queried protein in a Pfam or for determining whether a queried protein has a particular Pfam domain, where low e values (much less than 1.0, for example less than 0.1, or less than or equal to 0.01) represent low probabilities that a match is due to chance.

An enzyme such as an LPAAT or a thioesterase that acts on acyl thioester substrates (*e.g.,* uses acyl-ACP and/or acyl-CoA substrates) often has activity on substrates of different chain lengths, but can have greater activity on one or more substrates than on others. For example, "substrate preference" refers to the substrate or substrates an enzyme is most active on. Different acyl-ACP thioesterases may have different degrees of chain length specificity, sometimes referred to as the enzyme's "preference" for cleaving a particular length of fatty acid from ACP, and thioesterases are typically most active in cleaving a particular chain length fatty acid while having lesser activity in cleaving one or more other chain length fatty acids. Similarly, an LPAAT can have a substrate preference for one or more acyl substrates, such as acyl-ACP or acyl-CoA substrates of particular carbon chain lengths.

As used herein, a "medium chain length" fatty acid or acyl-ACP is a fatty acid or acyl-ACP having a chain length of from 8-14 carbons.

As used herein, a "long chain length" fatty acid or acyl-ACP is a fatty acid or acyl-ACP having a chain length of greater than 14 carbons.

As used herein, the term "fatty acid product" includes free fatty acids; mono-, di- or triglycerides; fatty aldehydes; a fatty alcohols; fatty acid esters (including, but not limited to, wax esters); and hydrocarbons (including, but not limited to, alkanes and alkenes).

### Metabolic Pathways for Producing Fatty Acids

The fatty acid biosynthesis pathway is highly conserved in prokaryotes and in the chloroplasts of eukaryotic algae and higher plants. The fatty acid biosynthesis pathway starts from the central metabolite acetyl-CoA. Fatty acid biosynthesis is initiated by the conversion of acetyl-CoA to malonyl-CoA, catalyzed by acetyl-CoA carboxylase (ACCase). Malonyl-CoA is then converted to malonyl-ACP, catalyzed by malonyl-CoA-ACP transacylase (FabD in *E. coli*)*.* Finally, malonyl-ACP is converted to acyl-ACP, catalyzed by the enzyme complex fatty acid synthase (FAS). The fatty acid synthase complex initiates the elongation cycle by first condensing malonyl-ACP with acetyl-ACP, catalyzed by a beta-ketoacyl-ACP synthase III (*e.g.,* FabH of *E. coli*)*.* The β-ketoacyl-ACP (3-ketoacyl-ACP) formed by the FabH reaction is reduced to a β-hydroxyacyl-ACP (3-hydroxyacyl-ACP) by 3-ketoacyl-ACP reductase (*e.g*. FabG). The β-hydroxyacyl-ACP is then acted on by a β-hydroxyacyl-ACP dehydratase (*e.g*. FabA, FabZ) to form trans-2-enoyl-ACP, which in turn is reduced by enoyl-ACP reductase (*e.g*. Fab I, Fab K, FabL) to form the 2 carbon-elongated acyl-ACP product. Subsequent cycles are initiated by a beta-ketoacyl-ACP synthase I or II (*e.g.,* FabB or FabF) catalyzed condensation of malonyl-ACP with acyl-ACP. The cycles of condensation, reduction, dehydration, and reduction are repeated, with each cycle adding two carbons from malonyl-ACP, until the acyl chain is transferred to another molecule (*e.g*. glycerol 3-phosphate) by a transacylase or cleaved from ACP by a thioesterase, such as FatA or FatB in chloroplasts, to form free fatty acids.

Unlike plant chloroplasts, cyanobacteria do not produce free fatty acids, and unlike *E*. *coli* and other heterotrophic bacteria, cyanobacteria do not produce acyl-CoA. After fatty acid elongation with the acyl chain covalently bound to acyl carrier protein, acyl transferases of cyanobacteria transfer the acyl chain to a glycerol backbone to produce membrane lipids.

To produce fatty acid derivatives such as fatty alcohols, fatty aldehydes, wax esters, alkanes, or alkenes in microorganisms, it is typically necessary to introduce one or more genes encoding one or more enzymes to convert acyl-thioester intermediates (*e.g*., acyl-CoA or acyl-ACP) to the desired end product (*e.g*., an alcohol, aldehyde, alkane, alkene, or wax ester). For example, if fatty aldehydes and/or alkanes are the desired end product, a gene encoding an aldehyde-forming fatty aldehyde reductase (*e.g*., aldehyde-forming acyl-CoA reductase, 1.2.1.42 or 1.2.1.50; see also U.S. Patent No. 6,143,538) may be introduced to reduce acyl-CoA to fatty aldehydes; additionally or alternatively, a carboxylic acid reductase gene (see, *e.g.,* WO 2010/135624 and WO 2010/042664) may be introduced to reduce free fatty acids to fatty aldehydes. Further, a gene encoding a fatty alcohol oxidase (*e.g*., 1.1.3.20) or a fatty alcohol dehydrogenase (*e.g*., 1.1.1.164) may be introduced to convert fatty alcohols to fatty aldehydes. Fatty aldehydes may be processed further to alkanes with the introduction of a gene encoding a fatty aldehyde decarbonylase (*e.g*., 4.1.99.5). If fatty alcohols, alkenes and/or wax esters are the desired end product, a gene encoding an alcohol-forming fatty acyl reductase (*e.g*., alcohol-forming acyl-CoA reductase, 1.2.1.50) may be introduced into the host cell. Further, a fatty aldehyde reductase gene may be introduced to reduce fatty aldehydes to fatty alcohols. Fatty alcohols may be processed further to alkenes with the introduction of one or more genes encoding a fatty alcohol dehydratase. Fatty acid esters, including wax esters, may be formed by introducing genes encoding polypeptides that catalyze condensation of an alcohol with a fatty acyl thioester, such as acyltransferases and wax synthases.

In some embodiments of the invention described above, the conversion of acyl-ACP to fatty alcohol may occur via synthesis of a fatty aldehyde, wherein an acyl reductase (*e.g*., an aldehyde-forming acyl-CoA reductase or aldehyde-forming acyl-ACP reductase) expressed in the host cell first reduces acyl-ACP to a fatty aldehyde. For example, in certain embodiments, the host cell can be engineered to overexpress an endogenous fatty aldehyde-forming reductase (*e.g.,* by inserting promoter and/or enhancer transcriptional control elements near the fatty aldehyde-forming reductase gene). In other embodiments, the host cell may be engineered to express an exogenous fatty aldehyde-forming reductase.

### Lysophosphatidic Acid Acyltransferases

As used herein, the term "lysophosphatidic acid acyltransferase" or "LPAAT", or "1-acyl-sn-glycerol-3-phosphate acyltransferase" or "AGPAT" (encoded by *plsC* in *E. coli* and other prokaryotes), includes those enzymes capable of converting a lysophosphatidic acid to a phosphatidic acid by transferring an acyl chain from an acyl substrate such as acyl-CoA or acyl-ACP to the sn2 position of lysophosphatidic acid. LPAAT includes those enzymes that correspond to Enzyme Commission Number 2.3.1.51.

LPAAT and the enzyme glycerol-3-phosphate acyltransferase (glycerolphosphate acyltransferase; GPAT; encoded by *plsB* in *E. coli*) which catalyzes the conversion of glycerol-3-phosphate to lysophosphatidic acid, share canonical acyltransferase signatures such as HXXXXD or NHXXXXD and XXXXXXG, where X is an arbitrary amino acid, separated by 60 to 83 amino acid residues (Okazaki, K., et al. (2006) Plant Physiol. 141:546-56). Nucleic acids that encode GPATs and LPAATs having these signature sequences have been identified in many organisms, including *Arabidopsis thaliana* (Zheng et al. (2003) Plant Cell 15:1872-87; Kim and Huwang (2004) Plant Physiol. 134:1206-16; Yu et al. (2003) Plant Cell Physiol. 15:1872-87) and *Synechocystis* sp. PCC6803 (Weier et al. (2005) Biochem. Biophys. Res. Commun. 334:1127-34; Okazaki, K., et al. (2006) Plant Physiol. 141:546-56). For example, an endoplasmic reticulum-located (microsomal) LPAAT, which is encoded by the *LAT2* gene from *Limnanthes douglasii* or by the *LPAT2* gene from *A. thaliana,* have 68 or 69 amino acid residues between the canonical acyltransferase signatures and can use C18 acyl substrates, such as C18:1 CoA, for membrane lipid synthesis (Brown et al. (1995) Plant Mol. Biol. 29:267-78; Kim et al. (2005) Plant Cell 17:1073-89). Other seed-specific endoplasmic reticulum-located LPAATs may have 63 amino acid residues or approximately 63 amino acid residues between the canonical acyltransferase signatures and may use unusual acyl substrates, *e.g.,* C12:0 or C22:1 acyl substrates which in various plant species are incorporated into seed storage lipids (Brown et al. (1995) Plant Mol. Biol. 29:267-78; Knutzon et al. (1995) Plant Physiol. 109:999-1006; Lassner et al. (1995) Plant Physiol. 109:1389-94).

LPAATs useful in the microorganisms and methods provided herein may have one or more conserved domains found in cyanobacterial LPAAT sll1752 (SEQ ID NO:2), as characterized in the Conserved Domain Database maintained by the National Center for Biotechnology Information (available at ncbi.nlm.nih.gov/Structure/cdd/cdd.shtml), such as but not limited to: cd07992 (Lysophospholipid Acyltransferases (LPLATs) of Glycerophospholipid Biosynthesis: Unknown AAK14816-like), cd07989 (Lysophospholipid Acyltransferases (LPLATs) of Glycerophospholipid Biosynthesis: AGPAT-like), cd06551 (Lysophospholipid acyltransferases (LPLATs) of glycerophospholipid biosynthesis), cd07993 (Lysophospholipid Acyltransferases (LPLATs) of Glycerophospholipid Biosynthesis: GPAT-like), cd07988 (Lysophospholipid Acyltransferases (LPLATs) of Glycerophospholipid Biosynthesis: Unknown ABO13168), cd07987 (Lysophospholipid Acyltransferases (LPLATs) of Glycerophospholipid Biosynthesis: MGAT-like), COG0204: PlsC (1-acyl-sn-glycerol-3-phosphate acyltransferase), TIGR00530: AGP acyltrn (1-acyl-sn-glycerol-3-phosphate acyltransferases), cd07991: LPLAT LPCAT1-like (Lysophospholipid Acyltransferases (LPLATs) of Glycerophospholipid Biosynthesis: LPCAT1-like), cd07990 (Lysophospholipid Acyltransferases (LPLATs) of Glycerophospholipid Biosynthesis: LCLAT1-like), and cd07986 (Lysophospholipid Acyltransferases (LPLATs) of Glycerophospholipid Biosynthesis: Unknown ACT 14924).

Because LPAATs are essential to the production of phosphatidic acid in all cellular organisms except archea, LPAATs can be identified from a large variety of species, using, *e.g.,* the canonical acyltransferase sequence signatures described above and/or by bioinformatics to identify conserved domains characteristic of LPAATs, such as, but not limited to, those provided above, as well as by inclusion in Pfam PF01553 ("Acyltransferase"), which has a gathering cut-off of 21.1. LPAAT activity and substrate specificity can be determined using, *e.g.,* an acyltransferase assay as described in Okazaki, K. et al. (2006) Plant Physiol. 141:546-56, WO2007141257, or US Patent 5,563,058, in which any of the methods can be adapted to use either acyl-ACP or acyl-CoA as a substrate, or by using any of the methods disclosed herein.

A gene encoding an LPAAT with a particular acyl chain length substrate preference can be expressed in a recombinant cyanobacterium that expresses a non-native thioesterase gene as provided herein to increase the levels of free fatty acids produced by the recombinant host cells or microorganisms. An LPAAT gene can be selected based on the substrate preference of the encoded enzyme in relation to the substrate preference of the thioesterase produced by the recombinant microorganism or host cell. In some aspects of the invention, an LPAAT can be selected that has a different acyl chain length substrate preference than the thioesterase produced by expression of a non-native gene, which can be for example, an acyl-ACP thioesterase, an acyl-CoA thioesterase, or a 4-hydroxybenzoyl thioesterase. In some aspects of the invention, an LPAAT is selected that has a complementary acyl substrate chain length preference with respect to the substrate preference of the thioesterase produced by the recombinant cyanobacterium, where acyl substrates preferred by the LPAAT are not preferred substrates of the thioesterase, and acyl substrates preferred by the thioesterase are not preferred substrates of the LPAAT. For example, in certain aspects an LPAAT is selected that has a medium chain-length acyl substrate preference, *e.g.,* a preference for using a C12 and/or a C14 acyl-ACP or acyl-CoA, and in other aspects, an LPAAT is selected that has a long chain-length acyl-ACP substrate preference, *e.g.,* a preference for using a C16 and/or a C18 acyl-ACP substrate. In certain aspects, an LPAAT is selected that has a medium chain-length acyl-ACP substrate preference and an acyl-ACP thioesterase is selected that has a long chain-length acyl-ACP or acyl-CoA substrate preference. In certain aspects, an LPAAT is selected that has a long chain-length acyl substrate preference and an acyl-ACP thioesterase is selected that has a medium chain-length acyl-ACP substrate preference. The preferred acyl-ACP substrate can have a saturated acyl chain or can have an unsaturated acyl chain.

In some preferred embodiments of the foregoing, the LPAAT encoded by a non-native nucleic acid sequence in the recombinant cyanobacterium has a substrate preference for acyl-ACP or acyl-CoA molecules having longer acyl chain lengths than the acyl substrates preferred by the thioesterase produced by expression of a non-native thioesterase gene in the recombinant cyanobacterium. Without limiting the invention to any particular mechanism, the LPAAT can have a substrate preference for an acyl substrate that has a longer acyl chain than the acyl substrate preferred by the thioesterase produced by the recombinant cyanobacterium, such that during fatty acid biosynthesis, acyl-ACP intermediates are either cleaved by the thioesterase or, if they escape cleavage at the preferred substrate chain length, their acyl chains become further elongated in the fatty acid biosynthesis cycle such that they attain a chain length preferred by the LPAAT. The LPAAT can then act on the acyl-ACP substrate, transferring the acyl chain to a glycerolipid and thereby removing acyl-ACP intermediates that have been elongated beyond the substrate preference of the thioesterase. Such intermediates could otherwise build up in the cell and thereby downregulate fatty acid synthesis. For example, the recombinant microorganism can express an acyl-ACP thioesterase, acyl-CoA thioesterase, or 4-hydroxybenzoyl thioesterase having a medium chain acyl substrate preference (for example, for C8, C10, C12, and/or C14 acyl substrates such as acyl-ACP and/or acyl-CoA) and an LPAAT having a long chain acyl substrate preference (for example, for C16, C18, C20, C22, or C24 acyl substrates such as acyl-ACP and/or acyl-CoA). In some examples, the recombinant cyanobacterium expresses a non-native gene encoding a thioesterase that has a preference for a C12 and/or C14 acyl substrate and expresses a non-native gene encoding an LPAAT that has a preference for one or more C16 and/or C18 acyl substrates. In another example, both the thioesterase and the LPAAT encoded by non-native genes in the recombinant cell can have long chain acyl substrate preferences, where the LPAAT prefers one or more substrates having a longer acyl chain length than the acyl chain length of the substrate(s) preferred by the thioesterase. For example, a recombinant cyanobacterium can express a non-native gene encoding a thioesterase having a C16 acyl substrate preference and a non-native gene encoding an LPAAT having a C18 acyl substrate preference. In further examples, the recombinant cyanobacterium expresses a non-native gene encoding a thioesterase that has a preference for a C12, C14, and/or C16 acyl substrate and expresses a non-native gene encoding an LPAAT that has a preference for a C18 acyl substrate. For example, the recombinant cyanobacterium can express a non-native gene encoding a thioesterase that has a C12 acyl substrate preference or a C14 acyl substrate preference. For example, the recombinant cyanobacterium can express an LPAAT having a C18 acyl substrate preference and can express a non-native gene encoding a thioesterase that has a C16 acyl substrate preference, or the recombinant cyanobacterium can express a non-native gene encoding a thioesterase that has a C14 and C16 acyl substrate preference or a preference for a C12, C14, and C16 acyl substrates.

Prokaryotic LPAATs commonly have a C16 acyl substrate preference, although the invention considers prokaryotic LPAATs having substrate preferences for any acyl chain length, particularly but not exclusively C16 and C18 chain lengths.

The LPAAT encoded by the *sll1752* gene from *Synechocystis* sp. PCC 6803 (GenBank/EMBL accession number NP 440396 or BAA17076; SEQ ID NO:2), orthologs of which are widely conserved in published genomes of cyanobacteria, has been shown to have a preference for C18:0 and C18:1 acyl substrates (Okazaki, K., et al. (2006) Plant Physiol. 141:546-56). The LPAAT encoded by the *sll1752* gene from *Synechocystis* sp. PCC 6803 has a distance of 64 amino acid residues between the canonical acyltransferase signatures, which is similar to seed-specific LPAATs of higher plants.

Cyanobacterial LPAATs considered to be orthologs of the polypeptide encoded by sll1752 (Genbank Accession NP_440396; gene identifier 16329668; SEQ ID NO:2) of *Synechocystis* include, but are not limited to, the phospholipid/glycerol acyltransferase of *Cyanothece* sp. PCC 8801 (Genbank Accession YP_002372580; gene identifier 218247209; SEQ ID NO:3), the phospholipid/glycerol acyltransferase of *Crocosphaera watsonii* WH 8501 (Genbank Accession ZP_00513647; gene identifier 67920127; SEQ ID NO:4), hypothetical protein CY0110_27159 of *Cyanothece* sp. CCY0110 (Genbank Accession ZP_01731155; gene identifier 126660033; SEQ ID NO:5), the unnamed protein product of *Microcystis aeruginosa* PCC 7806 (Genbank Accession CAO89042; gene identifier 159026728; SEQ ID NO:6), the hypothetical protein all4871 of *Nostoc* sp. PCC 7120 (Genbank Accession NP_488911; gene identifier 17232363; SEQ ID NO:7), the unnamed protein product of *Anabaena variabilis* ATCC 29413 (Genbank Accession YP_322656; gene identifier 75908360; SEQ ID NO:8), the phospholipid/glycerol acyltransferase of *'Nostoc azollae'* 0708 (Genbank Accession YP_003721758; gene identifier 298491581; SEQ ID NO:9), the phospholipid/glycerol acyltransferase of *Raphidiopsis brookii* D9 (Genbank Accession ZP_06305519; gene identifier 282897518; SEQ ID NO:10), the acyltransferase domain protein of *Microcoleus chthonoplastes* PCC 7420 (Genbank Accession ZP_05026385; gene identifier 254412612; SEQ ID NO:11), the phospholipid/glycerol acyltransferase of *Cylindrospermopsis raciborskii* CS-505 (Genbank Accession ZP_06306989; gene identifier 282899007; SEQ ID NO:12), the conserved hypothetical protein of *Oscillatoria* sp. PCC 6506 (Genbank Accession ZP_07110610; gene identifier 300865867; SEQ ID NO:13), the phospholipid/glycerol acyltransferase of *Nodularia spumigena* CCY9414 (Genbank Accession ZP_01628592; gene identifier 119509444; SEQ ID NO:14), and the phospholipid/glycerol acyltransferase of *Microcoleus vaginatus* FGP-2 (Genbank Accession ZP_08495621; gene identifier 334121556; SEQ ID NO:15). These and other orthologs of sll1752 (SEQ ID NO:2) of *Synechocystis* are considered for use in the microorganisms and methods provided herein. Recombinant cyanobacteria that include a non-native nucleic acid sequence that encodes an LPAAT and produce at least one free fatty acid or at least one fatty acid derivative can include non-native sequences encoding LPAATs having at least 85%, at least 90%, at least 95%, at least 96%, at least 97%, at least 98%, at least 99%, or 100% identity to SEQ ID NO:2.

### Thioesterases

As used herein, the term "thioesterase" is intended to include hydrolases capable of acting on a thioester bond. Such enzymes can correspond to, *e.g.,* Enzyme Commission Number 3.1.2.2, 3.1.2.14, 3.1.2.18, 3.1.2.19, 3.2.1.20, 3.1.2.22, 3.1.2.23, or 3.1.2.27. A thioesterase expressed in a recombinant microorganism or host cell of the invention is an acyl-ACP thioesterase. For example, a recombinant cyanobacterium or host cell can be transformed with a gene encoding an exogenous acyl-ACP thioesterase, such as a gene encoding a polypeptide that when queried against the Pfam database, provides a match with Pfam PF01643 having a bit score of less than or equal to 20.3 (the gathering cut-off for PF01643). The acyl-ACP thioesterase gene encodes an acyl-ACP thioesterase from a higher plant species. Genes encoding acyl-ACP thioesterases derived from higher plants can include, without limitation, genes encoding acyl-ACP thioesterases from *Cuphea* species (*e.g. Cuphea carthagenensis, Cuphea wrightii* (*e.g.,* AAC49784.1 GI:1336008), *Cuphea lanceolata* (*e.g.,* CAA54060, GI495227), *Cuphea palustris,* (*e.g.,* AAC49783.1 GI:1336006; AAC49179.1 GI:1215718); *Cuphea hookeriana* (*e.g.,* AAC72882.1 GI:3859830; AAC49269.1 GI:1292906; AAC72881.1 GI:3859828; AAC72883.1 GI:3859832), *Cuphea calophylla* (*e.g.,* ABB71580.1 GI:81361963) or genes of various Cuphea species disclosed in United States patent application publication US 2011/0020883, or genes from other higher plant species. For example, a cyanobacterium used in the methods and cultures disclosed herein can include a gene encoding an acyl-ACP thioesterase from species such as but not limited to, *Arabidopsis* (XP_002885681.1 GI:297835598; NP_172327.1 GI:15223236); *Arachis hypogaea* (*e.g.,* AB038556.1 GI:133754634); *Brassica* species (*e.g.,* CAA52069.1 GI:435011), *Camellia oleifera* (*e.g.,* ACQ57189.1 GI:229358082); *Cinnamonum camphorum* (*e.g.,* AAC49151.1 GI:1143156); *Cocos nucifera; Glycine max* (*e.g.,* ABD91726.1 GI:90192131); *Garcinia mangostana* (*e.g.,* AAB51525.1 GI:1930081); *Gossypium hirsutum (e.g.,* AAD01982.1 GI:4104242); *Helianthus annuus* (*e.g.,* AAQ08226 GI:33325244); *Jatropha curcas* (*e.g.,* ABU96744.1 GI:156900676); *Macadamia tetraphylla* (*e.g.,* ADA79524.1 GI:282160399); *Elaeis oleifera* (*e.g.,* AAM09524.1 GI:20067070); *Elaeis guineensis* (*e.g.,* AAD42220 GI:30962820); *Oryza sativa* (*e.g.,* BAA83582.1 GI:5803272); *Populus tomentosa* (*e.g.,* ABC47311 GI:83778888); *Umbellularia californica* (*e.g.,* AAC49001.1 GI:595955); *Ulmus Americana* (*e.g.,* AAB71731.1 GI:2459533); and *Zea mays* (ACG41291.1 GI:195643646), or any of those disclosed in U.S. Patent No. 5,455,167; U.S. Patent No. 5,654,495; and U.S. Patent No. 5,455,167; and in U.S. Patent Appl. Pub. Nos. 2009/0298143 and 2011/0020883. Further included are acyl-ACP thioesterases from mosses (Bryophyta), such as, for example, *Physcomitrella patens,* (*e.g.,* XP 001770108 GI:168035219). These examples are not limiting with regard to the types or specific examples of acyl-ACP thioesterase genes that can be used.

Genes encoding acyl-ACP thioesterases of plants and bryophytes may include sequences encoding transit peptides for transport into plastids that can optionally be deleted for expression of N-terminally truncated thioesterases in host cyanobacteria. Genes encoding thioesterases may further optionally include additional truncations, such as but not limited to N-terminal truncations that include a portion, such as up to ten, up to twenty, or up to thirty amino acids of a mature acyl-ACP thioesterase sequence.

Acyl-ACP thioesterases typically can be active to some degree on acyl-ACP substrates having a plurality of different acyl chain lengths, but can have higher activity on (*e.g.,* have a substrate preference for) one or more acyl-ACP substrates having particular acyl chain lengths than on other chain length substrates. In some examples, an enzyme referred to as having a substrate preference for particular acyl chain length substrates produces at least twice as much product of the preferred substrate or substrates as it does from a non-preferred substrate, and for example can produce at least three times, at least four times, or at least five times as much product from a preferred substrate as from a non-preferred substrate. For example, an acyl-ACP thioesterase may have a substrate preference for one or more of acyl-ACP substrates having acyl chain lengths of 8, 10, 12, 14, 16, 18, 20, 22, and/or 24 carbons. Additionally or alternately, the acyl-ACP thioesterase can hydrolyze one or more acyl-ACP substrates having an acyl chain length from 8 to 18 carbons, for example from 12 to 18 carbons. For example, the acyl-ACP thioesterase can hydrolyze one or more acyl-ACP substrates having an acyl chain length from 12 to 16 carbons. Further additionally or alternately, an acyl-ACP thioesterases of the present invention can, in some embodiments, have its highest level of activity on an acyl-ACP substrate having an acyl chain length of 12, 14, and/or 16 carbons.

### Microorganisms and Host Cells

Algae for use in the invention, include without limitation, microalgae, such as but not limited to, an *Achnanthes, Amphiprora, Amphora, Ankistrodesmus, Asteromonas, Boekelovia, Borodinella, Botryococcus, Bracteococcus, Chaetoceros, Carteria, Chlamydomonas, Chlorococcum, Chlorogonium, Chlorella, Chroomonas, Chrysosphaera, Cricosphaera, Crypthecodinium, Cryptomonas, Cyclotella, Dunaliella, Ellipsoidon, Emiliania, Eremosphaera, Ernodesmius, Euglena, Franceia, Fragilaria, Gloeothamnion, Haematococcus, Halocafeteria, Hymenomonas, Isochrysis, Lepocinclis, Micractinium, Monoraphidium, Nannochloris, Nannochloropsis, Navicula, Neochloris, Nephrochloris, Nephroselmis, Nitzschia, Ochromonas, Oedogonium, Oocystis, Ostreococcus, Pavlova, Parachlorella, Pascheria, Phaeodactylum, Phagus, Picochlorum, Platymonas, Pleurochrysis, Pleurococcus, Prototheca, Pseudochlorella, Pseudoneochloris, Pyramimonas, Pyrobotrys, Scenedesmus, Schizochytrium, Skeletonema, Spyrogyra, Stichococcus, Tetraselmis, Viridiella,* or *Volvox* species.

Cyanobacterial species that can be used for production of fatty acid products include, without limitation, *Agmenellum, Anabaena, Anabaenopsis, Anacystis, Aphanizomenon, Arthrospira, Asterocapsa, Borzia, Calothrix, Chamaesiphon, Chroococcus, Chlorogloeopsis, Chroococcidiopsis, Chroococcus, Crinalium, Cyanobacterium, Cyanobium, Cyanocystis, Cyanospira, Cyanothece, Cylindrospermopsis, Cylindrospermum, Dactylococcopsis, Dermocarpella, Fischerella, Fremyella, Geitleria, Geitlerinema, Gloeobacter, Gloeocapsa, Gloeothece, Flalospirulina, Iyengariella, Leptolyngbya, Limnothrix, Lyngbya, Microcoleus, Microcystis, Myxosarcina, Nodularia, Nostoc, Nostochopsis, Oscillatoria, Phormidium, Planktothrix, Pleurocapsa, Prochlorococcus, Prochloron, Prochlorothrix, Pseudanabaena, Rivularia, Schizothrix, Scytonema, Spirulina, Stanieria, Starria, Stigonema, Symploca, Synechococcus, Synechocystis, Thermosynechococcus, Tolypothrix, Trichodesmium, Tychonema* and *Xenococcus.* For example, the recombinant photosynthetic microorganism can be a *Cyanobium, Cyanothece,* or *Cyanobacterium* species, or further alternatively, the recombinant photosynthetic microorganism can be a *Gloeobacter, Lyngbya or Leptolyngba* species. Alternatively, the recombinant photosynthetic microorganism can be a *Synechococcus, Synechocystis,* or *Thermosynechococcus* species. A number of cyanobacterial species are known and have been manipulated using molecular biological techniques, including the unicellular cyanobacteria *Synechocystis* sp. PCC6803 and *Synechococcus* elongates PCC7942, whose genomes have been completely sequenced.

The invention provides a recombinant cyanobacterium comprising a non-native nucleic acid molecule that includes a nucleic acid sequence encoding an LPAAT and a non-native nucleic acid sequence that encodes a thioesterase. The nucleic acid sequence encoding the LPAAT and the nucleic acid sequence encoding the thioesterase can be present on the same or different nucleic acid molecules. The recombinant cyanobacterium can produce a fatty acid product, such as a fatty acid, fatty alcohol, fatty aldehyde, fatty acid ester, wax ester, or hydrocarbon (such as an alkane or alkene). The recombinant host cell may comprise, *e.g.,* any of the nucleic acid sequences encoding an LPAAT described herein and may comprise any of the nucleic acid sequences encoding a thioesterase described herein. The recombinant host cell may comprise, *e.g.,* any of the vectors described herein.

The recombinant host cell can comprise a non-native gene encoding an LPAAT with at least 85%, 86%, 87%, 88%, 89%, 90%, 91%, 92%, 93%, 94%, 95%, 96%, 97%, 98%, or 99% sequence identity to SEQ ID NO: 2.

Illustrative examples of recombinant cyanobacteria that express a non-native LPAAT gene and a non-native thioesterase gene include recombinant cyanobacteria that express a non-native gene encoding an LPAAT having a C18 substrate preference, such as an LPAAT having at least 85% identity to SEQ ID NO:2 (for example, at least 90% or at least 95% identity to SEQ ID NO:2) and a higher plant acyl-ACP thioesterase having a substrate preference for one or more of a C12, C14, or C16 acyl substrate, for example, the C12-preferring FatB1 thioesterase of *Umbellularia californica* (AAC49001.1 GI:595955), the C14-preferring thioesterase of *Cinnamomum camphorum* (Q39473.1 GI:8469216), the N-terminally truncated C16-preferring acyl-ACP thioesterase of *Elaeis oleifera* (SEQ ID NO:56), the C16-preferring acyl-ACP thioesterase of *Elaeis guineensis* (SEQ ID NO:57) or a truncated version thereof, the C14 and C16-preferring FatB2 thioesterase of *Cuphea palustris* (AAC49180.1 GI:1215720), Cc1FatB1 thioesterase of *Cuphea carthagenensis,* or a variant thereof (*e.g.,* the truncated polypeptide of SEQ ID NO:44), or acyl-ACP thioesterases of other *Cuphea* species (see, for example, United States patent application publication US 2011/0020883), including, for example, the N-terminally truncated C14, C16-preferring Cd1FatB1 acyl-ACP thioesterase of SEQ ID NO:54 or a variant thereof, the N-terminally truncated C16-preferring Cp1FatB1 thioesterase of SEQ ID NO:55 or a variant thereof, or the C14, the C16-preferring *C*. *hookeriana* FatB1 thioesterase (Q39513.1 GI:8469217). Also specifically considered are thioesterases having at least 85% identity, for example at least 90% or at least 95% identity to the aforementioned *Umbellularia, Cinnamomum, Cuphea* and *Elaeis* acyl-ACP thioesterases, in which the acyl-ACP thioesterases have C12, C14, and/or C16 substrate specificities.

The recombinant host cell expressing an LPAAT gene in combination with a thioesterase gene produces a greater amount of a fatty acid product than a control host cell that does not express the LPAAT gene. In some embodiments, the amount of fatty acid product produced by a culture of the recombinant host cell expressing a non-native gene encoding an LPAAT and a non-native gene encoding a thioesterase is at least 10%, 15%, 20%, 25%, 30%, 40%, 50%, 60%, 65%, 70%, 75%, 80%, 90%, 95%, 100%, 125%, 150%, 175%, 200%, 225%, 250%, 275%, 300%, 325%, 350%, 375%, 400%, 425%, 450%, 475%, 500%, 525%, 550%, 575%, 600%, 625%, 650%, 675%, 700%, 725%, 750%, 775%, 800%, 825%, 850%, 875%, 900%, 925%, 950%, 975%, or 1000% greater than the amount of wax ester produced by a control host cell that does not express the non-native LPAAT gene.

In some embodiments, the recombinant host cell expressing an LPAAT and a thioesterase produces a greater amount of a fatty acid product than a control host cell that does not express the LPAAT and the thioesterase. In some embodiments, the amount of a fatty acid product produced by a culture of the recombinant host cell expressing an LPAAT and a thioesterase is at least 10%, 15%, 20%, 25%, 30%, 40%, 50%, 60%, 65%, 70%, 75%, 80%, 90%, 95%, 100%, 125%, 150%, 175%, 200%, 225%, 250%, 275%, 300%, 325%, 350%, 375%, 400%, 425%, 450%, 475%, 500%, 525%, 550%, 575%, 600%, 625%, 650%, 675%, 700%, 725%, 750%, 775%, 800%, 825%, 850%, 875%, 900%, 925%, 950%, 975%, or 1000% greater than the amount of fatty acid product produced by a control host cell that does not express the LPAAT and the thioesterase.

### Additional Host Cell Modifications for Free Fatty Acid (FFA) Production

Further modifications to the recombinant cyanobacteia or host cells of the invention can be made to affect free fatty acid production. For example, the recombinant cyanobacterium can include one or more additional non-native genes whose expression increases the production of a fatty acid product. The additional gene may be encoded by a nucleic acid molecule that is the same as the nucleic acid molecule that encodes the LPAAT and/or the nucleic acid molecule that encodes the thioesterase, or the additional gene may be encoded by a separate nucleic acid molecule. Where two or more genes are encoded by the same nucleic acid molecule (*e.g*., on the same expression vector), the expression of each gene may optionally be independently regulated by a same or a different promoter and/or enhancer. In certain embodiments, the additional gene may increase the rate and/or level of free fatty acid or fatty acid derivative production.

For example, the recombinant host cell expressing an LPAAT, and a thioesterase, can further express at least one additional recombinant or exogenous gene that encodes a polypeptide having lipolytic activity. For example, a recombinant host cell of the invention can include one or more genes encoding one or more polypeptides having lipolytic activity, where the polypeptide(s) having lipolytic activity are capable of producing free fatty acids from membrane lipids or storage lipids, *e.g.,* phospholipids, triacylglycerols, diacylglycerols, monoacylglycerols, or the like, or combinations thereof. The polypeptides having lipolytic activity can be, for example, lipases, esterases, cutinases, or amidases.

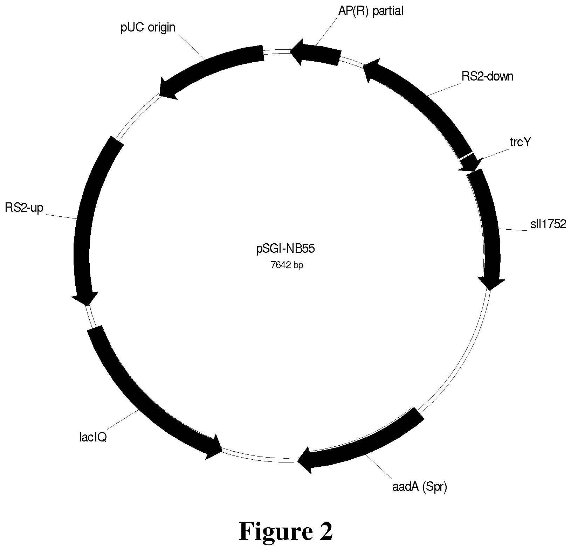

Without limiting the invention to any particular mechanisms, it is considered that overexpression of an LPAAT gene may result in significantly increased production of membrane or storage lipids. Excess membrane or storage lipids can be acted on by polypeptides having lipolytic activity (such as, *e.g.,* one or more lipases or amidases) to release additional fatty acids (that can optionally be further converted to fatty acid derivatives), thereby further increasing the amount of fatty acid products made by the recombinant microorganism (see, for example, the model provided in Figure 2).

The use of genes encoding polypeptides having lipolytic activity in microorganisms for the production of free fatty acids is disclosed in commonly-assigned U.S. patent application No. 13/324,653 entitled "Production of Free Fatty Acids and Fatty Acid Derivatives by Recombinant Microorganisms Expressing Polypeptides Having Lipolytic Activity," filed on December 13, 2011. The polypeptide having lipolytic activity can be for example a lipase, *e.g.,* that liberates a fatty acid from a glycerolipid (including a monoglyceride, a diglyceride, a triglyceride, a phospholipid, a galactolipid, *etc.*) or can be an amidase. For example, the recombinant microorganism can include a non-native gene encoding a lipase, such as but not limited to a lipase that is a member of a Pfam belonging to the AB Hydrolase Pfam clan (CL0028). For example, a non-native gene encoding a polypeptide having lipolytic activity can encode a lipase that includes a LipA domain identified as conserved protein domain COG1075, or is included in the protein family Pfam PF01674 (Lipase 2); a non-native nucleic acid molecule that encodes a lipase that includes a Lipase 3 domain identified as conserved protein domain COG3675, or is included in the protein family Pfam PF01764 (Lipase 3); a non-native nucleic acid molecule that encodes a lipase that is included in the protein family Pfam PF07819 (PGAP1); or a non-native nucleic acid molecule that encodes a polypeptide that is included in any of the protein families Pfam PF03583, Pfam PF00151 (Lipase), Pfam PF00561 (Ab hydrolase 1), Pfam PF02230 (Ab hydrolase 2), Pfam PF07859 (Ab hydrolase 3), Pfam PF08386 (Ab hydrolase 4), Pfam PF12695 (Ab hydrolase 5), Pfam PF12697 (Ab hydrolase 6), Pfam PF12715 (Ab hydrolase 7), Pfam PF04083 (Ab hydro lipase). In some embodiments, an exogenous lipase gene introduced into a microorganism can encode a protein with an amino acid sequence having an E-value parameter of 0.01 or less when queried using the Pfam Profile HMM for any of Pfam PF01674, Pfam PF 01764, Pfam PF07819, Pfam PF03583, and/or Pfam PF00151. Further, the recombinant microorganism can include a non-native gene encoding an amidase having lipolytic activity, such as but not limited to an amidase that recruits to Pfam PF01425 (Amidase) with a bit score greater than the gathering cutoff of 20.1 and can catalyze the release of fatty acids from lipids.

Additionally or alternately contemplated are recombinant cyanobacteria that are engineered to include gene regulatory sequences that induce or increase expression of an endogenous lipase gene. For example, a cyanobacterium can be engineered such that a heterologous promoter is inserted upstream of a coding region of an endogenous lipase gene. The heterologous promoter can replace an endogenous promoter and/or can be inserted upstream or downstream of the endogenous promoter that regulates expression of the endogenous lipase gene, for example using homologous recombination or site-specific recombination. The heterologous promoter can be a constitutive promoter or an inducible promoter that increases expression of the endogenous lipase gene.

Further modifications to the recombinant cyanobacteria of the invention can be made to affect free fatty acid production. For example, a recombinant cyanobacterium or host cell of the invention can include one or more exogenous nucleic acid molecules that encode a polypeptide that participates in the synthesis of a fatty acid, including, but not limited to, an acetyl-CoA carboxylase, a malonyl CoA: ACP transacylase, or a beta-ketoacyl-ACP synthase.

Additionally or alternatively, the recombinant cyanobacterium can comprise a modification of an endogenous nucleic acid molecule that encodes, *e.g.,* an acyl-CoA synthetase, acyl-ACP synthetase, acyl CoA dehydrogenase, glycerol-3-phosphate dehydrogenase, acetaldehyde CoA dehydrogenase, pyruvate dehydrogenase, acetate kinase, and the like, and combinations thereof. In certain embodiments, the modification down-regulates the endogenous nucleic acid and includes partial, substantial, or complete deletion, silencing, or inactivation of the nucleic acid or its regulatory elements.

In some examples, the host cell can have attenuated expression of an endogenous gene encoding an acyl-ACP synthetase which participates in the recycling of fatty acids into lipids. The endogenous acyl-ACP synthetase gene can be, for example, downregulated by deletion or mutation of the promoter, or the protein-encoding of the gene can be internally deleted or disrupted, for example, by insertional mutagenesis. Alternatively, the entire acyl-CoA synthetase gene can be deleted, for example, by homologous recombination or other genome modification techniques. In yet further alternatives, gene knockdown constructs such as but not limited to ribozyme, antisense, or RNAi constructs can be introduced into the host cell to attenuate expression of the endogenous acyl-ACP synthetase gene.

Additionally or alternatively, the recombinant cyanobacterium can be modified such that one or more genes that encode beta-oxidation pathway enzymes have been inactivated and/or down-regulated, and/or such that the enzymes themselves that are operative on such beta-oxidation pathways may be inhibited. This could prevent the degradation of fatty acids released from acyl-ACPs, thus enhancing the yield of fatty acid products. In cases where the desired products are medium-chain fatty acids, the inactivation and/or down-regulation of genes that encode acyl-CoA synthetase and/or acyl-CoA oxidase enzymes that preferentially use these chain lengths as substrates can be made. Mutations in the genes encoding medium-chain-specific acyl-CoA synthetase and/or medium-chain-specific acyl-CoA oxidase enzymes, such that the activity of the enzymes are diminished, may additionally or alternately be effective in increasing the yield of produced and/or released fatty acid products. Mutations in the genes can be introduced either by recombinant or non-recombinant methods.

Genes may be targeted specifically by disruption, deletion, generation of antisense sequences, generation of ribozymes, RNAi, meganuclease genome modification, and/or other recombinant approaches. Inactivation of the genes can additionally or alternately be accomplished by random mutation techniques such as exposure to UV and/or chemical mutagens, and the resulting genes and/or enzymes can be screened for mutants with the desired activity. The proteins themselves can be inhibited by intracellular generation of appropriate antibodies, intracellular generation of peptide inhibitors, or the like, or some combination thereof.

In additional embodiments, a recombinant cyanobacterium of the invention comprises can be modified such that one or more genes that encode storage carbohydrate and/or polyhydroxyalkanoate (PHA) biosynthesis pathway enzymes are inactivated or down-regulated, and/or such that the enzymes themselves that are operative on such pathways are inhibited. Examples include, but are not limited to, enzymes involved in glycogen, starch, or chrysolaminarin synthesis, including glucan synthases and/or branching enzymes. Other examples include enzymes involved in PHA biosynthesis such as acetoacetyl-CoA synthase and PHA synthase.

### Modifications for Fatty Acid Derivative Production

The recombinant cyanobacteria of the invention can include additional modifications for the production of fatty acid derivatives such as, *e.g.,* fatty aldehydes, fatty alcohols, fatty acid esters, wax esters, and hydrocarbons, including alkanes and alkenes. In some embodiments, the recombinant cyanobacterium comprises one or more nucleic acid molecules encoding an exogenous acyl-CoA reductase, carboxylic acid reductase, and/or acyl-ACP reductase and can produce a fatty alcohol.

For example, for the production of fatty aldehydes, which can optionally be further converted to products such as fatty alcohols, wax esters, or alkanes, a transgenic cyanobacterium as provided herein can include an exogenous gene(s) that encodes an aldehyde-forming reductase, such as, for example, an aldehyde-forming acyl-CoA reductase, an aldehyde-forming acyl-ACP reductase, or a carboxylic acid reductase. Genes or portions of genes that are listed in GenBank and other genetic databases and that are predicted to encode proteins that are homologous to known acyl-CoA reductases that produce fatty aldehydes, referred to herein as "aldehyde-generating fatty acyl-CoA reductases", can be introduced into various microorganisms in order to test for the production of specific fatty aldehydes or fatty alcohols produced therefrom. Nonlimiting examples of fatty aldehyde-generating acyl-CoA reductases include the Acr1 gene of *Acinetobacter baylyi* (Accession U77680, GI:1684885), the AcrM-1 gene of *Acinetobacter* sp. M-1 (Accession YP 001086217, GI:18857900), and the luxC and luxE genes of various photoluminescent bacteria, *e.g,* an *Altermonas, Photobacterium, Shewanella, Vibrio,* or *Xenorhabdus* species. The enzymes encoded by these and other genes identified, for example, by sequence homology or protein domain can be tested to determine their substrates and products using assays know in the art.

Nonlimiting examples of carboxylic acid reductases that can be used in the invention include the *Nocardia* CAR gene (Accession AY495697; GI:40796034) and homologs thereof, some of which are disclosed in US2010/0105963.

In some examples, the host cell can include a non-native gene encoding an aldehyde-forming acyl-ACP reductase such as but not limited to any of those disclosed in WO 2009/140696 and WO 2011/066137. For example, the recombinant host cell may comprise an aldehyde-forming acyl-ACP reductase that has at least 50%, 60%, 70%, 80%, 90% or 95% sequence identity to an aldehyde-forming reductase, *e.g.,* as disclosed in WO 2009/140696 or WO 2011/066137, such as, for example, any of the reductases having the accession numbers AAM82647; AAM82647; BAD78241; ABA22149; BAB76983; ZP_03763674; ACL42791; ZP_01628095; ZP_01619574; YP_001865324; YP_721978; NP_682102; YP_001518341; YP_002371106; ZP_05027136; ZP_03273554; NP_442146; ZP_01728620; ZP_05039135; YP_001802846; NP_926091; YP_001660322; ZP_00516920; CAO90781; ZP_01085337; YP_001227841; ABD96327; NP_897828; YP_001224378; ABD96480; ZP_01123215; ABB92249; ZP_01079773; YP_377636; NP_874926; NP_895058; ABD96274; ABD96442; ZP_01469469; ZP_05045052; YP_001014416; YP_001010913; YP_381056; YP_001550421; NP_892651; YP_001090783; ZP_01472595; YP_293055; ZP_05138243; YP_731192; YP_001483815; YP_001008982; YP_473896; YP_478638; or YP_397030. In some embodiments the recombinant host cell includes an exogenous gene encoding an aldehyde-forming acyl-ACP reductase, where the aldehyde-forming acyl-ACP reductase can be from a cyanobacterial species, and may be from the same species as the host microorganism, or may be from a different species. Alternatively, a cyanobacterial host can be engineered to overexpress an endogenous acyl-ACP reductase gene.
For the production of fatty alcohols, a recombinant cyanobacterium as provided herein can include an exogenous gene encoding an alcohol-forming acyl reductase such as bfar from *Bombyx mmori;* jjfar from *Simmondsia chinensis,* an acyl-CoA reductase from *Titicum aestivum,* mfar1 of *Mus musculus,* mfar2 from *Mus musculus,* hfar from *H. sapiens,* FARXIII of *Ostrinia scapulalis,* MS2 of *Z*. *mays,* the putative fatty acyl-coA reductase of *Oryza sativa* (GenBank accession BAC84377) or MS2, FAR4, FAR6, or CER4 of *Arabidopsis thaliana.* An alcohol-forming fatty acyl-CoA reductase can also be a prokaryotic enzyme, such as for example, those having Genbank accession numbers AAC45217 (*Acinetobacter baylyi* fatty acyl-CoA reductase), YP_047869 (*Acinetobacter* sp. ADP1 fatty acyl-CoA reductase), BAB85476 (*Acinetobacter* sp. M-1 acyl coenzyme A reductase), YP_001086217 (*Acinetobacter baumannii* ATCC 17978 acyl coenzyme A reductase), YP_580344 short-chain dehydrogenase/reductase SDR [*Psychrobacter cryohalolentis* K5], YP_001280274 (*Psychrobacter* sp. PRwf-1 short-chain dehydrogenase/reductase SDR), the acyl reductase of *Marinobacter algicola* DG893 (Accession ZP_01892457), the short chain acyl dehydrogenase of *Marinobacter aquaeolei* Maqu_2507 (YP_959769) *Marinobacter aquaeolei* VT8 Maqu_2220 (YP_959486), *Hahella chejuensis* Hch_05075 (YP_436183), *Marinobacter adhaerens* HP15_810 (ADP96574), or an acyl reductase of an *Oceanobacter* species (*e.g.,* RED65_09894, Accession EAT13695). Alcohol-forming reductases may include those that are able to use acyl-ACP as a substrate, as disclosed in the co-pending, commonly-assigned U.S. patent application No. 61/539,640 entitled "Fatty Alcohol-Forming Acyl-ACP Reductases", filed on September 27, 2011.

Alternatively or in addition, the recombinant cyanobacterium comprises one or more nucleic acid molecules encoding an exogenous acyl-CoA reductase, carboxylic acid reductase, and/or acyl-ACP reductase, and an exogenous wax synthase and can produce a wax ester. Wax esters include an A chain and a B chain linked through an ester bond, one or both of which can be derived from a fatty acid generated by the recombinant cyanobacteria of the invention. Wax esters produced by the recombinant cyanobacteria of the invention include, *e.g.,* A chain lengths of from 8 to 24 carbons, for example, 12 to 18 carbons, or 12 to 16 carbons, and/or B chain lengths of from 8 to 24 carbons, for example, 12 to 18 carbons, or 12 to 16 carbons. For example, the wax esters can have A+B chain lengths including, but not limited to, of 16 to 48 carbons, 24 to 36 carbons, or 24 to 32 carbons.

Wax synthases include polypeptides having enzyme classification number EC 2.3.1.75, as well as any other peptide capable of catalyzing the conversion of an acyl-thioester to fatty esters, *e.g.,* some acyltransferases, including some DGATs. Some wax synthase peptides can catalyze other reactions as well, for example some wax synthase peptides will accept short chain acyl-CoAs and short chain alcohols to produce fatty esters. Methods to identify wax synthase activity are provided in U.S. Pat. No. 7,118,896. Nonlimiting examples of wax synthases that can be encoded by an exogenous nucleic acid molecule introduced into a recombinant cyanobacterium as disclosed herein include the bifunctional wax ester synthase/acyl-CoA:diacylglycerol acyltransferase of *Simmondsia chinensis* (AAD38041), the wax synthase of *Acinetobacter* sp. strain ADP 1 (CAG67733), *Pseudomonas aeruginosa* (AAG06717), *Arabidopsis thaliana* (Q93ZR6), *Alcanivorax* (EDX90960), *Rhodococcus opacus* (YP_002782647), *Homo sapiens* (Q6E213), *Mus musculus* (Q6E1M8), or *Petunia x hybrida* (AAZ08051).

In some embodiments, the recombinant cyanobacteria of the invention comprise at least one nucleic acid molecule encoding an exogenous fatty acid decarboxylase or an exogenous fatty aldehyde decarbonylase, or additionally at least one exogenous nucleic acid molecule encoding an exogenous acyl-CoA reductase, carboxylic acid reductase, or acyl-ACP reductase, and can produce an alkane and/or alkene. Alkanes and alkenes produced by the recombinant microorganisms or host cells of the invention can, for example, have chain lengths of 7, 9, 11, 13, 15, 17, 19, 21, and/or 23 carbons, including, for example, chain lengths of 7, 9, 11, 13, 15, and/or 17 carbons, or chain lengths of 7, 9, 11, 13, and/or 15 carbons, or chain lengths of 11, 13, and/or 15 carbons.

Additionally, the recombinant cyanobacteria of the invention that produce a fatty alcohol, fatty aldehyde, fatty acid ester, wax ester, or hydrocarbons, including an alkane or an alkene, may optionally include a nucleic acid molecule encoding an exogenous acyl-CoA synthetase, or may be engineered to have upregulated expression of an endogenous acyl-CoA synthetase gene.

Additionally, the recombinant host cell may optionally be engineered to express an exogenous transmembrane transporter to facilitate secretion of one or more fatty acid products. For example, the recombinant host cell can include a non-native gene encoding an ATP-binding cassette (ABC) transporter or an RND pump. In some embodiments, the transporter is at least 80% identical in sequence to a transporter protein encoded by an *Arabidopsis* genes CER5, WBC11, AtMRPS, AmiS2 and AtPGP1, or fatty acid transporter (FATP) genes from *Saccharomyces, Drosophila,* mycobacterial species, or mammalian species.

Also disclosed are genes encoding variants of these and other naturally-occurring enzymes that participate in the synthesis of fatty acid products having at least 65% identity to the referenced or naturally-occurring proteins, in which the activity of the enzyme is not substantially reduced with respect to the wild-type or above-referenced enzyme.

The above-described recombinant host cells may be used in any of the methods of producing a fatty acid product as described herein.

### Nucleic Acid Molecules

The nucleic acid molecules and polypeptides described herein can be used in any of the methods of the invention, and may be included in any of the recombinant cyanobacteria of the invention. Nucleic acid molecules comprising sequences that encode LPAATs are provided for use in host cyanobacteria and methods for producing fatty acid products, including free fatty acids, fatty aldehydes, fatty alcohols, fatty acid esters, wax esters, alkanes, and/or alkenes. A nucleic acid molecule as disclosed herein can be isolated and/or purified.

In some embodiments, expression in a host cyanobacterium of a recombinant nucleic acid molecule or sequence encoding an LPAAT as described herein results in a higher production level of a fatty acid or a fatty acid derivative by the host cyanobacterium than the production level in a control cyanobacterium, where the control cyanobacterium is cultured under the same conditions and is substantially identical to the cyanobacterium expressing the non-native nucleic acid molecule or sequence encoding the LPAAT in all respects, with the exception that the control cyanobacterium does not express a non-native nucleic acid molecule that encodes an LPAAT.

In further embodiments, expression in a host cyanobacterium of a non-native nucleic acid sequence encoding an LPAAT and a non-native nucleic acid sequence encoding a thioesterase as described herein results in a higher production level of a fatty acid product by the host cyanobacterium than the production level in a control cyanobacterium, where the control cyanobacterium is cultured under the same conditions and is substantially identical to the cyanobacterium expressing the recombinant nucleic acid molecule(s) or sequences in all respects, with the exception that the control cyanobacterium does not express a thioesterase and does not express a non-native nucleic acid sequences encoding an LPAAT.

In some embodiments, the invention relates to nucleic acid molecules encoding deletion mutants of an LPAAT where one or more amino acids have been deleted from the protein. For example, the encoded polypeptide can lack at least 1, 2, 3, 4, 5, 6, 7, 8, 9, 10, 20, 30, 40, 50, 60, 70, or 80 amino acids from the N- and/or C-terminus and can have an amino acid sequence at least 85%, 86%, 87%, 88%, 89%, 90%, 91%, 92%, 93%, 94%, 95%, 96%, 97%, 98%, 99%, or 100% identical to the corresponding amino acid sequence of SEQ ID NO: 2. In some examples, the deleted sequences may include targeting sequences, for example, at least a portion of a chloroplast transit peptide, at least a portion of a mitochondrial targeting sequence, at least a portion of an endoplasmic reticulum targeting sequence, *etc.*

Further, the invention relates to nucleic acid molecules encoding variants of naturally-occurring LPAAT amino acid sequences, such as but not limited to variants of the LPAAT sequence of SEQ ID NO: 2. Variants may be naturally occurring, or non-naturally-occurring, such as those induced by various mutagens and mutagenic processes. In some embodiments, a nucleic acid molecule encodes a variant of an LPAAT in which at least one amino acid residue has been inserted N- and/or C-terminal to, and/or within, the reference sequence. In some embodiments, at least one amino acid residue has been deleted N- and/or C-terminal to, and/or within, the reference sequence. In some disclosures, the nucleic acid molecules may encode variants that may be sequences containing predetermined mutations by, *e.g.,* homologous recombination or site-directed or PCR mutagenesis; corresponding proteins of other species; alleles or other naturally occurring variants; and/or derivatives wherein the protein has been covalently modified by chemical, enzymatic or other appropriate means with a moiety other than a naturally occurring amino acid.

A substitution, insertion or deletion can adversely affect the protein when the altered sequence substantially inhibits a biological function associated with the protein. In certain aspects of the disclosure, a variant of an LPAAT may have activity that is reduced by not more than about 1%, 2%, 3%, 4%, 5%, 6%, 7%, 8%, 9%, 10%, 15%, 20%, 30%, 40%, or 50%, in comparison to the activity of the LPAAT from which the variant is derived (*e.g*., SEQ ID NO: 2). In some aspects of the disclosure, the amount of a fatty acid product produced by a host cell expressing the LPAAT variant is not less than about 99%, 98%, 97%, 96%, 95%, 94%, 93%, 92%, 91%, 90%, 85%, 80% or 75% of the amount or the fatty acid product produced by a host cell expressing the LPAAT from which the variant is derived (*e.g*., SEQ ID NO: 2).

The invention also relates to fragments and variants of an LPAAT that have increased activity in comparison to the reference polypeptides. In certain aspects, the LPAAT fragment or variant may have activity that is increased by at least 5%, 10%, 20%, 30%, 40%, 50%, 60%, 70%, 80%, 90%, 100%, 200%, 300%, 400%, 500%, 600%, 700%, 800%, 900%, or 1000% in comparison to the activity of the LPAAT from which the variant is derived. In certain aspects, the amount of fatty acid or fatty acid derivative produced by a host cell expressing the fragment or variant is at least 5%, 10%, 20%, 30%, 40%, 50%, 60%, 70%, 80%, 90%, 100%, 200%, 300%, 400%, 500%, 600%, 700%, 800%, 900% or 1000% of the amount of fatty acid product made by a host cell expressing the LPAAT from which the fragment or variant is derived.

Any of the disclosed nucleic acid molecules can optionally further comprise an additional nucleic acid sequence of at least 50, 60, 70, 80, 90, 100, 150, 200, 250, 300, 350, 400, 450, 500, 550, 600, 700, 800, 900, 1000, or 1500 nucleotides from a photosynthetic organism.

### Other Modifications

The invention also relates to further variants of the nucleotide sequences of the invention. In some embodiments, the nucleotide sequence variants encode fragments or variants of the polypeptides as described herein. In some embodiments, the nucleotide sequence variants are naturally-occurring. In other embodiments, the nucleotide sequence variants are non-naturally-occurring, such as those induced by various mutagens and mutagenic processes. In certain embodiments, the nucleotide sequence variants are a combination of naturally- and non-naturally-occurring. A given nucleic acid sequence may be modified, for example, according to standard mutagenesis or artificial evolution or domain swapping methods to produce modified sequences. Accelerated evolution methods are described, *e.g.* by Stemmer (1994) Nature 370, 389-391, and Stemmer (1994) Proc. Natl. Acad. Sci. USA 91, 10747-10751. Chemical or enzymatic alteration of expressed nucleic acids and polypeptides can be performed by standard methods. For example, a sequence can be modified by addition of phosphate groups, methyl groups, lipids, sugars, peptides or organic or inorganic compounds, by the inclusion of modified nucleotides or amino acids, or the like.

For optimal expression of a recombinant protein, in certain instances it may be beneficial to employ coding sequences that produce mRNA with codons preferentially used by the host cell to be transformed ("codon optimization"). Thus, for enhanced expression of transgenes, the codon usage of the transgene can be matched with the specific codon bias of the organism in which the transgene is desired to be expressed. The precise mechanisms underlying this effect are believed to be many, but can include the proper balancing of available aminoacylated tRNA pools with proteins being synthesized in the cell, coupled with more efficient translation of the transgenic messenger RNA (mRNA) when this need is met. In some embodiments, only a portion of the codons is changed to reflect a preferred codon usage of a host cyanobacterium. In certain embodiments, one or more codons are changed to codons that are not necessarily the most preferred codon of the host microorganism encoding a particular amino acid. Additional information for codon optimization is available, *e.g*. at the codon usage database of GenBank. The coding sequences may be codon optimized for optimal production of a desired product in the host organism selected for expression. In certain embodiments, the non-native nucleic acid sequence encoding an LPAAT and/or the non-native nucleic acid sequence encoding a thioesterase is codon optimized for expression in a cyanobacterium.

In some embodiments, the nucleic acid molecules of the disclosure encode fusion proteins that comprise an LPAAT. For example, the nucleic acids of the invention may comprise polynucleotide sequences that encode glutathione-S-transferase (GST) or a portion thereof, thioredoxin or a portion thereof, maltose binding protein or a portion thereof, poly-histidine (e.g. His₆), poly-HN, poly-lysine, a hemagglutinin tag sequence, HSV-Tag and/or at least a portion of HIV-Tat fused to the LPAAT-encoding sequence.

In some embodiments, the nucleic acid molecules of the disclosure comprise additional non-coding sequences such as non-coding 3' and 5' sequences (including, *e.g.,* regulatory sequences) that may be homologous or heterologous to the LPAAT gene.

### Nucleic Acid Constructs

The invention also relates to constructs comprising a nucleic acid sequence encoding an LPAAT and/or a thioesterase that can further include one or more sequences that regulate or mediate transcription, translation, or integration of nucleotide sequences into a host genome. For example, the invention relates to expression constructs that comprise one or more "expression control elements" or sequences that regulate expression transcription of an operably linked gene, or translation of the transcribed RNA. For example, an expression control element can be a promoter that may be operably linked to the gene of interest (*e.g*., an LPAAT gene) in an expression construct or "expression cassette." In some embodiments of the foregoing, the promoter is regulatable, *e.g.,* inducible.

In aspects of the disclosure where the nucleic acid construct does not contain a promoter in operable linkage with the nucleic acid sequence encoding the gene of interest (*e.g*., an LPAAT gene) the nucleic acid sequence can be transformed into the cells such that it becomes operably linked to an endogenous promoter by, *e.g.,* homologous recombination, site specific integration, and/or vector integration. In some aspects, genomic host sequences included in a nucleic acid construct for mediating homologous recombination into the host genome can include gene regulatory sequences, for example, a promoter sequence, that can regulate expression of an LPAAT gene of the nucleic acid construct. In such aspects, the transgene(s) of the construct can become operably linked to a promoter that is endogenous to the host microorganism. In some embodiments, the endogenous promoter(s) are regulatable, *e.g.,* inducible.

A promoter operably linked to a nucleic acid sequence encoding an LPAAT may be a promoter that is heterologous with respect to the LPAAT gene. In some embodiments of the foregoing disclosure, the promoter may be an inducible promoter, *i.e.,* a promoter that mediates transcription of an operably linked gene in response to a particular stimulus. Such promoters may be advantageous, *e.g.,* to minimize any deleterious effects on the growth of the host cell and/or to maximize production of the fatty acid product. An inducible promoter can be responsive to, *e.g.,* light or dark or high or low temperature, and/or can be responsive to specific compounds. The inducible promoter may be, an ara promoter, a lac promoter, a trp promoter, a tet promoter (*e.g*., U.S. Patent No. 5,851,796), a hybrid promoter that includes a portion of a trp, lac, or tet promoter, a hormone-responsive promoter (*e.g*., an ecdysone-responsive promoter, such as described in U.S. Patent No. 6,379,945), a metallothionien promoter (*e.g*., U.S. Patent No. 6,410,828), a pathogenesis-related (PR) promoter that can be responsive to a chemical such as, for example, salicylic acid, ethylene, thiamine, and/or BTH (U.S. Patent No. 5,689,044), or the like, or some combination thereof. An inducible promoter can also be responsive to light or dark (U.S. Patent No. 5,750,385, U.S. Patent No. 5,639,952), metals (Eukaryotic Cell 2:995-1002 (2003)) or temperature (U.S. Patent No. 5,447,858; Abe et al. Plant Cell Physiol. 49: 625-632 (2008); Shroda et al. Plant J. 21: 121-131 (2000)). The foregoing list is exemplary and not limiting.. The promoter sequence can be from any organism, provided that it is functional in the host organism. In certain embodiments, inducible promoters are formed by fusing one or more portions or domains from a known inducible promoter to at least a portion of a different promoter that can operate in the host cell, *e.g.* to confer inducibility on a promoter that operates in the host species.

For transformation of cyanobacteria, a variety of promoters that function in cyanobacteria can be utilized, including, but not limited to, the ara, lac, tac, and trc promoters, as well as derivatives that are also inducible by the addition of isopropyl β-D-1-thiogalactopyranoside (IPTG) such as the *trcY* or *trcE* promoter. Other promoters that may find use in the invention include promoters that are naturally associated with transposon- or bacterial chromosome-borne antibiotic resistance genes (*e.g.,* neomycin phosphotransferase, chloramphenicol acetyltransferase, spectinomycin adenyltransferase, or the like, or combinations thereof), promoters associated with various heterologous bacterial and native cyanobacterial genes, promoters from viruses and phages, synthetic promoters, or the like, or combinations thereof. For example, the promoter(s) can be selected from prokaryotic promoters from a range of species, including eubacterial and cyanobacterial species, such as, for example, an araC or pBAD promoter, a rha promoter, a Pm promoter, a xylS promoter, a nir promoter, a nar promoter, a pho promoter, a tet promoter, a cys promoter, a metallothionien promoter, an ftf promoter, a gln promoter, a heat shock promoter, a cold-inducible promoter or a viral promoter. The foregoing promoters are exemplary and are not limiting. Promoters isolated from cyanobacteria that can be used can include but are not limited to the following: nrs (nickel-inducible), secA (secretion; controlled by the redox state of the cell), rbc (Rubisco operon), psaAB (PS I reaction center proteins; light regulated), psbA (D1 protein of PSII; light- inducible), and the like, and combinations thereof. In some embodiments, the promoters are regulated by nitrogen compounds, such as, for example, nar, ntc, nir or nrt promoters. In some aspects of the disclosure, the promoters are regulated by phosphate (*e.g.,* pho or pst promoters) or metals, *e.g.,* the nrs promoter (Liu and Curtis (2009) Proc Natl Acad Sciences USA 106: 21550-21554), or the petE promoter (Buikema and Haselkorn (2001) Proc Natl Acad Sciences USA 98: 2729-2734)). Inducible promoters, as used in the constructs of the present disclosure, can use one or more portions or domains of the aforementioned promoters and/or other inducible promoters fused to at least a portion of a different promoter that can operate in the host organism, *e.g.,* to confer inducibility on a promoter that operates in the host species.

Likewise, a wide variety of transcriptional terminators can be used for expression vector construction. Examples of possible terminators can include, but are not limited to, psbA, psaAB, rbc, secA, T7 coat protein, and the like, and combinations thereof.

In some aspects of the disclosure, an isolated or recombinant nucleic acid molecule of the invention can comprise both a nucleic acid sequence that encodes an LPAAT and a nucleic acid sequence that encodes a thioesterase. The nucleic acid sequences encoding the LPAAT and the thioesterase may be, for example, any of the nucleic acid sequences described herein.

In certain aspects of the disclosure, the nucleic acid sequence that encodes an LPAAT and the nucleic acid sequence that encodes a thioesterase can be operably linked to the same promoter and/or enhancer. For example, in particular aspects of the disclosure the two genes (encoding an LPAAT and a thioesterase) may be organized as an operon, in which, for example, a promoter sequence is followed, in the 5' to 3' direction, by a thioesterase-encoding sequence and then an LPAAT -encoding sequence. In an alternative configuration of the operon, a promoter sequence is followed, in the 5' to 3' direction, by an LPAAT -encoding sequence and then a thioesterase-encoding sequence. In some aspects of the disclosure, an isolated nucleic acid molecule can include two or more genes arranged in tandem, where the isolated nucleic acid molecule does not include a promoter sequence that operates in the intended host microorganism upstream of the two or more genes. In these aspects, the promoterless operon can be designed for integration (*e.g*., homologous recombination) into a site of the host genome that may include a promoter sequence, such that the synthetic operon can be transcriptionally regulated by a promoter in the genome of the host microorganism. Further, the operon may be designed for integration (*e.g*., homologous recombination) into a site of the host genome that may include an enhancer sequence, such that the introduced operon can be transcriptionally regulated by an enhancer in the genome of the host microorganism. In any of the above disclosed operons that include LPAAT and thioesterase genes, one or more additional regulatory sequences can be included in the isolated nucleic acid molecule, for example, a sequence for enhancing translation can be included upstream of any of the gene-encoding sequences, and/or a transcriptional terminator can optionally be included at or near the 3' end of the synthetic operon.

In addition to an LPAAT gene and a thioesterase gene, one or more additional genes can optionally be included in a synthetic operon as provided herein, where the one or more additional genes may include, for example, one or more genes encoding enzymes or proteins of the fatty acid synthesis pathway and/or one or more genes encoding enzymes or proteins that may enhance fatty acid product synthesis, one or more genes that may enhance photosynthesis or carbon-fixation, and/or one or more reporter genes or selectable markers.

The nucleic acid sequence that encodes an LPAAT and the nucleic acid sequence that encodes a thioesterase may be provided on the same nucleic acid construct where they are operably linked to different promoters and/or transcriptional enhancers. The promoters and enhancers may be, *e.g.,* any of the promoters and transcriptional enhancers described herein.

The vector comprising a nucleic acid sequence encoding an LPAAT is designed for transformation into cyanobacteria. The vector may permit homologous recombination of the LPAAT-encoding sequence with the cyanobacterial genome.

An isolated nucleic acid molecule of the present disclosure can include the sequences disclosed herein that encode an LPAAT or other polypeptide in a vector, such as, but not limited to, an expression vector. A vector can be a nucleic acid that has been generated via human intervention, including by recombinant means and/or direct chemical synthesis, and can include, for example, one or more of: 1) an origin of replication for propagation of the nucleic acid sequences in one or more hosts (which may or may not include the production host); 2) one or more selectable markers; 3) one or more reporter genes; 4) one or more expression control sequences, such as, but not limited to, promoter sequences, enhancer sequences, terminator sequences, sequence for enhancing translation, *etc.;* and/or 5) one or more sequences for promoting integration of the nucleic acid sequences into a host genome, for example, one or more sequences having homology with one or more nucleotide sequences of the host microorganism. A vector can be an expression vector that includes one or more specified nucleic acid "expression control elements" that permit transcription and/or translation of a particular nucleic acid in a host cell. The vector can be a plasmid, a part of a plasmid, a viral construct, a nucleic acid fragment, or the like, or a combination thereof.

The vector can be a high copy number vector, a shuttle vector that can replicate in more than one species of cell, an expression vector, an integration vector, or a combination thereof. Typically, the expression vector can include a nucleic acid comprising a gene of interest operably linked to a promoter in an "expression cassette," which can also include, but is not limited to, a transcriptional terminator, a ribosome binding site, a splice site or splicing recognition sequence, an intron, an enhancer, a polyadenylation signal, an internal ribosome entry site, and similar elements. The present disclosure can involve recombinant microorganisms transformed with an isolated nucleic acid comprising a gene of interest under control of a heterologous promoter. Alternatively, if the vector does not contain a promoter operably linked with an isolated nucleic acid comprising a gene of interest, the isolated nucleic acid can be transformed into the microorganisms or host cells such that it becomes operably linked to an endogenous promoter by homologous recombination, site specific integration, and/or vector integration.

The present invention additionally provides recombinant cyanobacteria transformed with an isolated nucleic acid comprising a gene of interest that is operably linked to one or more expression control elements. In some instances, it can be advantageous to express the protein at a certain point during the growth of the recombinant microorganism, *e.g.,* to minimize any deleterious effects on the growth of the recombinant microorganism and/or to maximize production of the fatty acid product of interest. In such instances, one or more exogenous genes introduced into the recombinant cyanobacterium can be operably linked to an inducible promoter, which mediates transcription of an operably linked gene in response to a particular stimulus.

Transformation vectors can additionally or alternately include a selectable marker, such as, but not limited to, a drug resistance gene, an herbicide resistance gene, a metabolic enzyme and/or factor required for survival of the host (for example, an auxotrophic marker), or the like, or a combination thereof. Transformed cells can be selected based upon the ability to grow in the presence of the antibiotic and/or other selectable marker under conditions in which cells lacking the resistance cassette or auxotrophic marker could not grow. Further, a non-selectable marker may be present on a vector, such as a gene encoding a fluorescent protein or enzyme that generates a detectable reaction product.

A vector comprising an isolated nucleic acid comprising a gene of interest can also be an integration vector that includes one or more sequences that promote integration of the gene of interest or a gene expression cassette into the genome of the host microorganism or host cell. For example, an integration vector used to transform cyanobacteria can include at least one sequence of at least 50, at least 100, at least 200, at least 300, at least 400, at least 500, or at least 600 nucleotides with homology to a sequence in the genome of the host organism to allow integration of the gene of interest or gene expression cassette into the genome of the host microorganism or host cell to occur via homologous recombination. In some examples, the gene or gene expression cassette is flanked by sequences homologous to a region of the host chromosome to promote integration of the gene of interest or gene expression cassette into the host chromosome. Alternatively or in addition, an integration vector can include one or more sequences that promote site-specific recombination or random integration such as, but not limited to, sequences recognized by recombinases, integrases, or transposases. The integration vector may further include a gene encoding a recombinase, integrase, or transposase.

### Transformation of Microorganisms and Host Cells

A vector comprising an isolated nucleic acid comprising a gene of interest can be introduced into cyanobacteria via conventional transformation and/or transfection techniques. The terms "transformation," "transfection," "conjugation," and "transduction," as used in the present context, are intended to comprise a multiplicity of methods known to those skilled in the art for the introduction of foreign nucleic acid (for example, exogenous DNA) into a host cell, including calcium phosphate and/or calcium chloride coprecipitation, DEAE-dextran-mediated transfection, lipofection, natural competence, chemically mediated transfer, electroporation, particle bombardment, or the like, or combinations thereof. Examples of suitable methods for the transformation and/or transfection of host cells, *e.g.,* can be found in Molecular Cloning - A Laboratory Manual (2010), Cold Spring Harbor Laboratory Press.

Host cells for use in the invention can be transformed by any feasible means, including, without limitation, the use of particle gun-mediated transformation, laser-mediated transformation, or electroporation. Photosynthetic bacteria can be transformed by any suitable methods, including, as nonlimiting examples, natural DNA uptake (Chung et al. (1998) FEMS Microbiol. Lett. 164: 353-361; Frigaard et al. (2004) Methods Mol. Biol. 274: 325-40; Zang et al. (2007) J. Microbiol. 45: 241-245), conjugation, transduction, glass bead transformation (Kindle et al. (1989) J. Cell Biol. 109: 2589-601; Feng et al. (2009) Mol. Biol. Rep. 36: 1433-9; U.S. Pat. No. 5,661,017), silicon carbide whisker transformation (Dunahay et al. (1997) Methods Mol. Biol. (1997) 62: 503-9), biolistics (Dawson et al. (1997) Curr. Microbiol. 35: 356-62; Hallmann et al. (1997) Proc. Natl. Acad. USA 94: 7469-7474; Jakobiak et al. (2004) Protist 155:381-93; Tan et al. (2005) J. Microbiol. 43: 361-365; Steinbrenner et al. (2006) Appl Environ. Microbiol. 72: 7477-7484; Kroth (2007) Methods Mol. Biol. 390: 257-267; U.S. Pat. No. 5,661,017) electroporation (Kjaerulff et al. (1994) Photosynth. Res. 41: 277-283; Iwai et al. (2004) Plant Cell Physiol. 45: 171-5; Ravindran et al. (2006) J. Microbiol. Methods 66: 174-6; Sun et al. (2006) Gene 377: 140-149; Wang et al. (2007) Appl. Microbiol. Biotechnol. 76: 651-657; Chaurasia et al. (2008) J. Microbiol. Methods 73: 133-141; Ludwig et al. (2008) Appl. Microbiol. Biotechnol. 78: 729-35), laser-mediated transformation, or incubation with DNA in the presence of or after pre-treatment with any of poly(amidoamine) dendrimers (Pasupathy et al. (2008) Biotechnol. J. 3: 1078-82), polyethylene glycol (Ohnuma et al. (2008) Plant Cell Physiol. 49: 117-120), cationic lipids (Muradawa et al. (2008) J. Biosci. Bioeng. 105: 77-80), dextran, calcium phosphate, or calcium chloride (Mendez-Alvarez et al. (1994) J. Bacteriol. 176: 7395-7397), optionally after treatment of the cells with cell wall-degrading enzymes (Perrone et al. (1998) Mol. Biol. Cell 9: 3351-3365).

### Methods of Producing Fatty Acid Products

The invention encompasses methods of producing a fatty acid product by culturing the recombinant cyanobacteria described herein, under conditions in which at least one fatty acid product is produced. The methods can further comprise isolating at least one fatty acid product. In some embodiments, at least a portion of the fatty acid and/or fatty acid derivative produced by the recombinant cyanobacteria is released or secreted into the growth media by the microorganism. In some embodiments, the expression of a polypeptide encoded by the nucleic acid molecules described herein can be induced in the recombinant cyanobacterium to produce the fatty acid product.

Releasing and secreting, as used herein in the context of products of the invention, are used interchangeably to refer to a process by which active and/or passive transport mechanisms allow products of the invention to cross the cell membrane to exit the cell. Examples of such transport mechanisms can include, but are not limited to, gradient diffusion, facilitated diffusion, active transport, and combinations thereof.

Culturing refers to the intentional fostering of growth (*e.g*., increases in cell size, cellular contents, and/or cellular activity) and/or propagation (*e.g*., increases in cell numbers via mitosis) of one or more cells by use of selected and/or controlled conditions. The combination of both growth and propagation may be termed proliferation. Non-limiting examples of selected and/or controlled conditions can include the use of a defined medium (with known characteristics such as pH, ionic strength, and/or carbon source), specified temperature, oxygen tension, carbon dioxide levels, growth in a bioreactor, or the like, or combinations thereof. In some embodiments, the cyanobacterium can be grown heterotrophically, using a reduced carbon source, or mixotrophically, using both light and a reduced carbon source. Additionally or alternately, the cyanobacterium can be cultured phototrophically. When growing phototrophically, the cyanobacterium can advantageously use light as an energy source. An inorganic carbon source, such as CO₂ or bicarbonate, can be used for synthesis of biomolecules by the microorganism. "Inorganic carbon", as used herein, includes carbon-containing compounds or molecules that cannot be used as a sustainable energy source by an organism. Typically "inorganic carbon" can be in the form of CO₂ (carbon dioxide), carbonic acid, bicarbonate salts, carbonate salts, hydrogen carbonate salts, or the like, or combinations thereof, which cannot be further oxidized for sustainable energy nor used as a source of reducing power by organisms. If an organic carbon molecule or compound is provided in the culture medium of a cyanobacterium grown phototrophically, it generally cannot be taken up and/or metabolized by the cell for energy and/or typically is not present in an amount sufficient to provide sustainable energy for the growth of the cell culture. However, organic carbon molecules can be used by cyanobacteria growing heterotrophically. Thus, the present invention includes a process for converting a carbon source to a fatty acid product comprising contacting the carbon source with a recombinant cyanobacterium of the invention. In some aspects the carbon source is an inorganic carbon source and in other aspects the carbon source is an organic carbon source.

Cyanobacteria that can be useful in accordance with the methods of the present invention can be found in various locations and environments throughout the world. Without wishing to be bound by theory, it is observed that, perhaps as a consequence of their isolation from other species and their evolutionary divergence, the particular growth medium for optimal growth and generation of lipid and/or other hydrocarbon constituents can vary. In some cases, certain strains of cyanobacteria may be unable to grow in a particular growth medium because of the presence of some inhibitory component or the absence of some essential nutritional requirement of the particular strain of cyanobacterium.

Solid and liquid growth media are generally available from a wide variety of sources, as are instructions for the preparation of particular media suitable for a wide variety of strains of cyanobacteria.

In some embodiments, media used for culturing an organism that produces fatty acids can include an increased concentration of a metal (typically provided as a salt and/or in an ionic form) such as, for example, sodium, potassium, magnesium, calcium, strontium, barium, beryllium, lead, iron, nickel, cobalt, tin, chromium, aluminum, zinc, copper, or the like, or combinations thereof (particularly multivalent metals, such as magnesium, calcium, and/or iron), with respect to a standard medium formulation, such as, for example, standard BG-11 medium (ATCC Medium 616, Table 5), or a modified medium such as ATCC Medium 854 (BG-11 modified to contain vitamin B12) or ATCC Medium 617 (BG-11 modified for marine cyanobacteria, containing additional NaCl and vitamin B12).

For example, a medium used for growing microorganisms that produce free fatty acids can include at least 2-fold, for example at least 3-fold, at least 4-fold, at least 5-fold, at least 6-fold, at least 7-fold, at least 8-fold, at least 9-fold, at least 10-fold, between 2-fold and 10-fold, and/or between 10-fold and 100-fold the amount of metal (*e.g*., calcium) as compared to a standard medium. The medium used for growing microorganisms that can produce free fatty acids can include, for example, at least 0.5 mM, between about 0.5 mM and about 1 mM, between about 1 mM and about 2 mM, between about 2 mM and about 5 mM, between about 5 mM and about 10 mM, between about 10 mM and about 25 mM, and greater than 25 mM metal (*e.g*., calcium) in the formulation.

In further embodiments, by using the excess amount of metal (*e.g*., calcium) in the medium, at least a portion of the fatty acid(s) can be sequestered as soap precipitates, which may result in decreasing the toxic effects of free fatty acid(s). Addition of metal (*e.g*., calcium) in the medium can additionally or alternately increase the tolerance of microorganism in media with a relatively high concentration of free fatty acids. Additionally or alternately, fatty acid-producing strains can advantageously be more robust with excess metal (*e.g*., calcium) content. Although the excess component is described herein as a metal, it is contemplated that the component can more generally be described as a carboxylate counterion source, for example a soap-forming counterion source, a metal ion source (noted as "metal" herein), a multivalent (*i.e*., having a valence of +2 or higher) counterion source, a divalent counterion source, or some combination thereof. Other details regarding this metal/carboxylate counterion source are described in the co-pending, commonly-assigned U.S. Patent Application No. 13/324,636, entitled "Culturing a Microorganism in a Medium with an Elevated Level of a Carboxylate Counterion Source", filed on December 13, 2011.

The culture methods can include inducing expression of a particular gene described herein for the production of fatty acid products, and/or regulating metabolic pathway in the cyanobacterium. Inducing expression can include adding a nutrient or compound to the culture, removing one or more components from the culture medium, increasing or decreasing light and/or temperature, and/or other manipulations that promote expression of the gene of interest. Such manipulations can largely depend on the nature of the (heterologous) promoter operably linked to the gene of interest.

In some embodiments of the present invention, the recombinant cyanobacteria can be cultured in a bioreactor. "Bioreactor" refers to an enclosure or partial enclosure in which cells are cultured, optionally in suspension and, when suspended, preferably in an aqueous liquid. Bioreactors can offer many advantages for use in heterotrophic growth and propagation methods. To produce biomass for use as food, cyanobacteria are preferably fermented in large quantities in liquid, such as in suspension cultures as an example. Bioreactors such as steel fermentors can accommodate very large culture volumes (40,000 liter and greater capacity bioreactors can be used in various embodiments of the invention). Bioreactors can also typically allow for the control of one or more culture conditions such as temperature, pH, oxygen tension, carbon dioxide levels, and the like, as well as combinations thereof. Bioreactors can typically be configurable, for example, using ports attached to tubing, to allow gaseous components, such as CO₂, CO₂-enriched air, oxygen, and/or nitrogen, to be contacted with (*e.g*., bubbled through) a liquid culture. Other culture parameters, such as the pH of the culture media, the identity and/or concentration of trace elements and/or nutrients, the identity and/or concentration of other media constituents, or the like, or combinations thereof, can typically be more readily manipulated using a bioreactor.

Cyanobacteria can additionally or alternately be cultured in a bioreactor equipped with an artificial light source, a "photobioreactor", and/or can have one or more walls that is transparent enough to light, including sunlight, to enable, facilitate, and/or maintain acceptable cyanobacterium growth. For production of fatty acid products, photosynthetic microorganisms or host cells can additionally or alternately be cultured in shake flasks, test tubes, vials, microtiter dishes, petri dishes, or the like, or combinations thereof.

Additionally or alternately, recombinant cyanobacteria may be grown in ponds, canals, sea-based growth containers, trenches, raceways, channels, or the like, or combinations thereof. As with standard bioreactors, a source of inorganic carbon (such as, but not limited to, CO₂, bicarbonate, carbonate salts, and the like), including, but not limited to, air, CO₂-enriched air, flue gas, or the like, or combinations thereof, can be supplied to the culture. When supplying flue gas and/or other sources of inorganic that may contain CO in addition to CO₂, it may be necessary to pre-treat such sources such that the CO level introduced into the (photo)bioreactor do not constitute a dangerous and/or lethal dose with respect to the growth and/or survival of the cyanobacteria.

The methods include culturing a recombinant cyanobacterium that includes a protein(s) as described herein to produce at least one fatty acid product, in which the method results in production of at least 0.1%, 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100%, 110%, 120%, 130%, 140%, 150%, 160%, 170%, 180%, 190%, 200%, 300%, 400%, 500%, 600%, 700%, 800%, 900%, or 1000% more than the amount of the fatty acid product produced by an otherwise identical cyanobacterium not including the protein(s), cultured under identical conditions. Additionally or alternately, the methods include producing at least 100 mg, at least 110 mg, at least 120 mg, at least 130 mg, at least 140 mg, at least 150 mg, at least 160 mg, at least 170 mg, at least 180 mg, at least 190 mg, at least 200 mg, at least 210 mg, at least 220 mg, at least 230 mg, at least 240 mg, at least 250 mg, at least 260 mg, at least 270 mg, at least 280 mg, at least 290 mg, at least 300 mg, at least 310 mg, at least 320 mg, at least 330 mg, at least 340 mg, at least 350 mg, at least 360 mg, at least 370 mg, at least 380 mg, at least 390 mg, at least 400 mg, at least 450 mg, at least 500 mg, at least 550 mg, at least 600 mg, at least 650 mg, at least 700 mg, at least 750 mg, at least 800 mg, at least 850 mg, at least 900 mg, at least 950 mg, per liter of culture of a fatty acid product by culturing the recombinant cyanobacteria described herein. Although many times the goal can be to produce and/or recover as much fatty acid product as possible, in some instances the amount of the fatty acid product produced and/or recovered by the method described herein can be limited to 600 mg or less per liter of culture, for example to 550 mg or less per liter of culture, or to 500 mg or less per liter of culture.

Fatty acid products can be recovered from culture by recovery means known to those of ordinary skill in the art, such as by whole culture extraction, for example, using organic solvents. In some cases, recovery of fatty acid products can be enhanced by homogenization of the cells. When fatty acid products are sufficiently released or secreted from the microorganisms into the culture medium, the recovery method can be adapted to efficiently recover only the released fatty acid products, only the fatty acid products produced and stored within the microorganisms, or both the produced and released fatty acid products.

Fatty acid products secreted/released into the culture medium by the recombinant cyanobacteria described above can be recovered in a variety of ways. A straightforward isolation method, *e.g.,* by partition using immiscible solvents, may be employed. Additionally or alternately, particulate adsorbents can be employed. These can include lipophilic particulates and/or ion exchange resins, depending on the design of the recovery method. They may be circulating in the separated medium and then collected, and/or the medium may be passed over a fixed bed column, for example a chromatographic column, containing these particulates. The fatty acid products can then be eluted from the particulate adsorbents, *e.g.,* by the use of an appropriate solvent. In such circumstances, one isolation method can include carrying out evaporation of the solvent, followed by further processing of the isolated fatty acid products, to yield chemicals and/or fuels that can be used for a variety of commercial purposes.

In some embodiments of the methods described herein, the level of a fatty acid product, for example a C8-C24 fatty acid, a C12-C24 fatty acid, or a C12-C18 fatty acid, such as, for example, at least one of a C12, C14, C16, and/or a C18 fatty acid, can be increased in the culture with respect to a culture of an otherwise identical cyanobacterium, but lacking the genetic modifications of the invention.

It is to be understood that the disclosure of the present invention extends to methods, products and systems according to the various aspects of the invention which comprise combinations of one or more features discussed herein by reference to certain embodiments of the invention with one or more further features discussed herein by reference to certain other embodiments of the invention.

### Examples

The invention as described above can be readily understood by reference to the following examples, which are included for purposes of illustration of certain aspects and embodiments of the present invention, and are not intended to limit the invention.

### Example 1. Production of Free Fatty Acids in Reduced Feedback Inhibition Synechocystis sp. PCC6803 Strains

While the invention is not bound by any one theory, the inventors hypothesized that C18:0 acyl-ACP, which is not a preferred substrate of the non-native Cc1FatB1 acyl-ACP thioesterase expressed in the cell, might accumulate in fatty acid-producing cells that express the C12-C16-preferring Cc1FatB1 acyl-ACP thioesterase, possibly limiting fatty acid production by feedback inhibition, and that overexpressing a C18-preferring LPAAT gene might limit any inhibitory effects by reducing the C18:0 acyl-ACP pool through LPAAT-catalyzed condensation of C18:0 acyl-ACP with lysophosphatidic acid to generate phosphatidic acid. *See, e.g.,* Figure 1. In addition to limiting feedback inhibition of fatty acid synthesis, the inventors considered the modification might help to stabilize the cell membrane by promoting membrane lipid biosynthesis.

### 1.1. LPAAT vector construction

An expression construct was designed that included a gene encoding an LPAAT with a preference for C18:0 acyl-ACPs. The pSGI-NB55 vector (SEQ ID NO:27; Figure 2) was constructed to overexpress the Lysophophatidic Acid Acyl Transferase (LPAAT) gene sll1752 (Genbank Accession Number, Gene ID ; SEQ ID NO:1) from *Synechocystis* sp. PCC 6803 using a trcY promoter (SEQ ID NO:28). The sll1752 gene was amplified from genomic *Synechocystis* DNA using primers NB395 (SEQ ID NO:29) and NB396 (SEQ ID NO:30) which contained approximately 15 bp sequence overlap with cyanobacterial integration vector pSGI-YC63 (SEQ ID NO:31). pSGI-YC63 contained a spectinomycin marker for selection, homologous "RS2 up" (SEQ ID NO:32) and "RS2 down" (SEQ ID NO:33) arms for integration in the RS2 site of the *Synechocystis* sp. PCC 6803 genome, the lacIQ repressor for the trcY promoter (SEQ ID NO:28) to drive sll1752 expression, and a pUC origin of replication for propagation of the vector in *E. Coli.* The pSGI-YC63 vector (SEQ ID NO:31) was PCR amplified to produce a linearized nucleic acid molecule using primers NB393 (SEQ ID NO:34) and NB394 (SEQ ID NO:35). Purified PCR product for the vector backbone and the sll1752 gene was combined in *E. coli* using the Quick PCR Cloning Kit and propagated in Alpha-Select Gold *E. Coli* cells.

### 1.2. Thioesterase Cc1FatB1 vector construction

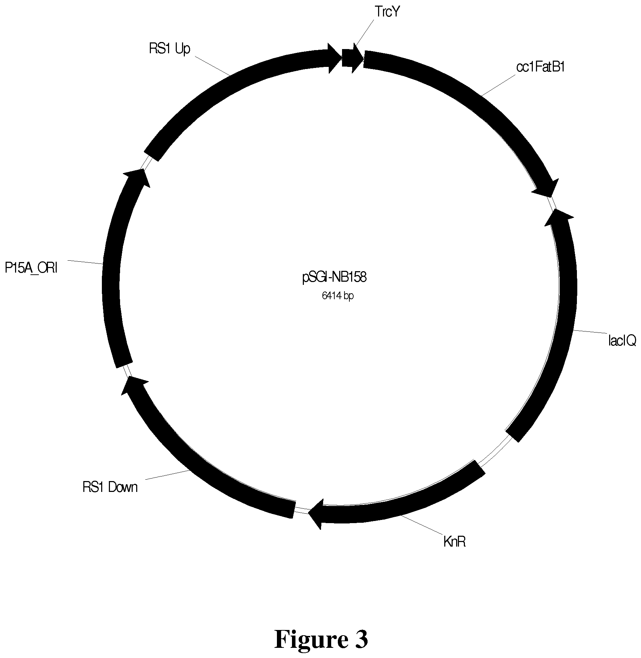

A thioesterase gene from the higher plant *Cuphea carthagenensis,* Cc1FatB1 (US 2009/0298143; WO 2009/076559), was cloned into a vector designed for promoting gene integration into the RS1 site of the *Synechocystis* sp. PCC 6803 genome. The pSGI-NB158 acyl-ACP thioesterase construct (SEQ ID NO: 36; Figure 3) contained a P15A origin of replication for propagation in *E. Coli,* amplified from vector pACYC184 using primers NB470 (SEQ ID NO:37) and NB471 (SEQ ID NO:38); a kanamycin resistance marker for selection, amplified from cyanobacterial integration vector SGI-YC28 using primers NB466 (SEQ ID NO:39) and NB467 (SEQ ID NO:40), "RS1 up" (SEQ ID NO:41) and "RS1 down" (SEQ ID NO:42) fragments for homologous recombination in *Synechocystis* 6803, amplified using primers NB466 and NB467, respectively; the trcY promoter (SEQ ID NO:28), and a lacIQ repressor for the trcY promoter-driven *Cuphea* Cc1FatB1 thioesterase gene (SEQ ID NO:43) encoding an N-terminally truncated acyl-ACP thioesterase (SEQ ID NO:44) having a substrate preference for C12- C16 acyl-ACPs. Primers NB 462 (SEQ ID NO:45) and NB 463 (SEQ ID NO:46) were used to amplify the trcY-Cc1FatB1 thioesterase cassette from a prior expression vector. The lac IQ fragment was amplified using primers NB464 (SEQ ID NO:47) and NB 465 (SEQ ID NO:48) from another vector, and the kanamycin resistance gene was amplified using primers NB466 (SEQ ID NO:49) and NB467 (SEQ ID NO:50) from vector pSGI-YC28 (see Table 1). Primers were designed to include sequences that would be incorporated into the termini of the final PCR products such that the amplified fragments would have 10-20 bps terminal homology to nucleic acid fragments to be cloned adjacent to the fragments. Cloning was performed using the Quick PCR Cloning Kit (BPS Biosciences, San Diego). The clones were transformed and propagated in Alpha-Select Gold competent *E. coli* cells (Bioline, Tauton).

**Table 1. Primers Used in Vector Construction**

| | | |
|---|---|---|
| NB460 | GTTTATCACAGTTAAATTATTGCTGAAGCGGAATCCCTGG | SEQ ID NO:58 |
| NB461 | CAGCCTCAGGCCATATAACCATCAAAGCCATAGT TGG C | SEQ ID NO:59 |
| NB462 | | SEQ ID NO:45 |
| NB463 | | SEQ ID NO:46 |
| NB464 | | SEQ ID NO:47 |
| NB465 | ATATTTTTATCTTGTGGTCCGGAATTCGCCCTTCTAAGC | SEQ ID NO:48 |
| NB466 | | SEQ ID NO:49 |
| NB467 | GGTGCCATCCATACCGGGCCGCCGTCCCGTCAAG | SEQ ID NO:50 |
| NB468 | GGGACGGCGGCCCGGTATGGATGGCACCGATG | SEQ ID NO:60 |
| NB469 | | SEQ ID NO:64 |
| NB470 | AGAGAATGGTCCCCCATGTTCAGCTACTGACGGGGTG | SEQ ID NO:37 |
| NB471 | | SEQ ID NO:38 |
| NB1 | CTCGAGCCCCCGTGCTATGACTAGC | SEQ ID NO:52 |
| NB9 | CTCGAGCCCGGAACGTTTTTTGTACCCC | SEQ ID NO:53 |
| NB87 | CAATTGGCATGCACCCCTATTTGTTTATTTTTCTAAATAC | SEQ ID NO:61 |
| NB88 | | SEQ ID NO:62 |
| NB5 | GATCGACGTATAAACTGTCAACATATTTCTGCAAG | SEQ ID NO:63 |
| NB6 | CTCGAGTGTGGTGCCGGAGGTGTAG | SEQ ID NO:65 |
| NB393 | TTCTAGATATCTGCAGGCCTAAGCTTTATGC | SEQ ID NO:34 |
| NB394 | TTTTTTTCCTCCTTAGTGTGAAATTGTTATCCGC | SEQ ID NO:35 |
| NB395 | CACTAAGGAGGAAAAAAAATGACCAATTCTCCCCTGGCG | SEQ ID NO:29 |
| NB396 | CCTGCAGATATCTAGAATCACGAAGCGGCGATCG | SEQ ID NO:30 |

### 1.3. Acyl-ACP synthetase knockout vector construction

The pSGI-NB19 vector (SEQ ID NO:51) was used to knock out the slr1609 Acyl-ACP synthetase gene of *Synechocystis* sp. PCC 6803. Primers NB1 (SEQ ID NO:52) and NB9 (SEQ ID NO:53) were used to amplify the slr1609 gene fragment from *Synechocystis* sp. PCC 6803. This fragment was ligated into the TOPO vector PCR2.1 (Life Technologies, Carlsbad, CA) via the manufacturer's protocol, and the plasmid construct was propagated in TOP10 *E. Coli* cells (Life Technologies, Carlsbad, CA). This plasmid was cut with and Mfe1 restriction Enzyme (New England Biolabs, Ipswich, MA). The GmR fragment was PCR amplified from a pAW4 plasmid with primers NB87 and NB88 containing Mfe1 restrictions sites used to cut the ends of the PCR fragment. This was then ligated into the TOPO vector containing the slr1609 fragment from the previous step to create plasmid pSGI-NB19 (SEQ ID NO:51).

### 1.4. Production of free fatty acids

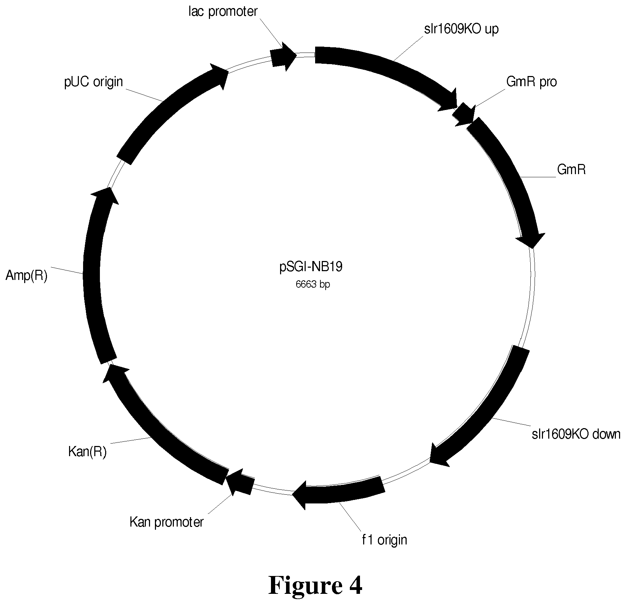

*Synechocystis* sp. PCC 6803 was transformed with the pSGI-NB55 LPAAT gene expression construct (SEQ ID NO:27; Figure 2), the pSGI-NB158 acyl-ACP thioesterase expression construct (SEQ ID NO: 36; Figure 3), or the pSGI-NB19 acyl-ACP synthetase gene knock out (AAS KO) construct (SEQ ID NO:51; Figure 4) or the combinations of pSGI-NB55 and pSGI-NB158 or pSGI-NB55 and pSGI-NB19 essentially according to Zang et al. (2007) J. Microbiology 45:241-245. Briefly, cells were grown under constant light to an optical density 730 (O.D.₇₃₀) of approximately 0.7 to 0.9 (an OD₇₃₀ of ∼0.25 corresponds to ∼1 x 10⁸ cells/ml) and harvested by centrifugation at ∼2,000g for ∼15 mins at room temperature (∼20-25°C). The cell pellet was resuspended in approximately 0.3 times the growth volume of fresh BG-11 medium and used immediately for transformation. About 1 microgram of plasmid DNA (containing pSGI-NB55 construct (SEQ ID NO:27) that included an LPAAT gene, or the pSGI-NB158 acyl-ACP thioesterase construct (SEQ ID NO:36), or the AAS knock out construct pSGI-NB19 (SEQ ID NO:51)) was added to ∼0.3 ml of cells, gently mixed, and incubated approximately 5 hours with illumination at ∼30°C without agitation. Cells were spread on a filter (Whatmann Nuclepore Polycarbonate Track-Etched membrane, PC ∼47 mm, ∼0.2 micron) positioned on a ∼50 ml BG-11 agar plates and allowed to recover for about 16 to 24 hours under light, after which the filter was lifted and placed on a fresh BG-11 plate containing spectinomycin (10 µg/ml) for selection of the LPAAT expression construct pSGI-NB55 (SEQ ID NO:27), kanamycin (10 µg/ml) for selection of the acyl-ACP thioesterase expression construct pSGI-NB158 (SEQ ID NO: 36), or gentamycin (5 µg/ml) for selection of the acyl-ACP synthetase knock out construct pSGI-NB19 (SEQ ID NO:51) to select for transformants. Resulting colonies were screened further for the presence of the thioesterase genes by PCR using the primers used to generate the gene fragments.

**Table 2. BG-11 Medium**

| | | |
|---|---|---|
| NaNO₃ | 1.5 g | |
| K₂HPO₄ | 0.04 g | |
| MgSO₄ ^{∗} 7H₂O | 0.075 g | |
| CaCl₂ ^{∗} 2H₂O | 0.036 g | |
| Citric acid | 6.0 mg | |
| Ferric ammonium citrate | 6.0 mg | |
| EDTA | 1.0 mg | |
| Na₂CO₃ | 0.02 g | |
| Trace Metal Mix A5^{#} | 1.0 ml | |
| Agar (if needed) | (up to) 10.0 g | |
| Distilled water | 1.0 L | |
| ^{#}Trace Metal Mix A5 | H₃BO₃ | 2.86 g |
| | MnCl₂ ^{∗} 4H₂O | 1.81 g |
| | ZnSO₄ ^{∗} 7H₂O | 0.22 g |
| | Na₂MoO₄ ^{∗} 2H₂O | 0.39 g |
| | CuSO₄ ^{∗} 5H₂O | 0.080 g |
| | Co(NO₃)₂ ^{∗} 6H₂O | 49.4 mg |
| | Distilled water | to 1.0L |

For production of fatty acids, patches of *Synechocystis* cells transformed with desired constructs (based on size selection using specific primers for PCR screening) were scraped and grown in 25mL of BG-11 medium (ATCC 616, as shown in Table 2; component weights are approximate; final pH 7.1; autoclaved at about 121°C for about 15 mins.) with appropriate antibiotics. Liquid cultures were grown in shake flasks at 30°C, ∼65 µmol/m²/s light, about 215 rpm with the supply of ∼5% CO₂ until turbid (approximately 3 days). The O.D. (730 nm) of each culture was measured in order to determine the volume of BG-11 medium for resuspending the cells for production cultures. Resuspensions were performed to provide all cultures at or above O.D. 1.1 (730 nm). To ensure that the desired starting O.D. 1.1 (730 nm) for induction was achieved, cultures were spun down at 2,000 g for 10 minutes (20-25°C) and resuspended in 9-10 mL BG-11 medium supplemented with 10 mg/mL BSA. O.D. readings were measured again after resuspension and diluted to O.D 1.1 (730 nm), again in BG-11 medium supplemented with 10 mg/mL BSA. BSA supplementation reduced fatty acid toxicity that could cause bleaching of the production cultures. Appropriate antibiotics were added to the cultures and the cells were induced with IPTG on day 1 in ∼4 ml screw thread glass vials with gas permeable tape for sealing, growing at about 30°C, ∼65 µmol/m²/s light, shaking at about 215 rpm with a supply of ∼5% CO₂. The BG-11 medium does not provide a reduced carbon source that can be used as an energy source for the growth of the cells. Rather, the cells were grown phototrophically using CO₂ as substantially the sole carbon source, using light as the energy sources, and incorporating carbon from CO₂ into biomolecules, including fatty acids. The final concentration of ∼1 mM IPTG was added to all samples to induce the free fatty acid production. The whole vials were submitted for GC-free fatty acid analysis.

### 1.5. Analysis of free fatty acids

Free fatty acids were analyzed by gas chromatography with flame ion detection (GC-FID). About 1.0 mL of the *Synechocystis* cultures were added to ∼4 mL glass GC vials with PTFE-lined caps (National Scientific). About eighty-four microliters of an internal standard (I.S.) set that included the free fatty acids C9:0, C13:0, and C17:0, each at about 600 µg/ml in hexane, were added to the culture sample followed by about 83 microliters of ∼50% H₂SO₄, about 167 microliters of ∼5M NaCl, and about 1.4 milliliters of hexane. The final concentration of each I.S. was about 50 µg/mL. The fatty acids for making the internal standard set were purchased either from Fluka or Nu Chek Prep. The cultures were then vortexed on a Multi-tube vortexer at about 2,500 rpm for about 30 minutes. The vials were finally centrifuged for about 3 minutes at about 2,000rpm, in order to provide good separation between organic and aqueous phases. The hexane layer was analyzed by gas chromatography (GC).

*Synechocystis* fatty acid samples were analyzed on an Agilent model 7890A GC/FID that included a J&W Scientific DB-FFAP capillary column (∼10 m length, ∼0.10 mm internal diameter, ∼0.10 µm film thickness). For analysis of cyanobacterial samples, the GC oven was programmed as follows: about 120°C for about 0.1 minute, then heated at about 40°C/minute to about 240°C (hold ∼3.5 minutes). The injector temperature was kept at about 260°C, and a ∼30:1 split ∼0.9 µl injection was used. Helium was used as a carrier gas at a flow rate of about 0.599 mL/minute. The analytes were identified by comparison of retention times to individually injected standards. The calibration range for the analytes was about 2 µg/ml to about 200 µg/ml for C8:0 - C16:1 fatty acids and about 0.625 µg/ml to about 50 µg/ml for C18:0-C18:3 fatty acids.

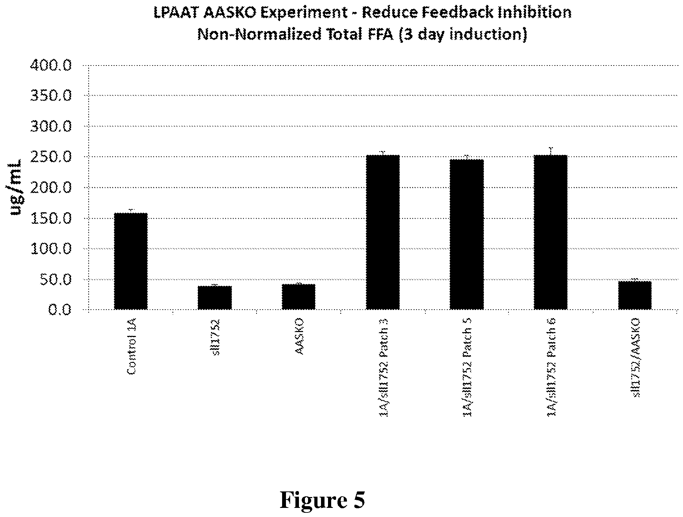

As shown in Figure 5, a 60% increase in total FFA production over the Cc1FatB1 thioesterase-containing strain (Control 1A) was detected for the combination Cc1FatB1 thioesterase and LPAAT-containing strain (sll1752). Over-expression of sll1752 alone, however, resulted in a greater than 3-fold drop in total FFA as compared to Control 1A. A similar drop in total FFA as compared to Control 1A was observed in strains with an attenuated acyl-ACP synthetase gene (AASKO) or a combination of sll1752 and AASKO.

Figure 6 shows the chain lengths of the fatty acids produced by the strains expressing the acyl-ACP thioesterase Cc1FatB1 with and without expression of the sll1752 LPAAT. The substrate preference for C14 and C16, as well as C12 acyl substrates is clearly seen in the 1A control and is maintained in the strain that co-expressed the C18-preferring LPAAT gene; however the yield of free fatty acids is significantly increased.

## Claims

1. A recombinant cyanobacterium transformed with a non-native nucleic acid sequence encoding an acyl-ACP thioesterase derived from a higher plant operably linked to a heterologous promoter and a non-native nucleic acid sequence encoding a lysophosphatidic acid acyltransferase (LPAAT) derived from a cyanobacterium operably linked to a heterologous promoter, wherein the cyanobacterium produces at least one free fatty acid or at least one fatty acid derivative, and wherein the LPAAT has at least 85% identity to SEQ ID NO:2, and the thioesterase has at least 85% identity to SEQ ID NO:44.

2. The recombinant cyanobacterium of claim 1, wherein the LPAAT transfers acyl groups onto C16 or C18 substrates.

3. The recombinant cyanobacterium of claim 1 or 2, wherein the recombinant cyanobacterium further comprises a construct for downregulating the expression of an endogenous acyl-ACP synthetase gene or an acyl-CoA synthetase gene, wherein the construct is an antisense construct, a micro RNA construct, a ribozyme construct, or a gene disruption construct.

4. The recombinant cyanobacterium of any one of the previous claims, wherein the recombinant cyanobacterium further comprises a construct for downregulating the expression of an endogenous acyl-ACP synthetase gene or an acyl-CoA synthetase gene, wherein the construct is an antisense RNA construct, a microRNA construct, or a ribozyme construct.

5. The recombinant cyanobacterium of any one of claims 1 to 4, wherein the recombinant cyanobacterium is of the genus *Agmenellum, Anabaena, Anabaenopsis, Anacystis, Aphanizomenon, Arthrospira, Asterocapsa, Borzia, Calothrix, Chamaesiphon, Chlorogloeopsis, Chroococcidiopsis, Chroococcus, Crinalium, Cyanobacterium, Cyanobium, Cyanocystis, Cyanospira, Cyanothece, Cylindrospermopsis, Cylindrospermum, Dactylococcopsis, Dermocarpella, Fischerella, Fremyella, Geitleria, Geitlerinema, Gloeobacter, Gloeocapsa, Gloeothece, Halospirulina, Iyengariella, Leptolyngbya, Limnothrix, Lyngbya, Microcoleus, Microcystis, Myxosarcina, Nodularia, Nostoc, Nostochopsis, Oscillatoria, Phormidium, Planktothrix, Pleurocapsa, Prochlorococcus, Prochloron, Prochlorothrix, Pseudanabaena, Rivularia, Schizothrix, Scytonema, Spirulina, Stanieria, Starria, Stigonema, Symploca, Synechococcus, Synechocystis, Tolypothrix, Trichodesmium, Tychonema* or *Xenococcus.*

6. The recombinant cyanobacterium of any one of the previous claims, wherein the cyanobacterium produces: at least 10% more fatty acid product compared to an otherwise identical cyanobacterium lacking the non-native nucleic acid sequence encoding a lysophosphatidic acid acyltransferase (LPAAT), and optionally wherein the fatty acid product is one or more of a fatty acid, a fatty aldehyde, a fatty alcohol, a fatty acid ester, a wax ester, or a hydrocarbon.

7. The recombinant cyanobacterium of claim 6, wherein the fatty acid product is a C8-C24 fatty acid product, preferably a C12-C18 fatty acid product or a C12-C16 fatty acid product, optionally wherein the fatty acid product is saturated.

8. A method of producing a fatty acid product comprising culturing the recombinant cyanobacterium of any one of claims 1-7 under conditions in which at least one fatty acid product is produced, wherein the fatty acid product is one or more of a free fatty acid, a fatty aldehyde, a fatty alcohol, a fatty acid ester, a wax ester, or a hydrocarbon, and wherein the fatty acid product is secreted into the medium.

9. The method of claim 8, wherein the recombinant cyanobacterium is cultured photoautotrophically, and wherein the culture medium does not include a substantial amount of reduced carbon.

10. The method of claim 9, wherein expression of one or both of the non-native nucleic acid sequence that encodes an LPAAT and the non-native nucleic acid sequence that encodes a thioesterase is induced.

11. The method of any one of claims 8-10, wherein at least one fatty acid product produced is a C8-C24 free fatty acid, fatty alcohol, or fatty aldehyde.

## Patentansprüche

1. Rekombinantes Cyanobakterium, das mit einer von einer höheren Pflanze stammenden, funktionsfähig mit einem heterologen Promotor verbundenen nicht-nativen Nukleinsäuresequenz, die für eine Acyl-ACP-Thioesterase kodiert, und mit einer von einem Cyanobakterium stammenden, funktionsfähig mit einem heterologen Promotor verbundenen nicht-nativen Nukleinsäuresequenz, die für eine Lysophosphatidsäure-Acyltransferase (LPAAT) kodiert, transformiert ist, wobei das Cyanobakterium mindestens eine freie Fettsäure oder mindestens ein Derivat einer freien Fettsäure produziert, und wobei die LPAAT mindestens 85% Identität mit SEQ ID NO:2 hat und die Thioesterase mindestens 85% Identität mit SEQ ID NO:44 hat.

2. Rekombinantes Cyanobakterium nach Anspruch 1, wobei die LPAAT Acylgruppen auf C16- oder C18-Substrate transferiert.

3. Rekombinantes Cyanobakterium nach Anspruch 1 oder 2, wobei das rekombinante Cyanobakterium außerdem ein Konstrukt zum Herabregulieren der Expression eines endogenen Acyl-ACP-Synthetasegens oder eines Acyl-CoA-Synthetasegens aufweist, wobei das Konstrukt ein Antisense-Konstrukt, ein Mikro-RNA-Konstrukt, ein Ribozym-Konstrukt oder ein Genunterbrechungskonstrukt ist.

4. Rekombinantes Cyanobakterium nach einem der vorangehenden Ansprüche, wobei das rekombinante Cyanobakterium außerdem ein Konstrukt zum Herabregulieren der Expression eines endogenen Acyl-ACP-Synthetasegens oder eines Acyl-CoA-Synthetasegens aufweist, wobei das Konstrukt ein Antisense-RNA-Konstrukt, ein Mikro-RNA-Konstrukt oder ein Ribozym-Konstrukt ist.

5. Rekombinantes Cyanobakterium nach einem der Ansprüche 1 bis 4, wobei das rekominante Cyanobakterium aus der Gattung *Agmenellum, Anabaena, Anabaenopsis, Anacystis, Aphanizomenon, Arthrospira, Asterocapsa, Borzia, Calothrix, Chamaesiphon, Chlorogloeopsis, Chroococcidiopsis, Chroococcus, Crinalium, Cyanobacterium, Cyanobium, Cyanocystis, Cyanospira, Cyanothece, Cylindrospermopsis, Cylindrospermum, Dactylococcopsis, Dermocarpella, Fischerella, Fremyella, Geitleria, Geitlerinema, Gloeobacter, Gloeocapsa, Gloeothece, Halospirulina, lyengariella, Leptolyngbya, Limnothrix, Lyngbya, Microcoleus, Microcystis, Myxosarcina, Nodularia, Nostoc, Nostochopsis, Oscillatoria, Phormidium, Planktothrix, Pleurocapsa, Prochlorococcus, Prochloron, Prochlorothrix, Pseud-anabaena, Rivularia, Schizothrix, Scytonema, Spirulina, Stanieria, Starria, Stigonema, Symploca, Synechococcus, Synechocystis, Tolypothrix, Trichodesmium, Tychonema* oder *Xenococcus* ist.

6. Rekombinantes Cyanobakterium nach einem der vorangehenden Ansprüche, wobei das Cyanobakterium produziert: mindestens 10% mehr Fettsäureprodukt im Vergleich zu einem ansonsten identischen Cyanobakterium, dem die nichtnative Nukleinsäuresequenz, die für eine Lysophosphatidsäure-Acyltransferase (LPAAT) kodiert, fehlt, und wobei optional das Fettsäureprodukt eines oder mehrere der Produkte Fettsäure, Fettaldehyd, Fettalkohol, Fettsäureester, Wachsester oder Kohlenwasserstoff ist.

7. Rekombinantes Cyanobakterium nach Anspruch 6, wobei das Fettsäureprodukt ein C8- bis C24-Fettsäureprodukt, bevorzugt ein C12- bis C18-Fettsäureprodukt oder ein C12- bis C16-Fettsäureprodukt ist, wobei optional das Fettsäureprodukt gesättigt ist.

8. Verfahren zur Produktion eines Fettsäureprodukts, aufweisend ein Kultivieren des rekombinanten Cyanobakteriums nach einem der Ansprüche 1 bis 7 unter Bedingungen, unter denen mindestens ein Fettsäureprodukt produziert wird, wobei das Fettsäureprodukt eines oder mehrere der Produkte freie Fettsäure, Fettaldehyd, Fettalkohol, Fettsäureester, Wachsester oder Kohlenwasserstoff ist, und wobei das Fettsäureprodukt in das Medium sezerniert wird.

9. Verfahren nach Anspruch 8, wobei das rekombinante Cyanobakterium photoautotroph kultiviert wird, und wobei das Kulturmedium keine substantielle Menge an reduziertem Kohlenstoff enthält.

10. Verfahren nach Anspruch 9, wobei die Expression der nicht-nativen Nukleinsäuresequenz, die für eine LPAAT kodiert, und/oder der nicht-nativen Nukleinsäuresequenz, die für eine Thioesterase kodiert, induziert wird.

11. Verfahren nach einem der Ansprüche 8 bis 10, wobei mindestens ein produziertes Fettsäureprodukt eine freie C8- bis C24-Fettsäure, ein Fettalkohol oder ein Fettaldehyd ist.

## Revendications

1. Cyanobactérie recombinante transformée avec une séquence d'acide nucléique non native codant pour une acyl-ACP thioestérase dérivée d'une plante supérieure en liaison fonctionnelle avec un promoteur hétérologue et une séquence d'acide nucléique non native codant pour une acide lysophosphatidique acyltransférase (LPAAT) dérivée d'une cyanobactérie en liaison fonctionnelle avec un promoteur hétérologue, où la cyanobactérie produit au moins un acide gras libre ou au moins un dérivé d'acide gras, et dans laquelle la LPAAT présente au moins 85 % d'identité avec SEQ ID NO: 2, et la thioestérase présente au moins 85 % d'identité avec SEQ ID NO: 44.

2. Cyanobactérie recombinante selon la revendication 1, dans laquelle la LPAAT transfère des groupes acyle sur des substrats C16 ou C18.

3. Cyanobactérie recombinante selon la revendication 1 ou 2, où la cyanobactérie recombinante comprend en outre un produit de recombinaison pour négativement réguler l'expression d'un gène d'acyl-ACP synthétase ou d'un gène d'acyl-CoA synthétase endogène, dans laquelle le produit de recombinaison est un produit de recombinaison antisens, un produit de recombinaison de microARN, un produit de recombinaison de ribozyme, ou un produit de recombinaison de perturbation génique.

4. Cyanobactérie recombinante selon l'une quelconque des revendications précédentes, où la cyanobactérie recombinante comprend en outre un produit de recombinaison pour négativement réguler l'expression d'un gène d'acyl-ACP synthétase ou d'un gène d'acyl-CoA synthétase endogène, dans laquelle le produit de recombinaison est un produit de recombinaison d'ARN antisens, un produit de recombinaison de microARN, ou un produit de recombinaison de ribozyme.

5. Cyanobactérie recombinante selon l'une quelconque des revendications 1 à 4, où la cyanobactérie recombinante est du genre *Agmenellum, Anabaena, Anabaenopsis, Anacystis, Aphanizomenon, Arthrospira, Asterocapsa, Borzia, Calothrix, Chamaesiphon, Chlorogloeopsis, Chroococcidiopsis, Chroococcus, Crinalium, Cyanobacterium, Cyanobium, Cyanocystis, Cyanospira, Cyanothece, Cylindrospermopsis, Cylindrospermum, Dactylococcopsis, Dermocarpella, Fischerella, Fremyella, Geitleria, Geitlerinema, Gloeobacter, Gloeocapsa, Gloeothece, Halospirulina, lyengariella, Leptolyngbya, Limnothrix, Lyngbya, Microcoleus, Microcystis, Myxosarcina, Nodularia, Nostoc, Nostochopsis, Oscillatoria, Phormidium, Planktothrix, Pleurocapsa, Prochlorococcus, Prochloron, Prochlorothrix, Pseudanabaena, Rivularia, Schizothrix, Scytonema, Spirulina, Stanieria, Starria, Stigonema, Symploca, Synechococcus, Synechocystis, Tolypothrix, Trichodesmium, Tychonema* ou *Xenococcus.*

6. Cyanobactérie recombinante selon l'une quelconque des revendications précédentes, où la cyanobactérie produit : au moins 10 % plus de produit d'acide gras par comparaison à une cyanobactérie autrement identique dépourvue de la séquence d'acide nucléique non native codant pour une acide lysophosphatidique acyltransférase (LPAAT), et facultativement dans laquelle le produit d'acide gras est un ou plusieurs d'un acide gras, un aldéhyde gras, un alcool gras, un ester d'acide gras, un ester de cire, ou un hydrocarbure.

7. Cyanobactérie recombinante selon la revendication 6, dans laquelle le produit d'acide gras est un produit d'acide gras en C8-C24, de préférence un produit d'acide gras en C12-C18 ou un produit d'acide gras en C12-C16, facultativement dans laquelle le produit d'acide gras est saturé.

8. Procédé de production d'un produit d'acide gras comprenant la mise en culture de la cyanobactérie recombinante selon l'une quelconque des revendications 1 à 7 dans des conditions dans lesquelles au moins un produit d'acide gras est produit, dans lequel le produit d'acide gras est un ou plusieurs d'un acide gras libre, un aldéhyde gras, un alcool gras, un ester d'acide gras, un ester de cire, ou un hydrocarbure, et dans lequel le produit d'acide gras est sécrété dans le milieu.

9. Procédé selon la revendication 8, dans lequel la cyanobactérie recombinante est mise en culture de manière photoautotrophe, et dans lequel le milieu de culture ne comprend pas de quantité substantielle de carbone réduit.

10. Procédé selon la revendication 9, dans lequel l'expression de l'une de la séquence d'acide nucléique non native qui code pour une LPAAT et de la séquence d'acide nucléique non native qui code pour une thioestérase, ou des deux, est induite.

11. Procédé selon l'une quelconque des revendications 8 à 10, dans lequel au moins un produit d'acide gras produit est un acide gras libre en C8-C24, un alcool gras, ou un aldéhyde gras.


### SEQUENCE LISTING

<110> ExxonMobil Research and Engineering Company
<120> ENHANCED PRODUCTION OF FATTY ACIDS AND FATTY ACID DERIVATIVES BY RECOMBINANT MICROORGANISMS
<130> 2012EM036PCT / 16244-000011/WO/PCT
<160> 57
<170> PatentIn version 3.5
<210> 1
   <211> 723
   <212> DNA
   <213> Synechocystis PCC6803
<400> 1
   atgaccaatt ctcccctggc gatcgaccct gctgtcattg gcccttccag ggtttccccc        60
   tggctaatca aattaattta tcccctaggc accaggtttt tacattggta ttttggcccg       120
   atcgccatcc atggtcagga gcatttaccc cgcagtggcc cgattattct ggcccctacc       180
   caccgttccc gttgggatgc aattttgctt tctttggccg ccggtcgggg ggtaactggt       240
   cgagacctcc gttttatggt ggcggtgacg gaagtgcagg gtttacaggg ttggtttatt       300
   cgccatttgg ggggatttcc cgttgacgtg aaaaggccgg aaatcagtag tgtgagttat       360
   agtgtgcagt tactccaagc aggggaaatg ttggtcattt ttcctgaggg gggcattttt       420
   cgggatcaac acactgtcca tcccctcaaa cggggtattg ggcgcattgc catggaagtt       480
   tgtaagcaaa accccaacac tgacattaaa gtgatcccag tgacgatcgc ctacagtgac       540
   ccctacccag gcaaaggcac ttcagtggaa attaattttg gccagggcat cgccgctagg       600
   gactacgacc ccagcaccat caaggctagc tcccaaaaac tgacccgttg tttggcccat       660
   agaatgcaga acctttacag ttccgagaat gtctctctgt gcgaggcgat cgccgcttcg       720
   tga                                                                     723
<210> 2
   <211> 240
   <212> PRT
   <213> Synechocystis PCC6803
<400> 2
   Met Thr Asn Ser Pro Leu Ala Ile Asp Pro Ala Val Ile Gly Pro Ser
   1               5                   10                  15
   Arg Val Ser Pro Trp Leu Ile Lys Leu Ile Tyr Pro Leu Gly Thr Arg
               20                  25                  30
   Phe Leu His Trp Tyr Phe Gly Pro Ile Ala Ile His Gly Gln Glu His
           35                  40                  45
   Leu Pro Arg Ser Gly Pro Ile Ile Leu Ala Pro Thr His Arg Ser Arg
   50 55 60
   Trp Asp Ala Ile Leu Leu Ser Leu Ala Ala Gly Arg Gly Val Thr Gly
   65 70 75 80
   Arg Asp Leu Arg Phe Met Val Ala Val Thr Glu Val Gln Gly Leu Gln
   85 90 95
   Gly Trp Phe Ile Arg His Leu Gly Gly Phe Pro Val Asp Val Lys Arg
   100 105 110
   Pro Glu Ile Ser Ser Val Ser Tyr Ser Val Gln Leu Leu Gln Ala Gly
   115 120 125
   Glu Met Leu Val Ile Phe Pro Glu Gly Gly Ile Phe Arg Asp Gln His
   130 135 140
   Thr Val His Pro Leu Lys Arg Gly Ile Gly Arg Ile Ala Met Glu Val
   145 150 155 160
   Cys Lys Gln Asn Pro Asn Thr Asp Ile Lys Val Ile Pro Val Thr Ile
   165 170 175
   Ala Tyr Ser Asp Pro Tyr Pro Gly Lys Gly Thr Ser Val Glu Ile Asn
   180 185 190
   Phe Gly Gln Gly Ile Ala Ala Arg Asp Tyr Asp Pro Ser Thr Ile Lys
   195 200 205
   Ala Ser Ser Gln Lys Leu Thr Arg Cys Leu Ala His Arg Met Gln Asn
   210 215 220
   Leu Tyr Ser Ser Glu Asn Val Ser Leu Cys Glu Ala Ile Ala Ala Ser
   225 230 235 240
<210> 3
   <211> 236
   <212> PRT
   <213> Cyanothece PCC 8801
<400> 3
   Met Asn Gln Ser Thr Thr Ser Asn Ser Ile Glu Ser Ser Ile Asn Pro
   1 5 10 15
   Trp Leu Ile Arg Leu Val Tyr Pro Leu Gly Cys Ser Leu Ile Met Pro
   20 25 30
   Leu Phe Phe Gly Arg Ile Thr Ile Ser Gly Gln Glu Asn Ile Pro Thr
   35 40 45
   Thr Gly Pro Val Ile Val Ala Pro Thr His Arg Ser Arg Trp Asp Ala
   50 55 60
   Leu Ile Val Pro Gln Ala Val Gly Arg Leu Val Ser Gly Arg Asp Leu
   65 70 75 80
   Arg Phe Met Val Met Ser Thr Glu Met Thr Gly Leu Gln Gly Trp Leu
   85 90 95
   Ile Arg Arg Leu Gly Gly Phe Pro Ile Asp Val Lys Arg Pro Gly Leu
   100 105 110
   Asp Ser Leu Glu His Ser Val Ser Ile Leu Lys Gln Gly Glu Met Leu
   115 120 125
   Val Ile Phe Pro Glu Gly Gly Ile Phe Arg Asp Asn His Val His Pro
   130 135 140
   Leu Lys Arg Gly Val Ala Arg Ile Ala Leu Glu Val Val Ser Gln Gln
   145 150 155 160
   Pro Asn Ser Gly Ile Lys Ile Leu Pro Val Ser Val Gln Tyr Thr Gln
   165 170 175
   Pro Tyr Pro Ala Trp Gly Thr Asp Val Ile Val Asn Ile Gly Gln Pro
   180 185 190
   Ile Asp Val Ala Lys Tyr Asn Pro Gln Arg Met Lys Ser Ser Ser Glu
   195 200 205
   Ala Leu Thr Asn Asp Leu Glu Ala Lys Leu Lys Asp Leu Tyr Gln Gly
   210 215 220
   Gln Lys Ala Leu Asn Leu Ala Glu Phe Val Gly Ser
   225 230 235
<210> 4
   <211> 251
   <212> PRT
   <213> Crocosphaera watsonii WH 8501
<400> 4
   Met Cys Asn Leu His Arg Glu Ile Val Phe Val Ala Phe Cys Leu Lys
   1 5 10 15
   Lys Met Asn Ile His Lys Asn Lys Ser Asp Arg Val Lys Ser Gln Ile
               20                  25                  30
   Ser Pro Trp Leu Ile Asn Ile Ala Tyr Pro Leu Gly Ser Lys Ile Val
           35                  40                  45
   Leu Pro Leu Tyr Phe Gly Ser Ile Thr Ile Asn Gly Gln Glu Asn Ile
       50                  55                  60
   Pro Thr Ser Gly Pro Ile Leu Ile Ala Pro Thr His Arg Ser Arg Trp
   65                  70                  75                  80
   Asp Ala Leu Ile Ile Pro Tyr Ala Val Gly Arg Ile Val Ser Gly Arg
                   85                  90                  95
   Asp Val Arg Phe Met Val Thr Ser Ser Glu Ile Thr Gly Ile Gln Gly
               100                 105                 110
   Trp Phe Ile Arg Arg Met Gly Gly Phe Pro Val Asp Leu Lys Arg Pro
           115                 120                 125
   Gly Ala Ser Ser Leu Glu His Ser Val Glu Ile Leu Lys Gln Gly Glu
       130                 135                 140
   Met Leu Val Ile Phe Pro Glu Gly Gly Ile Phe Arg Asp Lys Glu Val
   145                 150                 155                 160
   His Pro Leu Lys Arg Gly Val Ala Arg Ile Ala Leu Glu Val Glu Ser
                   165                 170                 175
   Gln Gln Pro Gly Cys Gly Met Lys Ile Leu Pro Val Ser Ile Glu Tyr
               180                 185                 190
   Thr Lys Pro Phe Pro Ser Trp Gly Thr His Ile Thr Val Asn Ile Gly
           195                 200                 205
   Cys Ser Leu Asp Val Ala Ser Tyr Asp Thr Thr Thr Leu Lys Arg Ser
       210                 215                 220
   Ser Gln Lys Leu Thr Gln Asp Leu Glu Ser His Leu Lys Trp Ile Phe
   225                 230                 235                 240
   Gln Gly Lys Leu Glu Gln Ile Val Ile Gln Lys
                   245                 250
<210> 5
   <211> 234
   <212> PRT
   <213> Cyanothece CCY0110
<400> 5
   Met Asn Ile Ser Thr Ile Lys Ser Asp Gln Val Lys Ser Arg Ile Ser
   1               5                   10                  15
   Pro Trp Leu Ile Arg Ile Thr Tyr Pro Leu Gly Ser Trp Leu Val Leu
               20                  25                  30
   Pro Leu Tyr Phe Gly Arg Ile Lys Ile Thr Gly Gln Glu Asn Ile Pro
           35                  40                  45
   Asp Thr Gly Pro Val Ile Ile Ala Pro Thr His Arg Ser Arg Trp Asp
       50                  55                  60
   Ala Leu Ile Ile Pro Tyr Ala Val Gly Arg Met Val Ser Gly Arg Asp
   65                  70                  75                  80
   Val Arg Phe Met Val Thr Ser Ser Glu Met Glu Gly Ile Gln Gly Trp
                   85                  90                  95
   Phe Ile Arg Arg Leu Gly Gly Phe Pro Val Asp Val Lys Arg Pro Gly
               100                 105                 110
   Pro Ser Ser Leu Glu His Ser Ile Glu Val Leu Lys Gln Gly Glu Met
           115                 120                 125
   Leu Val Ile Phe Pro Glu Gly Gly Ile Phe Arg Asp Thr Glu Val His
       130                 135                 140
   Pro Leu Lys Arg Gly Val Ala Arg Ile Ala Leu Asp Val Glu Ser Gln
   145                 150                 155                 160
   Gln Pro Gly Cys Gly Met Lys Ile Leu Pro Ile Gly Ile Glu Tyr Ser
                   165                 170                 175
   Gln Pro Phe Pro Ser Trp Gly Thr Asp Val Thr Val Asn Ile Gly Ser
               180                 185                 190
   Pro Leu Asp Val Ala Thr Tyr Asp Thr Thr Lys Met Lys Arg Ser Ser
           195                 200                 205
   Glu Lys Leu Thr Ile Asp Leu Glu Ser Asp Leu Lys Gln Val Phe His
       210                 215                 220
   His Gln Ser Glu Ala Ile Val Phe Gln Ser
                       225                 230
<210> 6
   <211> 236
   <212> PRT
   <213> Microcystis aeruginosa
<400> 6
   Met Ser Gln Ser Thr Ile Val Asp Glu Ser Leu Thr Ile Arg Lys Ser
   1               5                   10                  15
   Pro Ala Val Asn Ser Arg Ile Asn Pro Cys Leu Ile Arg Phe Cys Tyr
               20                  25                  30
   Phe Leu Gly Asn Tyr Leu Val Leu Pro Ala Phe Phe Ser Lys Ile Thr
           35                  40                  45
   Val Thr Gly Gln Glu Asn Ile Pro Leu Thr Gly Gly Val Ile Leu Ala
       50                  55                  60
   Pro Thr His Arg Ser Arg Trp Asp Ala Leu Ile Leu Pro Tyr Ala Val
   65                  70                  75                  80
   Gly Arg Leu Val Ser Gly Arg Asp Leu Arg Phe Met Val Ser Ala Asn
                   85                  90                  95
   Glu Ile Lys Gly Val Gln Gly Trp Phe Ile Arg Arg Leu Gly Gly Phe
               100                 105                 110
   Pro Val Asn Thr Asp His Pro Gly Met Gly Ser Leu Val His Ser Val
           115                 120                 125
   Glu Leu Leu Ala Ala Gly Glu Met Val Ala Ile Phe Pro Glu Gly Gly
       130                 135                 140
   Ile Cys Arg Asp Arg Val Val His Pro Leu Lys Pro Gly Val Ala Arg
   145                 150                 155                 160
   Ile Ala Leu Glu Val Lys Thr Met Lys Pro Asp Ala Asp Ile Lys Ile
                   165                 170                 175
   Leu Pro Val Ser Ile Ser Tyr Asn Gln Pro Tyr Pro Gly Trp Gly Ser
               180                 185                 190
   Glu Ala Thr Val Asn Ile Gly Gln Gly Ile Asn Val Leu Asp Tyr Gln
           195                 200                 205
   Gln Asn Ser Leu Lys Arg Glu Thr Val Arg Leu Thr Lys Thr Leu Ser
                   210                 215                 220
   Asp Arg Leu Lys Leu Leu His Glu Asp Gln Ser Pro
   225                 230                 235
<210> 7
   <211> 287
   <212> PRT
   <213> Nostoc PCC 7120
<400> 7
   Met Ile Glu Phe Phe Ser Val Ser Asp Thr His Gln Thr Gln Pro Thr
   1               5                   10                  15
   Asn Arg Leu Val His Pro Gln Val Ala Val Thr Thr Ser Arg Val Ser
               20                  25                  30
   Pro Trp Leu Ser Pro Val Leu Tyr Phe Val Gly Asn Tyr Phe Leu Leu
           35                  40                  45
   Pro Ser Phe Phe Gly Arg Ile Ser Ile Thr Gly Gln Glu Asn Ile Pro
       50                  55                  60
   Lys Thr Gly Pro Val Ile Leu Ala Pro Thr His Arg Ala Arg Trp Asp
   65                  70                  75                  80
   Ser Leu Leu Leu Pro Tyr Ala Thr Gly Arg Tyr Val Thr Gly Arg Asp
                   85                  90                  95
   Leu Lys Phe Met Val Thr Lys Thr Glu Cys Arg Gly Leu Gln Gly Trp
               100                 105                 110
   Phe Val Arg Arg Met Gly Gly Phe Pro Ile Asp Thr Gln His Pro Ala
           115                 120                 125
   Val Ser Thr Leu Arg His Ala Val Glu Leu Leu Gln Gln Gly Gln Met
       130                 135                 140
   Leu Val Ile Tyr Pro Glu Gly Asn Ile Phe Arg Asp Gly Lys Leu His
   145                 150                 155                 160
   Pro Leu Lys Ser Gly Ile Ser Arg Leu Ala Leu Ser Ala Glu Ser Ser
                   165                 170                 175
   His Pro Gly Leu Gly Val Lys Ile Leu Pro Ile Ser Ile Asn Tyr Ser
               180                 185                 190
   Gln Pro Tyr Pro Cys Trp Gly Thr Asp Val Ser Ile His Ile Gly Ser
   195 200 205
   Pro Ile Asn Val Gln Asp Tyr Thr Lys Gly Lys Val Lys Gln Asn Ala
   210 215 220
   Lys Arg Leu Thr Glu Asp Leu Ala Arg Asp Leu Gln Ser Leu Ser Asn
   225 230 235 240
   Pro Glu Leu Ala Phe Ser His His Asp Ala Ala Glu Val Ser Thr Ser
   245 250 255
   Pro Ile Val Ser Val Ser Asp Ser Thr Glu Arg Ser Pro Asn Val Arg
   260 265 270
   Gly Gly Ala Thr Ser Arg Lys Arg Glu Leu Leu Thr Ile Asp Tyr
   275 280 285
<210> 8
   <211> 309
   <212> PRT
   <213> Anabaena variabilis
<400> 8
   Met Ile Glu Phe Phe Ser Val Ser Asp Thr Arg Gln Thr Gln Pro Thr
   1               5                   10                  15
   Asn Thr Leu Phe Pro Pro Gln Val Ala Val Thr Thr Ser Lys Val Ser
               20                  25                  30
   Pro Trp Leu Ser Pro Val Leu Tyr Phe Val Gly Asn Tyr Leu Leu Leu
           35                  40                  45
   Pro Ser Phe Phe Gly Arg Ile Ser Ile Thr Gly Gln Glu Asn Ile Pro
       50                  55                  60
   Asn Thr Gly Pro Val Ile Leu Ala Pro Thr His Arg Ala Arg Trp Asp
   65                  70                  75                  80
   Ser Leu Leu Leu Pro Tyr Ala Thr Gly Arg Tyr Val Thr Gly Arg Asp
                   85                  90                  95
   Leu Lys Phe Met Val Thr Lys Thr Glu Cys Arg Gly Leu Gln Gly Trp
               100                 105                 110
   Phe Val Arg Arg Met Gly Gly Phe Pro Ile Asp Thr Gln His Pro Ala
           115                 120                 125
   Ile Ser Thr Leu Arg His Ala Val Glu Leu Leu Gln Gln Gly Gln Met
   130 135 140
   Leu Val Ile Tyr Pro Glu Gly Asn Ile Phe Arg Asp Gly Lys Leu His
   145 150 155 160
   Pro Leu Lys Ser Gly Ile Ser Arg Leu Ala Leu Ser Ala Glu Ser Ser
   165 170 175
   His Pro Gly Leu Gly Val Lys Ile Leu Pro Val Ser Ile Asn Tyr Ser
   180 185 190
   Gln Pro Tyr Pro Cys Trp Gly Thr Asp Val Ser Ile His Ile Gly Thr
   195 200 205
   Ala Ile Asn Val Gln Asp Tyr Thr Asn Gly Lys Val Lys Gln Asn Ala
   210 215 220
   Lys Arg Leu Thr Glu Asp Leu Ala Arg Asp Leu Gln Ser Leu Ser Asn
   225 230 235 240
   Pro Glu Leu Ala Phe Ser His His Asp Val Ala Glu Val Ser Asn Pro
   245 250 255
   Pro Lys Gly Lys Gly Asp Glu Gly Ala Glu Ala Val Glu Glu Gln Cys
   260 265 270
   Gly Leu Gly Leu Cys Pro Ser Gly Ala Ile Ala Glu Glu Gln Ala Ser
   275 280 285
   Ile Val Ser Val Ser Arg Thr Gly Ala Thr Ser Arg Arg Arg Glu Leu
   290 295 300
   Leu Thr Val Asp Tyr
   305
<210> 9
   <211> 256
   <212> PRT
   <213> Nostoc azollae
<400> 9
   Met Met Glu Phe Tyr Ser Ala Ser Thr Lys Pro Gln Ser Gln Ile Thr
   1               5                   10                  15
   Asn Thr Leu Val Asn Pro Lys Val Ala Cys Ser Thr Ser Arg Val Ser
               20                  25                  30
   Pro Trp Leu Thr Pro Leu Ala Tyr Leu Leu Gly Arg Asn Ile Val Leu
   35 40 45
   Pro Leu Phe Phe Gly Asp Ile His Ile Thr Gly Gln Glu Asn Ile Pro
   50 55 60
   Thr Ser Gly Pro Ile Ile Leu Ala Pro Thr His Arg Ser Arg Trp Asp
   65 70 75 80
   Ser Leu Leu Leu Pro Tyr Ala Thr Gly Arg Cys Val Thr Gly Arg Asp
   85 90 95
   Met Arg Phe Met Val Thr Ser Ser Glu Cys Lys Gly Val Gln Gly Trp
   100 105 110
   Phe Val Arg Arg Met Gly Gly Phe Pro Val Asp Thr Gln Arg Pro Ala
   115 120 125
   Ile Ala Thr Leu Arg His Thr Val Glu Leu Met Val Glu Gly Glu Met
   130 135 140
   Leu Val Ile Tyr Pro Glu Gly Gly Ile Tyr Arg Asp Gly Lys Val His
   145 150 155 160
   Ala Leu Lys Ser Gly Ile Ser Arg Leu Ala Leu Ser Ala Ala Ser Val
   165 170 175
   His Pro Gly Leu Glu Ile Lys Ile Leu Thr Val Gly Ile Asn Tyr Ser
   180 185 190
   Gln Pro Tyr Pro Asn Trp Gly Thr Asp Val Asn Ile His Ile Gly Lys
   195 200 205
   Pro Ile Lys Val Thr Asp Tyr Ile Ser Gly Cys Leu Lys Lys Glu Ala
   210 215 220
   Lys Arg Leu Thr Ala Asp Leu Thr Asn Gln Leu Gln Gln Leu Ser His
   225 230 235 240
   Gln Gly Leu Glu Val Lys Ser His Ala Phe Ala Glu Ile Pro Asn Ser
   245 250 255
<210> 10
   <211> 246
   <212> PRT
   <213> Raphidiopsis brookii
<400> 10
   Met Lys Glu Pro His Ser Ala Ser Thr Ser Phe Ala Asp Gln Ser Pro
   1               5                   10                  15
   Asn Ile Cys Leu Asp Pro Pro Phe Ser His Thr Arg Ser Trp Ile Ser
               20                  25                  30
   Pro Trp Leu Thr Pro Leu Ala Tyr Trp Leu Gly Tyr His Leu Val Ile
           35                  40                  45
   Pro Leu Phe Phe Gly Ser Ile His Val Glu Gly Gln Glu His Ile Pro
       50                  55                  60
   Ile Arg Gly Pro Val Ile Leu Ala Pro Thr His Arg Ser Arg Trp Asp
   65                  70                  75                  80
   Pro Leu Leu Leu Ala Tyr Ala Ala Gly Arg Tyr Ile Thr Gly Arg Asp
                   85                  90                  95
   Leu Arg Phe Met Val Met Ser Ser Glu Cys Arg Gly Ile Gln Gly Trp
               100                 105                 110
   Phe Ile Leu Ser Met Gly Gly Phe Ala Val Asn Leu Gln Arg Pro Gly
           115                 120                 125
   Ile Lys Ser Leu Arg His Val Ile Asp Leu Met Leu Gly Gly Glu Met
       130                 135                 140
   Leu Val Ile Tyr Pro Glu Gly Gly Ile Phe Arg Asp Gly Lys Ile His
   145                 150                 155                 160
   Pro Leu Lys Ser Gly Ile Ala Arg Leu Ser Ile Asn Ala Gln Ser Leu
                   165                 170                 175
   Asp Pro Asn Leu Asp Ile Lys Ile Ile Pro Val Ser Ile Asn Tyr Asp
               180                 185                 190
   Arg Pro Tyr Pro Asn Trp Gly Thr Lys Val Lys Ile Cys Ile Gly Glu
           195                 200                 205
   Pro Val Lys Val Ala Asp Tyr Met His Gly Cys Leu Lys Gln Asn Ser
       210                 215                 220
   Gln Tyr Leu Thr Arg Asp Leu Arg Ser Lys Leu Gln Glu Val Ala Asp
   225                 230                 235                 240
   Ser Gln Pro Val Ser Leu
                   245
<210> 11
   <211> 253
   <212> PRT
   <213> Microcoleus chthonoplastes
<400> 11
   Met Gln Leu Gln Ser Arg Glu Gln Asn Gln Thr Pro Ser Thr Leu Asp
   1               5                   10                  15
   Lys Pro Asp Ser Lys Thr Val Asn Ser Ala Lys Ile Val Pro Val Asn
               20                  25                  30
   Ser Asn Val Ser Pro Trp Leu Thr Ser Ile Phe Tyr Pro Leu Gly Arg
           35                  40                  45
   Arg Ile Leu Met Pro Leu Tyr Phe Gly Arg Leu Gln Val Thr Gly Gln
       50                  55                  60
   Asp Asn Ile Pro Lys Thr Gly Pro Val Ile Leu Ala Pro Thr His Arg
   65                  70                  75                  80
   Ser Arg Trp Asp Gly Leu Thr Met Ala Tyr Ala Ala Gly Lys Pro Val
                   85                  90                  95
   Thr Gly Arg Asp Leu Arg Phe Met Val Ser Gln Asp Glu Met Lys Gly
               100                 105                 110
   Leu Gln Gly Trp Phe Ile Arg Arg Leu Gly Gly Phe Pro Val Asn Thr
           115                 120                 125
   Lys His Pro Gly Ile Ser Ser Ile Arg His Ser Val Glu Leu Leu Arg
       130                 135                 140
   Arg Gly Glu Ala Leu Val Met Phe Pro Glu Gly Asn Ile Phe Arg His
   145                 150                 155                 160
   Gly Asp Val Asn Pro Leu Lys Pro Gly Met Ala Arg Ile Ala Leu Gln
                   165                 170                 175
   Ala Glu Ser Asn Gln Pro Gly Leu Gly Leu Lys Ile Val Pro Val Ser
               180                 185                 190
   Ile Arg Tyr Ser Asp Pro Val Pro Gln Trp Gly Ser Asp Ile Asp Ile
           195                 200                 205
   Gln Ile Gly Ser Ala Leu Asp Val Ala Lys Tyr Cys Asn Gln Ser Ala
       210                 215                 220
   Lys Lys Gly Ala Gln Gln Leu Thr Thr Asp Leu Glu Ile Ala Leu Lys
   225 230 235 240
   Gln Val Asp Gln Lys Leu Glu Val Ile Lys Glu Ala Val
   245 250
<210> 12
   <211> 255
   <212> PRT
   <213> Cylindrospermopsis raciborskii
<400> 12
   Met Lys Glu Ser His Ser Ala Ser Thr Ser Phe Ala Gly Gln Ser Pro
   1 5 10 15
   Asn Ile Cys Leu Asp Pro Pro Phe Ser His Thr Arg Ser Trp Ile Ser
   20 25 30
   Pro Trp Leu Thr Pro Leu Ala Tyr Trp Leu Gly Tyr Asn Leu Val Ile
   35 40 45
   Pro Ser Phe Phe Gly Arg Ile Asp Val Ala Gly Gln Gln His Ile Pro
   50 55 60
   Ile Lys Gly Pro Val Ile Leu Ala Pro Thr His Arg Ser Arg Trp Asp
   65 70 75 80
   Pro Leu Leu Leu Ala Tyr Ala Ala Gly Arg Tyr Ile Thr Gly Arg Asp
   85 90 95
   Leu Arg Phe Met Val Met Ser Ser Glu Cys Arg Gly Ile Gln Gly Trp
   100 105 110
   Phe Ile Leu Ser Met Gly Gly Phe Ala Val Asn Leu Gln Arg Pro Gly
   115 120 125
   Ile Arg Ser Leu Arg His Ala Val Asp Leu Met Leu Gly Gly Glu Met
   130 135 140
   Leu Val Ile Tyr Pro Glu Gly Gly Ile Phe Arg Asp Gly Lys Ile His
   145 150 155 160
   Pro Leu Lys Ser Gly Ile Ala Arg Leu Ser Ile Asn Ala Gln Ser Leu
   165 170 175
   Asp Pro Asn Leu Asp Ile Lys Ile Ile Pro Val Ala Ile Asn Tyr Asp
   180 185 190
   Arg Pro Tyr Pro Ser Trp Gly Thr Lys Val Lys Ile Arg Ile Gly Glu
           195                 200                 205
   Pro Val Lys Val Thr Asp Tyr Ile His Gly Cys Leu Lys Gln Asn Ala
       210                 215                 220
   Lys Tyr Leu Thr Arg Asp Leu Arg Ser Lys Leu Gln Glu Val Ala Asp
   225                 230                 235                 240
   Ser Gln Pro Asp Phe Ser Leu Arg Ala Asn Cys Leu Thr Asn Ser
                   245                 250                 255
<210> 13
   <211> 236
   <212> PRT
   <213> Oscillatoria PCC 6506
<400> 13
   Met Ile Thr Phe Asp Ser Thr Asn Ser Ser Leu Glu Thr Gln Met Pro
   1               5                   10                  15
   Ala Lys Ala Thr Ser Ile Asn Ser His Val Ser Pro Trp Leu Ala Pro
               20                  25                  30
   Leu Val Tyr Pro Leu Gly Arg Tyr Leu Ile Leu Pro Phe Tyr Phe Arg
           35                  40                  45
   Ser Ile Glu Val Leu Gly Gln Glu His Ile Pro Arg Glu Gly Pro Val
       50                  55                  60
   Ile Leu Ala Pro Thr His Arg Ser Arg Trp Asp Ser Gln Met Leu Pro
   65                  70                  75                  80
   Tyr Ser Thr Gly Arg Tyr Val Thr Gly Arg Asp Leu Arg Phe Met Val
                   85                  90                  95
   Ser Val Asn Glu Val Lys Gly Leu Gln Gly Trp Phe Ile Arg Arg Leu
               100                 105                 110
   Gly Gly Phe Pro Val Asp Pro Lys His Pro Ala Ile Ala Thr Leu Arg
           115                 120                 125
   His Gly Val Glu Leu Leu Leu Asp Gly Gln Met Phe Val Ile Phe Pro
       130                 135                 140
   Glu Gly Gly Ile Phe Gln Asp Asn Gln Val His Pro Leu Lys Pro Gly
   145                 150                 155                 160
   Leu Ala Arg Leu Ala Ile Gln Ala Glu Ser Ser His Pro Gly Leu Asp
   165 170 175
   Val Lys Ile Val Pro Met Ser Ile Arg Tyr His Pro Cys Ile Pro Arg
   180 185 190
   Trp Gly Ser Arg Val Lys Ile Thr Ile Gly Ser Pro Leu Ser Ala Ala
   195 200 205
   Asp Tyr Cys Thr Gly Ala Gly Lys Lys Asp Ala Gln Leu Leu Thr Ala
   210 215 220
   Asp Leu Glu Thr Ala Leu Lys Arg Ile Asp Arg Glu
   225 230 235
<210> 14
   <211> 250
   <212> PRT
   <213> Nodularia spumigena
<400> 14
   Met Glu Ile Tyr Thr Ser His Gln Thr Ser Gln Glu Thr Pro Ala Ser
   1 5 10 15
   Asn Lys Ala Asn Thr Lys Val Ala His Thr Thr Ser Arg Val Ser Pro
   20 25 30
   Trp Leu Ser Pro Leu Leu Tyr Leu Leu Gly Gln His Leu Leu Leu Pro
   35 40 45
   Phe Phe Phe Arg Gln Ile Glu Ile Thr Gly Gln Glu Asn Ile Pro Leu
   50 55 60
   Thr Gly Pro Val Ile Leu Ala Pro Thr His Arg Ser Arg Trp Asp Ser
   65 70 75 80
   Leu Leu Leu Pro Tyr Ala Thr Gly Arg Cys Val Thr Gly Arg Asp Leu
   85 90 95
   Arg Phe Met Val Thr Ile Asn Glu Cys Gln Gly Leu Gln Gly Trp Leu
   100 105 110
   Val Arg Ser Met Gly Gly Phe Pro Val Asn Pro Gln Arg Pro Ala Ile
   115 120 125
   Thr Thr Leu Arg His Ala Val Glu Leu Leu Gln Gln Gly Glu Ile Leu
   130 135 140
   Val Ile Tyr Pro Glu Gly Asn Ile Tyr Arg Asp Gly Lys Leu His Pro
   145                 150                 155                 160
   Leu Lys Pro Gly Ile Ala Arg Leu Ser Leu Thr Ala Glu Ser Ser His
                   165                 170                 175
   Pro Gly Leu Asp Val Lys Ile Leu Pro Val Ser Ile Asn Tyr Ser Gln
               180                 185                 190
   Pro Tyr Pro Gln Trp Gly Thr Ser Val Lys Ile Ser Ile Gly Thr Thr
           195                 200                 205
   Leu Asn Val Ala Asp Tyr Thr Gly Gly Lys Val Lys Gln Asn Ala Gln
       210                 215                 220
   Asn Leu Thr Ser Asp Leu Ser Leu Val Leu Gln Gln Leu Ser Pro Gln
   225                 230                 235                 240
   Glu Ser Val Ile Pro Glu Asn Val Asn Ser
                   245                 250
<210> 15
   <211> 242
   <212> PRT
   <213> Microcoleus vaginatus
<400> 15
   Met Pro His Thr Ala Thr Ile Ala Val Glu Ser Ser Lys Ser Ser Leu
   1               5                   10                  15
   Val Lys Pro Met Pro Ala Lys Val Ala Ser Asn Thr Ser Arg Val Ser
               20                  25                  30
   Pro Trp Leu Thr Ser Leu Leu Tyr Pro Leu Gly His Tyr Val Val Leu
           35                  40                  45
   Pro Phe His Phe Gly Lys Ile Glu Ile Thr Gly Gln Glu His Leu Pro
       50                  55                  60
   Lys Asp Gly Pro Val Ile Leu Ala Pro Thr His Arg Ser Arg Trp Asp
   65                  70                  75                  80
   Ala Leu Met Met Pro Tyr Ser Thr Gly Gln Lys Val Thr Gly Arg Asp
                   85                  90                  95
   Leu Arg Phe Met Val Thr Ala Asp Glu Val Lys Gly Leu Gln Gly Trp
               100                 105                 110
   Phe Ile Arg Asn Leu Gly Gly Phe Pro Val Asp Leu Lys His Pro Ala
           115                 120                 125
   Ile Gly Thr Leu Arg Tyr Gly Val Glu Leu Leu Leu Asp Arg Glu Met
       130                 135                 140
   Leu Val Ile Phe Pro Glu Gly Gly Ile Phe Gln Asp Gly Glu Val His
   145                 150                 155                 160
   Pro Ile Lys Pro Gly Leu Ala Arg Leu Ala Met Gln Ala Glu Leu Ser
                   165                 170                 175
   Lys Pro Gly Leu Gly Val Lys Ile Val Pro Met Ser Ile Arg Tyr Arg
               180                 185                 190
   Pro Arg Ile Pro Gln Trp Gly Cys Gln Val Lys Ile Ala Ile Gly Ser
           195                 200                 205
   Pro Ile Ser Val Ala Asp Tyr Ala Asn Gly Gly Gly Gly Lys Lys Glu
       210                 215                 220
   Ala Arg Leu Leu Ser Ala Gln Leu Glu Met Asp Leu Lys Lys Leu Asp
   225                 230                 235                 240
   Glu Ser
<210> 16
   <211> 245
   <212> PRT
   <213> Escherichia coli
<400> 16
   Met Leu Tyr Ile Phe Arg Leu Ile Ile Thr Val Ile Tyr Ser Ile Leu
   1               5                   10                  15
   Val Cys Val Phe Gly Ser Ile Tyr Cys Leu Phe Ser Pro Arg Asn Pro
               20                  25                  30
   Lys His Val Ala Thr Phe Gly His Met Phe Gly Arg Leu Ala Pro Leu
           35                  40                  45
   Phe Gly Leu Lys Val Glu Cys Arg Lys Pro Thr Asp Ala Glu Ser Tyr
       50                  55                  60
   Gly Asn Ala Ile Tyr Ile Ala Asn His Gln Asn Asn Tyr Asp Met Val
   65                  70                  75                  80
   Thr Ala Ser Asn Ile Val Gln Pro Pro Thr Val Thr Val Gly Lys Lys
   85 90 95
   Ser Leu Leu Trp Ile Pro Phe Phe Gly Gln Leu Tyr Trp Leu Thr Gly
   100 105 110
   Asn Leu Leu Ile Asp Arg Asn Asn Arg Thr Lys Ala His Gly Thr Ile
   115 120 125
   Ala Glu Val Val Asn His Phe Lys Lys Arg Arg Ile Ser Ile Trp Met
   130 135 140
   Phe Pro Glu Gly Thr Arg Ser Arg Gly Arg Gly Leu Leu Pro Phe Lys
   145 150 155 160
   Thr Gly Ala Phe His Ala Ala Ile Ala Ala Gly Val Pro Ile Ile Pro
   165 170 175
   Val Cys Val Ser Thr Thr Ser Asn Lys Ile Asn Leu Asn Arg Leu His
   180 185 190
   Asn Gly Leu Val Ile Val Glu Met Leu Pro Pro Ile Asp Val Ser Gln
   195 200 205
   Tyr Gly Lys Asp Gln Val Arg Glu Leu Ala Ala His Cys Arg Ser Ile
   210 215 220
   Met Glu Gln Lys Ile Ala Glu Leu Asp Lys Glu Val Ala Glu Arg Glu
   225 230 235 240
   Ala Ala Gly Lys Val
   245
<210> 17
   <211> 231
   <212> PRT
   <213> Marinobacter adhaerens
<400> 17
   Met Leu Arg Leu Leu Lys Ala Ala Trp Ile Leu Phe Trp Ala Ile Leu
   1 5 10 15
   Leu Thr Leu Ile Leu Phe Phe Pro Ile Val Ile Ala Ala Leu Leu Gly
   20 25 30
   Lys Arg Gly Asp Ala Ala Phe His Gly Thr Gln Ile Tyr Ala Trp Ile
   35 40 45
   Ile Leu Lys Val Cys Gly Ile Arg Leu Lys Val Arg Gly Arg Glu Asn
       50                  55                  60
   Ile Glu Pro Gly Gln Arg Tyr Val Ile Leu Ser Asn His Ala Ser Tyr
   65                  70                  75                  80
   Leu Asp Pro Pro Ala Leu Val Leu Ala Leu Gly Leu Gln Tyr Arg Trp
                   85                  90                  95
   Val Ile Lys Lys Glu Leu Arg Lys Val Pro Leu Phe Gly Leu Ala Leu
               100                 105                 110
   Glu Ala Ser Arg Asn Leu Phe Ile Asp Arg Ser Lys Gly Ser Asp Ala
           115                 120                 125
   Leu Glu Ser Ile Lys Arg Gly Val Gly Gln Leu Pro Asp Gly Thr Gly
       130                 135                 140
   Ile Leu Ile Phe Pro Glu Gly Thr Arg Ser Trp Asp Gly Lys Leu Leu
   145                 150                 155                 160
   Pro Phe Lys Lys Gly Gly Phe Val Ile Ala Gln Asp Gly Glu Leu Pro
                   165                 170                 175
   Ile Leu Pro Val Thr Ile Cys Gly Ser His Gln Arg Leu Pro Lys Gly
               180                 185                 190
   Ser Ala Ala Phe Ser Arg Gly Asp Ile Glu Ile Val Ile His Pro Pro
           195                 200                 205
   Met Ala Ser Gly Ala Leu Pro Leu Asp Asp Leu Met Thr Asp Val Arg
       210                 215                 220
   Asn Ser Ile Ala Ser Ser Leu
   225                 230
<210> 18
   <211> 332
   <212> PRT
   <213> Oryza sativa
<400> 18
   Met Gly Thr Leu Leu Arg Pro Arg Pro Leu Ala His Ala Ala Gly Ala
   1               5                   10                  15
   Gly Asp Ala Thr Pro Ser Thr Ala His Ala Val Val Val Ser Gly Gly
               20                  25                  30
   Arg Gly Arg Gly Val Glu Cys Gln Pro His Arg Val Arg Arg Arg Pro
           35                  40                  45
   Gly Pro Gln Val Ala Val Ala Thr Ala Ser Trp Arg Arg Arg Arg Glu
       50                  55                  60
   Thr Val Val Arg Ser Asp Phe Ala Ala Gly Gly Ala Ala Thr Met Gly
   65                  70                  75                  80
   Asp Ser Pro Gln Ala Leu Ser Asp Ile Asp Val Val Ser Arg Val Arg
                   85                  90                  95
   Gly Val Cys Phe Tyr Ala Val Thr Ala Val Ala Ala Ile Phe Leu Phe
               100                 105                 110
   Val Ala Met Val Val Val His Pro Leu Val Leu Leu Phe Asp Arg Tyr
           115                 120                 125
   Arg Arg Arg Ala Gln His Tyr Ile Ala Lys Ile Trp Ala Thr Leu Thr
       130                 135                 140
   Ile Ser Met Phe Tyr Lys Leu Asp Val Glu Gly Met Glu Asn Leu Pro
   145                 150                 155                 160
   Pro Asn Ser Ser Pro Ala Val Tyr Val Ala Asn His Gln Ser Phe Leu
                   165                 170                 175
   Asp Ile Tyr Thr Leu Leu Thr Leu Gly Arg Cys Phe Lys Phe Ile Ser
               180                 185                 190
   Lys Thr Ser Ile Phe Met Phe Pro Ile Ile Gly Trp Ala Met Tyr Leu
           195                 200                 205
   Leu Gly Val Ile Pro Leu Arg Arg Met Asp Ser Arg Ser Gln Leu Asp
       210                 215                 220
   Cys Leu Lys Arg Cys Val Asp Leu Val Lys Lys Gly Ala Ser Val Phe
   225                 230                 235                 240
   Phe Phe Pro Glu Gly Thr Arg Ser Lys Asp Gly Lys Leu Gly Ala Phe
                   245                 250                 255
   Lys Arg Gly Ala Phe Ser Val Ala Thr Lys Thr Gly Ala Pro Val Ile
               260                 265                 270
   Pro Ile Thr Leu Leu Gly Thr Gly Lys Leu Met Pro Ser Gly Met Glu
           275                 280                 285
   Gly Ile Leu Asn Ser Gly Ser Val Lys Leu Ile Ile His His Pro Ile
       290                 295                 300
   Glu Gly Asn Asp Ala Glu Lys Leu Cys Ser Glu Ala Arg Lys Val Ile
   305                 310                 315                 320
   Ala Asp Thr Leu Ile Leu Asn Gly Tyr Gly Val His
                   325                 330
<210> 19
   <211> 345
   <212> PRT
   <213> Glycine max
<400> 19
   Met Glu Val Thr Pro Leu Ser Ser Pro Ser Pro Ile His Arg Leu His
   1               5                   10                  15
   Leu Arg His Lys Glu Ala Arg Phe Leu Ala Val Pro Ser Thr Leu Leu
               20                  25                  30
   Cys Thr Arg Arg Gly Thr Thr Thr Tyr Val His Pro Ile Leu Arg Thr
           35                  40                  45
   Ser His Asn Ser Pro Pro Cys Ser Leu Gln Ala Ile Ser Lys Lys His
       50                  55                  60
   Glu Asn Val Ser Trp Leu Ser Val Ser Pro Lys Leu His Val Gln Asn
   65                  70                  75                  80
   Lys Phe Pro Arg Asp Val Val Val Arg Ser Glu Leu Thr Ala Ala Gly
                   85                  90                  95
   Ser Ala Gly Asp Gly Tyr Leu Leu Pro Glu Leu Lys Leu Glu Ser Lys
               100                 105                 110
   Val Arg Gly Val Cys Phe Tyr Val Val Thr Ala Phe Ser Ala Ile Phe
           115                 120                 125
   Leu Phe Met Leu Met Leu Val Gly His Pro Ser Val Leu Leu Phe Asp
       130                 135                 140
   Arg Tyr Arg Arg Met Phe His His Phe Val Ala Lys Val Trp Ala Ala
   145                 150                 155                 160
   Leu Thr Val Ala Pro Phe Tyr Lys Ile Glu Phe Glu Gly Leu Glu Asn
                   165                 170                 175
   Leu Pro Pro Pro Asp Thr Pro Ala Val Tyr Val Ser Asn His Gln Ser
   180 185 190
   Phe Leu Asp Ile Tyr Thr Leu Leu Thr Leu Gly Arg Ser Phe Lys Phe
   195 200 205
   Ile Ser Lys Thr Gly Ile Phe Leu Phe Pro Ile Ile Gly Trp Ala Met
   210 215 220
   Phe Leu Leu Gly Val Ile Pro Leu Lys Arg Met Asp Ser Arg Ser Gln
   225 230 235 240
   Leu Asp Cys Leu Lys Arg Cys Met Asp Leu Ile Lys Lys Gly Ala Ser
   245 250 255
   Val Phe Phe Phe Pro Glu Gly Thr Arg Ser Lys Asp Gly Lys Leu Gly
   260 265 270
   Thr Phe Lys Lys Gly Ala Phe Ser Val Ala Ala Lys Thr Asn Ala Pro
   275 280 285
   Val Val Pro Ile Ser Leu Ile Gly Thr Gly Gln Ile Met Pro Ala Gly
   290 295 300
   Lys Glu Gly Ile Val Asn Leu Gly Ser Val Lys Val Val Ile His Lys
   305 310 315 320
   Pro Ile Val Gly Lys Asp Pro Asp Met Leu Cys Lys Glu Ala Arg Lys
   325 330 335
   Thr Ile Ala Ser Val Leu Thr Gln Ser
   340 345
<210> 20
   <211> 344
   <212> PRT
   <213> Brassica napus
<400> 20
   Met Asp Val Ala Ser Ala Arg Gly Val Ser Ser His Pro Pro Tyr Tyr
   1 5 10 15
   Ser Lys Pro Ile Cys Ser Ser Gln Ser Ser Leu Ile Arg Ile Pro Ile
   20 25 30
   Ser Lys Gly Cys Cys Phe Ala Arg Ser Ser Asn Leu Ile Thr Ser Leu
   35 40 45
   His Ala Ala Ser Arg Gly Val Thr Arg Arg Thr Ser Gly Val Gln Trp
       50                  55                  60
   Cys Tyr Arg Ser Ile Arg Phe Asp Pro Phe Lys Val Asn Asp Lys Asn
   65                  70                  75                  80
   Ser Arg Thr Val Thr Val Arg Ser Asp Leu Ser Gly Ala Ala Thr Pro
                   85                  90                  95
   Glu Ser Thr Tyr Pro Glu Pro Glu Ile Lys Leu Ser Ser Arg Leu Arg
               100                 105                 110
   Gly Ile Cys Phe Cys Leu Val Ala Gly Ile Ser Ala Ile Val Leu Ile
           115                 120                 125
   Val Leu Met Ile Ile Gly His Pro Phe Val Leu Leu Phe Asp Arg Tyr
       130                 135                 140
   Arg Arg Lys Phe His His Phe Ile Ala Lys Leu Trp Ala Ser Ile Ser
   145                 150                 155                 160
   Ile Tyr Pro Phe Tyr Lys Thr Asp Ile Gln Gly Leu Glu Asn Leu Pro
                   165                 170                 175
   Ser Ser Asp Thr Pro Cys Val Tyr Val Ser Asn His Gln Ser Phe Leu
               180                 185                 190
   Asp Ile Tyr Thr Leu Leu Ser Leu Gly Gln Ser Tyr Lys Phe Ile Ser
           195                 200                 205
   Lys Thr Gly Ile Phe Val Ile Pro Val Ile Gly Trp Ala Met Ser Met
       210                 215                 220
   Met Gly Val Val Pro Leu Lys Arg Met Asp Pro Arg Ser Gln Val Asp
   225                 230                 235                 240
   Cys Leu Lys Arg Cys Met Glu Leu Val Lys Lys Gly Ala Ser Val Phe
                   245                 250                 255
   Phe Phe Pro Glu Gly Thr Arg Ser Lys Asp Gly Arg Leu Gly Pro Phe
               260                 265                 270
   Lys Lys Gly Ala Phe Thr Ile Ala Ala Lys Thr Gly Val Pro Val Val
           275                 280                 285
   Pro Ile Thr Leu Met Gly Thr Gly Lys Ile Met Pro Thr Gly Ser Glu
       290                 295                 300
   Gly Ile Leu Asn His Gly Asp Val Arg Val Ile Ile His Lys Pro Ile
   305 310 315 320
   Tyr Gly Ser Lys Ala Asp Val Leu Cys Glu Glu Ala Arg Asn Lys Ile
   325 330 335
   Ala Glu Ser Met Asn Leu Leu Ser
   340
<210> 21
   <211> 356
   <212> PRT
   <213> Arabidopsis thaliana
<400> 21
   Met Asp Val Ala Ser Ala Arg Ser Ile Ser Ser His Pro Ser Tyr Tyr
   1 5 10 15
   Gly Lys Pro Ile Cys Ser Ser Gln Ser Ser Leu Ile Arg Ile Ser Arg
   20 25 30
   Asp Lys Val Cys Cys Phe Gly Arg Ile Ser Asn Gly Met Thr Ser Phe
   35 40 45
   Thr Thr Ser Leu His Ala Val Pro Ser Glu Lys Phe Met Gly Glu Thr
   50 55 60
   Arg Arg Thr Gly Ile Gln Trp Ser Asn Arg Ser Leu Arg His Asp Pro
   65 70 75 80
   Tyr Arg Phe Leu Asp Lys Lys Ser Pro Arg Ser Ser Gln Leu Ala Arg
   85 90 95
   Asp Ile Thr Val Arg Ala Asp Leu Ser Gly Ala Ala Thr Pro Asp Ser
   100 105 110
   Ser Phe Pro Glu Pro Glu Ile Lys Leu Ser Ser Arg Leu Arg Gly Ile
   115 120 125
   Phe Phe Cys Val Val Ala Gly Ile Ser Ala Thr Phe Leu Ile Val Leu
   130 135 140
   Met Ile Ile Gly His Pro Phe Val Leu Leu Phe Asp Pro Tyr Arg Arg
   145 150 155 160
   Lys Phe His His Phe Ile Ala Lys Leu Trp Ala Ser Ile Ser Ile Tyr
   165 170 175
   Pro Phe Tyr Lys Ile Asn Ile Glu Gly Leu Glu Asn Leu Pro Ser Ser
               180                 185                 190
   Asp Thr Pro Ala Val Tyr Val Ser Asn His Gln Ser Phe Leu Asp Ile
           195                 200                 205
   Tyr Thr Leu Leu Ser Leu Gly Lys Ser Phe Lys Phe Ile Ser Lys Thr
       210                 215                 220
   Gly Ile Phe Val Ile Pro Ile Ile Gly Trp Ala Met Ser Met Met Gly
   225                 230                 235                 240
   Val Val Pro Leu Lys Arg Met Asp Pro Arg Ser Gln Val Asp Cys Leu
                   245                 250                 255
   Lys Arg Cys Met Glu Leu Leu Lys Lys Gly Ala Ser Val Phe Phe Phe
               260                 265                 270
   Pro Glu Gly Thr Arg Ser Lys Asp Gly Arg Leu Gly Ser Phe Lys Lys
           275                 280                 285
   Gly Ala Phe Thr Val Ala Ala Lys Thr Gly Val Ala Val Val Pro Ile
       290                 295                 300
   Thr Leu Met Gly Thr Gly Lys Ile Met Pro Thr Gly Ser Glu Gly Ile
   305                 310                 315                 320
   Leu Asn His Gly Asn Val Arg Val Ile Ile His Lys Pro Ile His Gly
                   325                 330                 335
   Ser Lys Ala Asp Val Leu Cys Asn Glu Ala Arg Ser Lys Ile Ala Glu
               340                 345                 350
   Ser Met Asp Leu
           355
<210> 22
   <211> 374
   <212> PRT
   <213> Zea mays
<400> 22
   Met Ala Ile Pro Leu Val Leu Val Val Leu Pro Leu Gly Leu Leu Phe
   1               5                   10                  15
   Leu Leu Ser Gly Leu Ile Val Asn Ala Ile Gln Ala Val Leu Phe Val
               20                  25                  30
   Thr Ile Arg Pro Phe Ser Lys Ser Phe Tyr Arg Arg Ile Asn Arg Phe
           35                  40                  45
   Leu Ala Glu Leu Leu Trp Leu Gln Leu Val Trp Val Val Asp Trp Trp
       50                  55                  60
   Ala Gly Val Lys Val Gln Leu His Ala Asp Glu Glu Thr Tyr Arg Ser
   65                  70                  75                  80
   Met Gly Lys Glu His Ala Leu Ile Ile Ser Asn His Arg Ser Asp Ile
                   85                  90                  95
   Asp Trp Leu Ile Gly Trp Ile Leu Ala Gln Arg Ser Gly Cys Leu Gly
               100                 105                 110
   Ser Thr Leu Ala Val Met Lys Lys Ser Ser Lys Phe Leu Pro Val Ile
           115                 120                 125
   Gly Trp Ser Met Trp Phe Ala Glu Tyr Leu Phe Leu Glu Arg Ser Trp
       130                 135                 140
   Ala Lys Asp Glu Lys Thr Leu Lys Trp Gly Leu Gln Arg Leu Lys Asp
   145                 150                 155                 160
   Phe Pro Arg Pro Phe Trp Leu Ala Leu Phe Val Glu Gly Thr Arg Phe
                   165                 170                 175
   Thr Pro Ala Lys Leu Leu Ala Ala Gln Glu Tyr Ala Ala Ser Gln Gly
               180                 185                 190
   Leu Pro Ala Pro Arg Asn Val Leu Ile Pro Arg Thr Lys Gly Phe Val
           195                 200                 205
   Ser Ala Val Ser Ile Met Arg Asp Phe Val Pro Ala Ile Tyr Asp Thr
       210                 215                 220
   Thr Val Ile Val Pro Lys Asp Ser Pro Gln Pro Thr Met Leu Arg Ile
   225                 230                 235                 240
   Leu Lys Gly Gln Ser Ser Val Ile His Val Arg Met Lys Arg His Ala
                   245                 250                 255
   Met Ser Glu Met Pro Lys Ser Asp Glu Asp Val Ser Lys Trp Cys Lys
               260                 265                 270
   Asp Ile Phe Val Ala Lys Asp Ala Leu Leu Asp Lys His Leu Ala Thr
               275                 280                 285
   Gly Thr Phe Asp Glu Glu Ile Arg Pro Ile Gly Arg Pro Val Lys Ser
       290                 295                 300
   Leu Leu Val Thr Leu Phe Trp Ser Cys Leu Leu Leu Phe Gly Ala Ile
   305                 310                 315                 320
   Glu Phe Phe Lys Trp Thr Gln Leu Leu Ser Thr Trp Arg Gly Val Ala
                   325                 330                 335
   Phe Thr Ala Ala Gly Met Ala Leu Val Thr Gly Val Met His Val Phe
               340                 345                 350
   Ile Met Phe Ser Gln Ala Glu Arg Ser Ser Ser Ala Arg Ala Ala Arg
           355                 360                 365
   Asn Arg Val Lys Lys Glu
       370
<210> 23
   <211> 389
   <212> PRT
   <213> Arabidopsis thaliana
<400> 23
   Met Val Ile Ala Ala Ala Val Ile Val Pro Leu Gly Leu Leu Phe Phe
   1               5                   10                  15
   Ile Ser Gly Leu Ala Val Asn Leu Phe Gln Ala Val Cys Tyr Val Leu
               20                  25                  30
   Ile Arg Pro Leu Ser Lys Asn Thr Tyr Arg Lys Ile Asn Arg Val Val
           35                  40                  45
   Ala Glu Thr Leu Trp Leu Glu Leu Val Trp Ile Val Asp Trp Trp Ala
       50                  55                  60
   Gly Val Lys Ile Gln Val Phe Ala Asp Asn Glu Thr Phe Asn Arg Met
   65                  70                  75                  80
   Gly Lys Glu His Ala Leu Val Val Cys Asn His Arg Ser Asp Ile Asp
                   85                  90                  95
   Trp Leu Val Gly Trp Ile Leu Ala Gln Arg Ser Gly Cys Leu Gly Ser
               100                 105                 110
   Ala Leu Ala Val Met Lys Lys Ser Ser Lys Phe Leu Pro Val Ile Gly
           115                 120                 125
   Trp Ser Met Trp Phe Ser Glu Tyr Leu Phe Leu Glu Arg Asn Trp Ala
       130                 135                 140
   Lys Asp Glu Ser Thr Leu Lys Ser Gly Leu Gln Arg Leu Ser Asp Phe
   145                 150                 155                 160
   Pro Arg Pro Phe Trp Leu Ala Leu Phe Val Glu Gly Thr Arg Phe Thr
                   165                 170                 175
   Glu Ala Lys Leu Lys Ala Ala Gln Glu Tyr Ala Ala Ser Ser Glu Leu
               180                 185                 190
   Pro Ile Pro Arg Asn Val Leu Ile Pro Arg Thr Lys Gly Phe Val Ser
           195                 200                 205
   Ala Val Ser Asn Met Arg Ser Phe Val Pro Ala Ile Tyr Asp Met Thr
       210                 215                 220
   Val Thr Ile Pro Lys Thr Ser Pro Pro Pro Thr Met Leu Arg Leu Phe
   225                 230                 235                 240
   Lys Gly Gln Pro Ser Val Val His Val His Ile Lys Cys His Ser Met
                   245                 250                 255
   Lys Asp Leu Pro Glu Ser Asp Asp Ala Ile Ala Gln Trp Cys Arg Asp
               260                 265                 270
   Gln Phe Val Ala Lys Asp Ala Leu Leu Asp Lys His Ile Ala Ala Asp
           275                 280                 285
   Thr Phe Pro Gly Gln Gln Glu Gln Asn Ile Gly Arg Pro Ile Lys Ser
       290                 295                 300
   Leu Ala Val Val Leu Ser Trp Ala Cys Val Leu Thr Leu Gly Ala Ile
   305                 310                 315                 320
   Lys Phe Leu His Trp Ala Gln Leu Phe Ser Ser Trp Lys Gly Ile Thr
                   325                 330                 335
   Ile Ser Ala Leu Gly Leu Gly Ile Ile Thr Leu Cys Met Gln Ile Leu
               340                 345                 350
   Ile Arg Ser Ser Gln Ser Glu Arg Ser Thr Pro Ala Lys Val Val Pro
           355                 360                 365
   Ala Lys Pro Lys Asp Asn His His Pro Glu Ser Ser Ser Gln Thr Glu
   370 375 380
   Thr Glu Lys Glu Lys
   385
<210> 24
   <211> 376
   <212> PRT
   <213> Arabidopsis thaliana
<400> 24
   Met Lys Ile Pro Ala Ala Leu Val Phe Ile Pro Val Gly Val Leu Phe
   1 5 10 15
   Leu Ile Ser Gly Leu Ile Val Asn Ile Ile Gln Leu Val Phe Phe Ile
   20 25 30
   Ile Val Arg Pro Phe Ser Arg Ser Leu Tyr Arg Arg Ile Asn Lys Asn
   35 40 45
   Val Ala Glu Leu Leu Trp Leu Gln Leu Ile Trp Leu Phe Asp Trp Trp
   50 55 60
   Ala Cys Ile Lys Ile Asn Leu Tyr Val Asp Ala Glu Thr Leu Glu Leu
   65 70 75 80
   Ile Gly Lys Glu His Ala Leu Val Leu Ser Asn His Arg Ser Asp Ile
   85 90 95
   Asp Trp Leu Ile Gly Trp Val Met Ala Gln Arg Val Gly Cys Leu Gly
   100 105 110
   Ser Ser Leu Ala Ile Met Lys Lys Glu Ala Lys Tyr Leu Pro Ile Ile
   115 120 125
   Gly Trp Ser Met Trp Phe Ser Asp Tyr Ile Phe Leu Glu Arg Ser Trp
   130 135 140
   Ala Lys Asp Glu Asn Thr Leu Lys Ala Gly Phe Lys Arg Leu Glu Asp
   145 150 155 160
   Phe Pro Met Thr Phe Trp Leu Ala Leu Phe Val Glu Gly Thr Arg Phe
   165 170 175
   Thr Gln Glu Lys Leu Glu Ala Ala Gln Glu Tyr Ala Ser Ile Arg Ser
   180 185 190
   Leu Pro Ser Pro Arg Asn Val Leu Ile Pro Arg Thr Lys Gly Phe Val
           195                 200                 205
   Ser Ala Val Ser Glu Ile Arg Ser Phe Val Pro Ala Ile Tyr Asp Cys
       210                 215                 220
   Thr Leu Thr Val His Asn Asn Gln Pro Thr Pro Thr Leu Leu Arg Met
   225                 230                 235                 240
   Phe Ser Gly Gln Ser Ser Glu Ile Asn Leu Gln Met Arg Arg His Lys
                   245                 250                 255
   Met Ser Glu Leu Pro Glu Thr Asp Asp Gly Ile Ala Gln Trp Cys Gln
               260                 265                 270
   Asp Leu Phe Ile Thr Lys Asp Ala Gln Leu Glu Lys Tyr Phe Thr Lys
           275                 280                 285
   Asp Val Phe Ser Asp Leu Glu Val His Gln Ile Asn Arg Pro Ile Lys
       290                 295                 300
   Pro Leu Ile Val Val Ile Ile Trp Leu Gly Phe Leu Val Phe Gly Gly
   305                 310                 315                 320
   Phe Lys Leu Leu Gln Trp Leu Ser Ile Val Ala Ser Trp Lys Ile Ile
                   325                 330                 335
   Leu Leu Phe Val Phe Phe Leu Val Ile Ala Thr Ile Thr Met Gln Ile
               340                 345                 350
   Leu Ile Gln Ser Ser Glu Ser Gln Arg Ser Thr Pro Ala Lys Arg Pro
           355                 360                 365
   Leu Gln Glu Gln Leu Ile Ser Ala
       370                 375
<210> 25
   <211> 390
   <212> PRT
   <213> Brassica napus
<400> 25
   Met Ala Met Ala Ala Ala Val Ile Val Pro Leu Gly Ile Leu Phe Phe
   1               5                   10                  15
   Ile Ser Gly Leu Val Val Asn Leu Leu Gln Ala Val Cys Tyr Val Leu
               20                  25                  30
   Val Arg Pro Met Ser Lys Asn Thr Tyr Arg Lys Ile Asn Arg Val Val
           35                  40                  45
   Ala Glu Thr Leu Trp Leu Glu Leu Val Trp Ile Val Asp Trp Trp Ala
       50                  55                  60
   Gly Val Lys Ile Gln Val Phe Ala Asp Asp Glu Thr Phe Asn Arg Met
   65                  70                  75                  80
   Gly Lys Glu His Ala Leu Val Val Cys Asn His Arg Ser Asp Ile Asp
                   85                  90                  95
   Trp Leu Val Gly Trp Ile Leu Ala Gln Arg Ser Gly Cys Leu Gly Ser
               100                 105                 110
   Ala Leu Ala Val Met Lys Lys Ser Ser Lys Phe Leu Pro Val Ile Gly
           115                 120                 125
   Trp Ser Met Trp Phe Ser Glu Tyr Leu Phe Leu Glu Arg Asn Trp Ala
       130                 135                 140
   Lys Asp Glu Ser Thr Leu Gln Ser Gly Leu Gln Arg Leu Asn Asp Phe
   145                 150                 155                 160
   Pro Arg Pro Phe Trp Leu Ala Leu Phe Val Glu Gly Thr Arg Phe Thr
                   165                 170                 175
   Glu Ala Lys Leu Lys Ala Ala Gln Glu Tyr Ala Ala Ser Ser Glu Leu
               180                 185                 190
   Pro Val Pro Arg Asn Val Leu Ile Pro Arg Thr Lys Gly Phe Val Ser
           195                 200                 205
   Ala Val Ser Asn Met Arg Ser Phe Val Pro Ala Ile Tyr Asp Met Thr
       210                 215                 220
   Val Ala Ile Pro Lys Thr Ser Pro Pro Pro Thr Met Leu Arg Leu Phe
   225                 230                 235                 240
   Lys Gly Gln Pro Ser Val Val His Val His Ile Lys Cys His Ser Met
                   245                 250                 255
   Lys Asp Leu Pro Glu Pro Glu Asp Glu Ile Ala Gln Trp Cys Arg Asp
               260                 265                 270
   Gln Phe Val Ala Lys Asp Ala Leu Leu Asp Lys His Ile Ala Ala Asp
           275                 280                 285
   Thr Phe Pro Gly Gln Lys Glu Gln Asn Ile Gly Arg Pro Ile Lys Ser
       290                 295                 300
   Leu Ala Val Val Val Ser Trp Ala Cys Leu Leu Thr Leu Gly Ala Met
   305                 310                 315                 320
   Lys Phe Leu His Trp Ser Asn Leu Phe Ser Ser Trp Lys Gly Ile Ala
                   325                 330                 335
   Leu Ser Ala Phe Gly Leu Gly Ile Ile Thr Leu Cys Met Gln Ile Leu
               340                 345                 350
   Ile Arg Ser Ser Gln Ser Glu Arg Ser Thr Pro Ala Lys Val Ala Pro
           355                 360                 365
   Ala Lys Pro Lys Asp Asn His Gln Ser Gly Pro Ser Ser Gln Thr Glu
       370                 375                 380
   Val Glu Glu Lys Gln Lys
   385                 390
<210> 26
   <211> 306
   <212> PRT
   <213> Prunus dulcis
<400> 26
   Met Gly Lys Glu His Ala Leu Val Ile Ser Asn His Arg Ser Asp Ile
   1               5                   10                  15
   Asp Trp Leu Val Gly Trp Val Leu Ala Gln Arg Ser Gly Cys Leu Gly
               20                  25                  30
   Ser Ser Leu Ala Val Met Lys Lys Ser Ser Lys Phe Leu Pro Val Ile
           35                  40                  45
   Gly Trp Ser Met Trp Phe Ser Glu Tyr Leu Phe Leu Glu Arg Ser Trp
       50                  55                  60
   Ala Lys Asp Glu Gly Thr Leu Lys Ser Gly Val Gln Arg Leu Lys Asp
   65                  70                  75                  80
   Phe Pro Gln Pro Phe Trp Leu Ala Leu Phe Val Glu Gly Thr Arg Phe
                   85                  90                  95
   Thr Gln Ala Lys Leu Leu Ala Ala Gln Glu Tyr Ala Ala Ala Thr Gly
               100                 105                 110
   Leu Pro Val Pro Arg Asn Val Leu Ile Pro Arg Thr Lys Gly Phe Val
           115                 120                 125
   Thr Ala Val Ser Gln Met Arg Ser Phe Ala Pro Ala Ile Tyr Asp Val
       130                 135                 140
   Thr Val Ala Ile Pro Lys Ser Ser Pro Ala Pro Thr Met Leu Arg Leu
   145                 150                 155                 160
   Phe Glu Gly Arg Pro Ser Val Val His Val His Ile Lys Arg His Val
                   165                 170                 175
   Met Arg Asp Leu Pro Glu Thr Asp Glu Ala Val Ala Gln Trp Cys Lys
               180                 185                 190
   Asp Ile Phe Val Ala Lys Asp Ala Leu Leu Asp Lys His Thr Val Glu
           195                 200                 205
   Gln Thr Phe Gly Asp Gln Gln Leu Lys Val Thr Gly Arg Pro Leu Lys
       210                 215                 220
   Ser Leu Leu Val Val Thr Ala Trp Ala Cys Leu Leu Ile Leu Gly Ala
   225                 230                 235                 240
   Leu Lys Phe Leu Tyr Trp Ser Ser Leu Leu Ser Ser Trp Lys Gly Ile
                   245                 250                 255
   Ala Phe Ser Ala Leu Gly Leu Gly Val Val Thr Val Leu Met Gln Ile
               260                 265                 270
   Leu Ile Arg Phe Ser Gln Ser Glu Arg Ser Thr Pro Ala Pro Val Ala
           275                 280                 285
   Pro Thr Asn Asn Lys Asn Lys Gly Glu Ser Ser Gly Lys Pro Glu Lys
       290                 295                 300
   Gln Gln
   305
<210> 27
   <211> 7642
   <212> DNA
   <213> Artificial Sequence
<220>
   <223> pSGI-NB55 vector that includes sll1752 LPAAT gene
<400> 27
   cagttaccaa tgcttaatca gtgaggcacc tatctcagcg atctgtctat ttcgttcatc 60
   catagttgcc tgactccccg tcgtgtagat aactacgata cgggagggct taccatctgg 120
   ccccagtgct gcaatgatac cgcgagaccc acgctcaccg gctccagatt tatcagcaat 180
   aaaccagcca gccggaaggg ccgagcgcag aagtggtcct gcaactttat ccgcctccat 240
   ccagtctatt aattgttgcc gggaagctag agtaagtagt tcgccagtta atagtttgcg 300
   caactgttgg gaagggcgat cggtgcgggc ctcttcgcta ttacgccagc tggcgaaagg 360
   gggatgtgct gcaaggcgat taagttgggt aacgccaggg ttttcccagt cacgacgttg 420
   taaaacgacg gccagtgaat tgggcccgac tgcctttggt ggtattaccg atgagtggca 480
   cgttattttc accgctctgg ccgtgttgag catggtgctg ggcaacgtgg tggctttagc 540
   ccaaaccagc atgaaacgga tgttggccta ctcttccatc ggtcaagcag gctttgtgat 600
   gattggccta gtggccggca gtgaagatgg ttacgccagc atggttttct acatgctcat 660
   ctatctgttt atgaacctgg gggcgtttag ttgcattatt ctcttcaccc tccgcactgg 720
   cagtgaccaa attagtgatt acgctggtct gtaccacaaa gaccccttgt taaccttggg 780
   cttgagcatt tgtttattat ccttgggggg cattcctcct ctggcgggct ttttcggcaa 840
   aatttacatc ttctgggccg gttggcaatc gggattgtat ggcctagtcc tacttggtct 900
   ggttaccagt gtagtttcca tctactacta catccgggtg gtgaaaatga tggtggtgaa 960
   ggagccccag gaaatgtccg aagtaatcaa aaattacccg gccatcaaat ggaatttacc 1020
   cggcatgcgt cccctacagg tgggcattgt cgctactttg gttgctacct cgctggcagg 1080
   tattctggct aatcccctct ttaacctcgc caccgattcc gtggtcagca ccaagatgtt 1140
   gcagacagcc ctccagcaaa caggagaaac tccggcgatc gccatttccc atgatttacc 1200
   ctaggggtat caggaaatat tgctttgcag gcaaaagcca atgagtgtaa ctatagaaac 1260
   cgatttaaag gagatccact agtcctgagg ctgaaatgag ctgttgacaa ttaatcatcc 1320
   ggctcgtata atgtgtggaa ttgtgagcgg ataacaattt cacactaagg aggaaaaaaa 1380
   ccatgaccaa ttctcccctg gcgatcgacc ctgctgtcat tggcccttcc agggtttccc 1440
   cctggctaat caaattaatt tatcccctag gcaccaggtt tttacattgg tattttggcc 1500
   cgatcgccat ccatggtcag gagcatttac cccgcagtgg cccgattatt ctggccccta 1560
   cccaccgttc ccgttgggat gcaattttgc tttctttggc cgccggtcgg ggggtaactg 1620
   gtcgagacct ccgttttatg gtggcggtga cggaagtgca gggtttacag ggttggttta 1680
   ttcgccattt ggggggattt cccgttgacg tgaaaaggcc ggaaatcagt agtgtgagtt 1740
   atagtgtgca gttactccaa gcaggggaaa tgttggtcat ttttcctgag gggggcattt 1800
   ttcgggatca acacactgtc catcccctca aacggggtat tgggcgcatt gccatggaag 1860
   tttgtaagca aaaccccaac actgacatta aagtgatccc agtgacgatc gcctacagtg 1920
   acccctaccc aggcaaaggc acttcagtgg aaattaattt tggccagggc atcgccgcta 1980
   gggactacga ccccagcacc atcaaggcta gctcccaaaa actgacccgt tgtttggccc 2040
   atagaatgca gaacctttac agttccgaga atgtctctct gtgcgaggcg atcgccgctt 2100
   cgtgattcta gatatctgca ggcctaagct ttatgcttgt aaaccgtttt gtgaaaaaat 2160
   ttttaaaata aaaaagggga cctctagggt ccccaattaa ttagtaatat aatctattaa 2220
   aggtcattca aaaggtcatc caccggatca attcccctgc tcgcgcaggc tgggtgccag 2280
   gcccgatcct tggagccctt gccctcccgc acgatgatcg tgccgtgatc gaaatccaga 2340
   tccttgaccc gcagttgcaa accctcactg atccgcatgc ccgttccata cagaagctgg 2400
   gcgaacaaac gatgctcgcc ttccagaaaa ccgaggatgc gaaccacttc atccggggtc 2460
   agcaccaccg gcaagcgccg cgacggccga ggtcttccga tctcctgaag ccagggcaga 2520
   tccgtgcaca gcaccttgcc gtagaagaac agcaaggccg ccaatgcctg acgatgcgtg 2580
   gagaccgaaa ccttgcgctc gttcgccagc caggacagaa atgcctcgac ttcgctgctg 2640
   cccaaggttg ccgggtgacg cacaccgtgg aaacggatga aggcacgaac ccagtggaca 2700
   taagcctgtt cggttcgtaa gctgtaatgc aagtagcgta tgcgctcacg caactggtcc 2760
   agaaccttga ccgaacgcag cggtggtaac ggcgcagtgg cggttttcat ggcttgttat 2820
   gactgttttt ttggggtaca gtctatgcct cggtcgggca tccaagcagc aagcgcgtta 2880
   cgccgtgggt cgatgtttga tgttatggag cagcaacgat gttacgcagc agggcagtcg 2940
   ccctaaaaca aagttaaaca tcatgaggga agcggtgatc gccgaagtat cgactcaact 3000
   atcagaggta gttggcgtca tcgagcgcca tctcgaaccg acgttgctgg ccgtacattt 3060
   gtacggctcc gcagtggatg gcggcctgaa gccacacagt gatattgatt tgctggttac 3120
   ggtgaccgta aggcttgatg aaacaacgcg gcgagctttg atcaacgacc ttttggaaac 3180
   ttcggcttcc cctggagaga gcgagattct ccgcgctgta gaagtcacca ttgttgtgca 3240
   cgacgacatc attccgtggc gttatccagc taagcgcgaa ctgcaatttg gagaatggca 3300
   gcgcaatgac attcttgcag gtatcttcga gccagccacg atcgacattg atctggctat 3360
   cttgctgaca aaagcaagag aacatagcgt tgccttggta ggtccagcgg cggaggaact 3420
   ctttgatccg gttcctgaac aggatctatt tgaggcgcta aatgaaacct taacgctatg 3480
   gaactcgccg cccgactggg ctggcgatga gcgaaatgta gtgcttacgt tgtcccgcat 3540
   ttggtacagc gcagtaaccg gcaaaatcgc gccgaaggat gtcgctgccg actgggcaat 3600
   ggagcgcctg ccggcccagt atcagcccgt catacttgaa gctagacagg cttatcttgg 3660
   acaagaagaa gatcgcttgg cctcgcgcgc agatcagttg gaagaatttg tccactacgt 3720
   gaaaggcgag atcaccaagg tagtcggcaa ataatgtcta acaattcgtt caagccgacg 3780
   ccgcttcgcg gcgcggctta actcaagcgt tagatgcact aagcacataa ttgctcacag 3840
   ccaaactatc aggtcaagtc tgcttttatt atttttaagc gtgcataata agccctacac 3900
   aaattgggag atatatcatg aaaggctggc tttttcttgt tatcgcaata gttggcgaag 3960
   taatcgcaac atccgcatta aaatctagcg agggctttac taagctgatc cggtggatga 4020
   ccttttgaat gacctttaat agattatatt actaattaat tggggaccct agaggtcccc 4080
   tttttatttt taaaaatttt ttcacaaaac ggtttacaag cataaagctt ccgcggtacc 4140
   cgggaattcg ccctttcaag cttcagatca attcgcgcta acttacatta attgcgttgc 4200
   gctcactgcc cgctttccag tcgggaaacc tgtcgtgcca gctgcattaa tgaatcggcc 4260
   aacgcgcggg gagaggcggt ttgcgtattg ggcgccaggg tggtttttct tttcaccagt 4320
   gagacgggca acagctgatt gcccttcacc gcctggccct gagagagttg cagcaagcgg 4380
   tccacgctgg tttgccccag caggcgaaaa tcctgtttga tggtggttaa cggcgggata 4440
   taacatgagc tgtcttcggt atcgtcgtat cccactaccg agatatccgc accaacgcgc 4500
   agcccggact cggtaatggc gcgcattgcg cccagcgcca tctgatcgtt ggcaaccagc 4560
   atcgcagtgg gaacgatgcc ctcattcagc atttgcatgg tttgttgaaa accggacatg 4620
   gcactccagt cgccttcccg ttccgctatc ggctgaattt gattgcgagt gagatattta 4680
   tgccagccag ccagacgcag acgcgccgag acagaactta atgggcccgc taacagcgcg 4740
   atttgctggt gacccaatgc gaccagatgc tccacgccca gtcgcgtacc gtcttcatgg 4800
   gagaaaataa tactgttgat gggtgtctgg tcagagacat caagaaataa cgccggaaca 4860
   ttagtgcagg cagcttccac agcaatggca tcctggtcat ccagcggata gttaatgatc 4920
   agcccactga cgcgttgcgc gagaagattg tgcaccgccg ctttacaggc ttcgacgccg 4980
   cttcgttcta ccatcgacac caccacgctg gcacccagtt gatcggcgcg agatttaatc 5040
   gccgcgacaa tttgcgacgg cgcgtgcagg gccagactgg aggtggcaac gccaatcagc 5100
   aacgactgtt tgcccgccag ttgttgtgcc acgcggttgg gaatgtaatt cagctccgcc 5160
   atcgccgctt ccactttttc ccgcgttttc gcagaaacgt ggctggcctg gttcaccacg 5220
   cgggaaacgg tctgataaga gacaccggca tactctgcga catcgtataa cgttactggt 5280
   ttcacattca ccaccctgaa ttgactctct tccgggcgct atcatgccat accgcgaaag 5340
   gttttgcacc attcgatggt gtcaacgtaa atgcatgccg cttcgccttc gcgcaagctt 5400
   agaagggcga attccggaca tatggatctt gggggaaatt aagaccaaac tcgatgacct 5460
   ccaaaaagat gtaacttctc ttaagatcga tatggcaacg gtgaaaaccg agttatctgc 5520
   ggtcaggatg gagataggta cagtcaagga tgatgttaaa gatgtcaaag ggcgggctaa 5580
   tgctcaaatt tgggcgttga ttcttgccgt catcggagcc ataattacca ccttggtgcg 5640
   ttttggcatt ttccctaatc cctaacaaaa aagcgaccag gcttttcttt caattgcccg 5700
   atcgcctttg atattttccc aaaggataaa agctagtcca ttcagaatcg agccttaaag 5760
   tactcccata ttggctagcc ccagaattac tccagcgccg aggatgtggc caaagctagc 5820
   ggtgcccagc acagccccta aaccaaagcc gccaaagaag ttagaggaag gcatgggggt 5880
   gcccacattt tgttgtttga tggtcaattt accaaaggcg atcgccaaaa tgttgcaagc 5940
   aatcatcacc ccagcaactt tagggctcca ggacagggtg gcgggaacgg cggtggccaa 6000
   caaaaagcta tgcattgaga ttctccagaa taaagacggt ttttaaaggg atagccccac 6060
   gctaatgggg gtctttaaaa atctcatctt acggggacgc tctgcccctg ggaaaccacc 6120
   gttgcaatac ttaacaaatt ttcgttttta gcttggcaaa tgtctttggc aaaattggtt 6180
   gatctggctt aaatcgtcag ttatttgccc tggaatagtc tggggacggg caattctgat 6240
   cagatttacc cccaacgctt ccgccacttt ttgcttaacc aattctcccc cctgggcacc 6300
   ggaggcttta gttaccaccc cttgaatttg ccattgttgc cacagggctt tttccaatgg 6360
   ttcggctacg ggggggcgca aagcaatgat acggtcggaa gtaaacccag cggcgatcgc 6420
   ctgggctagg gcttggggat agggcagaat acgggcaaat agggcccagc ttggcgtaat 6480
   catggtcata gctgtttcct gtgtgaaatt gttatccgct cacaattcca cacaacatac 6540
   gagccggaag cataaagtgt aaagcctggg gtgcctaatg agtgagctaa ctcacattaa 6600
   ttgcgttgcg ctcactgccc gctttccagt cgggaaacct gtcgtgccag ctgcattaat 6660
   gaatcggcca acgcgcgggg agaggcggtt tgcgtattgg gcgctcttcc gcttcctcgc 6720
   tcactgactc gctgcgctcg gtcgttcggc tgcggcgagc ggtatcagct cactcaaagg 6780
   cggtaatacg gttatccaca gaatcagggg ataacgcagg aaagaacatg tgagcaaaag 6840
   gccagcaaaa ggccaggaac cgtaaaaagg ccgcgttgct ggcgtttttc cataggctcc 6900
   gcccccctga cgagcatcac aaaaatcgac gctcaagtca gaggtggcga aacccgacag 6960
   gactataaag ataccaggcg tttccccctg gaagctccct cgtgcgctct cctgttccga 7020
   ccctgccgct taccggatac ctgtccgcct ttctcccttc gggaagcgtg gcgctttctc 7080
   atagctcacg ctgtaggtat ctcagttcgg tgtaggtcgt tcgctccaag ctgggctgtg 7140
   tgcacgaacc ccccgttcag cccgaccgct gcgccttatc cggtaactat cgtcttgagt 7200
   ccaacccggt aagacacgac ttatcgccac tggcagcagc cactggtaac aggattagca 7260
   gagcgaggta tgtaggcggt gctacagagt tcttgaagtg gtggcctaac tacggctaca 7320
   ctagaaggac agtatttggt atctgcgctc tgctgaagcc agttaccttc ggaaaaagag 7380
   ttggtagctc ttgatccggc aaacaaacca ccgctggtag cggtggtttt tttgtttgca 7440
   agcagcagat tacgcgcaga aaaaaaggat ctcaagaaga tcctttgatc ttttctacgg 7500
   ggtctgacgc tcagtggaac gaaaactcac gttaagggat tttggtcatg agattatcaa 7560
   aaaggatctt cacctagatc cttttaaatt aaaaatgaag ttttaaatca atctaaagta 7620
   tatatgagta aacttggtct ga 7642
<210> 28
   <211> 90
   <212> DNA
   <213> Artificial Sequence
<220>
   <223> TrcY promoter
<400> 28
   ctgaaatgag ctgttgacaa ttaatcatcc ggctcgtata atgtgtggaa ttgtgagcgg 60
   ataacaattt cacactaagg aggaaaaaaa 90
<210> 29
   <211> 39
   <212> DNA
   <213> Artificial Sequence
<220>
   <223> Primer NB395
<400> 29
   cactaaggag gaaaaaaaat gaccaattct cccctggcg 39
<210> 30
   <211> 34
   <212> DNA
   <213> Artificial Sequence
<220>
   <223> Primer NB396
<400> 30
   cctgcagata tctagaatca cgaagcggcg atcg 34
<210> 31
   <211> 8572
   <212> DNA
   <213> Artificial Sequence
<220>
   <223> YC63 expression/integration vector
<400> 31
   cagttaccaa tgcttaatca gtgaggcacc tatctcagcg atctgtctat ttcgttcatc      60
   catagttgcc tgactccccg tcgtgtagat aactacgata cgggagggct taccatctgg     120
   ccccagtgct gcaatgatac cgcgagaccc acgctcaccg gctccagatt tatcagcaat     180
   aaaccagcca gccggaaggg ccgagcgcag aagtggtcct gcaactttat ccgcctccat     240
   ccagtctatt aattgttgcc gggaagctag agtaagtagt tcgccagtta atagtttgcg     300
   caactgttgg gaagggcgat cggtgcgggc ctcttcgcta ttacgccagc tggcgaaagg     360
   gggatgtgct gcaaggcgat taagttgggt aacgccaggg ttttcccagt cacgacgttg 420
   taaaacgacg gccagtgaat tgggcccgac tgcctttggt ggtattaccg atgagtggca 480
   cgttattttc accgctctgg ccgtgttgag catggtgctg ggcaacgtgg tggctttagc 540
   ccaaaccagc atgaaacgga tgttggccta ctcttccatc ggtcaagcag gctttgtgat 600
   gattggccta gtggccggca gtgaagatgg ttacgccagc atggttttct acatgctcat 660
   ctatctgttt atgaacctgg gggcgtttag ttgcattatt ctcttcaccc tccgcactgg 720
   cagtgaccaa attagtgatt acgctggtct gtaccacaaa gaccccttgt taaccttggg 780
   cttgagcatt tgtttattat ccttgggggg cattcctcct ctggcgggct ttttcggcaa 840
   aatttacatc ttctgggccg gttggcaatc gggattgtat ggcctagtcc tacttggtct 900
   ggttaccagt gtagtttcca tctactacta catccgggtg gtgaaaatga tggtggtgaa 960
   ggagccccag gaaatgtccg aagtaatcaa aaattacccg gccatcaaat ggaatttacc 1020
   cggcatgcgt cccctacagg tgggcattgt cgctactttg gttgctacct cgctggcagg 1080
   tattctggct aatcccctct ttaacctcgc caccgattcc gtggtcagca ccaagatgtt 1140
   gcagacagcc ctccagcaaa caggagaaac tccggcgatc gccatttccc atgatttacc 1200
   ctaggggtat caggaaatat tgctttgcag gcaaaagcca atgagtgtaa ctatagaaac 1260
   cgatttaaag gagatccact agtcctgagg ctgaaatgag ctgttgacaa ttaatcatcc 1320
   ggctcgtata atgtgtggaa ttgtgagcgg ataacaattt cacactaagg aggaaaaaaa 1380
   ccatggaaga cgccaaaaac ataaagaaag gcccggcgcc attctatccg ctggaagatg 1440
   gaaccgctgg agagcaactg cataaggcta tgaagagata cgccctggtt cctggaacaa 1500
   ttgcttttac agatgcacat atcgaggtgg acatcactta cgctgagtac ttcgaaatgt 1560
   ccgttcggtt ggcagaagct atgaaacgat atgggctgaa tacaaatcac agaatcgtcg 1620
   tatgcagtga aaactctctt caattcttta tgccggtgtt gggcgcgtta tttatcggag 1680
   ttgcagttgc gcccgcgaac gacatttata atgaacgtga attgctcaac agtatgggca 1740
   tttcgcagcc taccgtggtg ttcgtttcca aaaaggggtt gcaaaaaatt ttgaacgtgc 1800
   aaaaaaagct cccaatcatc caaaaaatta ttatcatgga ttctaaaacg gattaccagg 1860
   gatttcagtc gatgtacacg ttcgtcacat ctcatctacc tcccggtttt aatgaatacg 1920
   attttgtgcc agagtccttc gatagggaca agacaattgc actgatcatg aactcctctg 1980
   gatctactgg tctgcctaaa ggtgtcgctc tgcctcatag aactgcctgc gtgagattct 2040
   cgcatgccag agatcctatt tttggcaatc aaatcattcc ggatactgcg attttaagtg 2100
   ttgttccatt ccatcacggt tttggaatgt ttactacact cggatatttg atatgtggat 2160
   ttcgagtcgt cttaatgtat agatttgaag aagagctgtt tctgaggagc cttcaggatt 2220
   acaagattca aagtgcgctg ctggtgccaa ccctattctc cttcttcgcc aaaagcactc 2280
   tgattgacaa atacgattta tctaatttac acgaaattgc ttctggtggc gctcccctct 2340
   ctaaggaagt cggggaagcg gttgccaaga ggttccatct gccaggtatc aggcaaggat 2400
   atgggctcac tgagactaca tcagctattc tgattacacc cgagggggat gataaaccgg 2460
   gcgcggtcgg taaagttgtt ccattttttg aagcgaaggt tgtggatctg gataccggga 2520
   aaacgctggg cgttaatcaa agaggcgaac tgtgtgtgag aggtcctatg attatgtccg 2580
   gttatgtaaa caatccggaa gcgaccaacg ccttgattga caaggatgga tggctacatt 2640
   ctggagacat agcttactgg gacgaagacg aacacttctt catcgttgac cgcctgaagt 2700
   ctctgattaa gtacaaaggc tatcaggtgg ctcccgctga attggaatcc atcttgctcc 2760
   aacaccccaa catcttcgac gcaggtgtcg caggtcttcc cgacgatgac gccggtgaac 2820
   ttcccgccgc cgttgttgtt ttggagcacg gaaagacgat gacggaaaaa gagatcgtgg 2880
   attacgtcgc cagtcaagta acaaccgcga aaaagttgcg cggaggagtt gtgtttgtgg 2940
   acgaagtacc gaaaggtctt accggaaaac tcgacgcaag aaaaatcaga gagatcctca 3000
   taaaggccaa gaagggcgga aagatcgccg tgtaattcta gatatctgca ggcctaagct 3060
   ttatgcttgt aaaccgtttt gtgaaaaaat ttttaaaata aaaaagggga cctctagggt 3120
   ccccaattaa ttagtaatat aatctattaa aggtcattca aaaggtcatc caccggatca 3180
   attcccctgc tcgcgcaggc tgggtgccag gcccgatcct tggagccctt gccctcccgc 3240
   acgatgatcg tgccgtgatc gaaatccaga tccttgaccc gcagttgcaa accctcactg 3300
   atccgcatgc ccgttccata cagaagctgg gcgaacaaac gatgctcgcc ttccagaaaa 3360
   ccgaggatgc gaaccacttc atccggggtc agcaccaccg gcaagcgccg cgacggccga 3420
   ggtcttccga tctcctgaag ccagggcaga tccgtgcaca gcaccttgcc gtagaagaac 3480
   agcaaggccg ccaatgcctg acgatgcgtg gagaccgaaa ccttgcgctc gttcgccagc 3540
   caggacagaa atgcctcgac ttcgctgctg cccaaggttg ccgggtgacg cacaccgtgg 3600
   aaacggatga aggcacgaac ccagtggaca taagcctgtt cggttcgtaa gctgtaatgc 3660
   aagtagcgta tgcgctcacg caactggtcc agaaccttga ccgaacgcag cggtggtaac 3720
   ggcgcagtgg cggttttcat ggcttgttat gactgttttt ttggggtaca gtctatgcct 3780
   cggtcgggca tccaagcagc aagcgcgtta cgccgtgggt cgatgtttga tgttatggag 3840
   cagcaacgat gttacgcagc agggcagtcg ccctaaaaca aagttaaaca tcatgaggga 3900
   agcggtgatc gccgaagtat cgactcaact atcagaggta gttggcgtca tcgagcgcca 3960
   tctcgaaccg acgttgctgg ccgtacattt gtacggctcc gcagtggatg gcggcctgaa 4020
   gccacacagt gatattgatt tgctggttac ggtgaccgta aggcttgatg aaacaacgcg 4080
   gcgagctttg atcaacgacc ttttggaaac ttcggcttcc cctggagaga gcgagattct 4140
   ccgcgctgta gaagtcacca ttgttgtgca cgacgacatc attccgtggc gttatccagc 4200
   taagcgcgaa ctgcaatttg gagaatggca gcgcaatgac attcttgcag gtatcttcga 4260
   gccagccacg atcgacattg atctggctat cttgctgaca aaagcaagag aacatagcgt 4320
   tgccttggta ggtccagcgg cggaggaact ctttgatccg gttcctgaac aggatctatt 4380
   tgaggcgcta aatgaaacct taacgctatg gaactcgccg cccgactggg ctggcgatga 4440
   gcgaaatgta gtgcttacgt tgtcccgcat ttggtacagc gcagtaaccg gcaaaatcgc 4500
   gccgaaggat gtcgctgccg actgggcaat ggagcgcctg ccggcccagt atcagcccgt 4560
   catacttgaa gctagacagg cttatcttgg acaagaagaa gatcgcttgg cctcgcgcgc 4620
   agatcagttg gaagaatttg tccactacgt gaaaggcgag atcaccaagg tagtcggcaa 4680
   ataatgtcta acaattcgtt caagccgacg ccgcttcgcg gcgcggctta actcaagcgt 4740
   tagatgcact aagcacataa ttgctcacag ccaaactatc aggtcaagtc tgcttttatt 4800
   atttttaagc gtgcataata agccctacac aaattgggag atatatcatg aaaggctggc 4860
   tttttcttgt tatcgcaata gttggcgaag taatcgcaac atccgcatta aaatctagcg 4920
   agggctttac taagctgatc cggtggatga ccttttgaat gacctttaat agattatatt 4980
   actaattaat tggggaccct agaggtcccc ttttttattt taaaaatttt ttcacaaaac 5040
   ggtttacaag cataaagctt ccgcggtacc cgggaattcg ccctttcaag cttcagatca 5100
   attcgcgcta acttacatta attgcgttgc gctcactgcc cgctttccag tcgggaaacc 5160
   tgtcgtgcca gctgcattaa tgaatcggcc aacgcgcggg gagaggcggt ttgcgtattg 5220
   ggcgccaggg tggtttttct tttcaccagt gagacgggca acagctgatt gcccttcacc 5280
   gcctggccct gagagagttg cagcaagcgg tccacgctgg tttgccccag caggcgaaaa 5340
   tcctgtttga tggtggttaa cggcgggata taacatgagc tgtcttcggt atcgtcgtat 5400
   cccactaccg agatatccgc accaacgcgc agcccggact cggtaatggc gcgcattgcg 5460
   cccagcgcca tctgatcgtt ggcaaccagc atcgcagtgg gaacgatgcc ctcattcagc 5520
   atttgcatgg tttgttgaaa accggacatg gcactccagt cgccttcccg ttccgctatc 5580
   ggctgaattt gattgcgagt gagatattta tgccagccag ccagacgcag acgcgccgag 5640
   acagaactta atgggcccgc taacagcgcg atttgctggt gacccaatgc gaccagatgc 5700
   tccacgccca gtcgcgtacc gtcttcatgg gagaaaataa tactgttgat gggtgtctgg 5760
   tcagagacat caagaaataa cgccggaaca ttagtgcagg cagcttccac agcaatggca 5820
   tcctggtcat ccagcggata gttaatgatc agcccactga cgcgttgcgc gagaagattg 5880
   tgcaccgccg ctttacaggc ttcgacgccg cttcgttcta ccatcgacac caccacgctg 5940
   gcacccagtt gatcggcgcg agatttaatc gccgcgacaa tttgcgacgg cgcgtgcagg 6000
   gccagactgg aggtggcaac gccaatcagc aacgactgtt tgcccgccag ttgttgtgcc 6060
   acgcggttgg gaatgtaatt cagctccgcc atcgccgctt ccactttttc ccgcgttttc 6120
   gcagaaacgt ggctggcctg gttcaccacg cgggaaacgg tctgataaga gacaccggca 6180
   tactctgcga catcgtataa cgttactggt ttcacattca ccaccctgaa ttgactctct 6240
   tccgggcgct atcatgccat accgcgaaag gttttgcacc attcgatggt gtcaacgtaa 6300
   atgcatgccg cttcgccttc gcgcaagctt agaagggcga attccggaca tatggatctt 6360
   gggggaaatt aagaccaaac tcgatgacct ccaaaaagat gtaacttctc ttaagatcga 6420
   tatggcaacg gtgaaaaccg agttatctgc ggtcaggatg gagataggta cagtcaagga 6480
   tgatgttaaa gatgtcaaag ggcgggctaa tgctcaaatt tgggcgttga ttcttgccgt 6540
   catcggagcc ataattacca ccttggtgcg ttttggcatt ttccctaatc cctaacaaaa 6600
   aagcgaccag gcttttcttt caattgcccg atcgcctttg atattttccc aaaggataaa 6660
   agctagtcca ttcagaatcg agccttaaag tactcccata ttggctagcc ccagaattac 6720
   tccagcgccg aggatgtggc caaagctagc ggtgcccagc acagccccta aaccaaagcc 6780
   gccaaagaag ttagaggaag gcatgggggt gcccacattt tgttgtttga tggtcaattt 6840
   accaaaggcg atcgccaaaa tgttgcaagc aatcatcacc ccagcaactt tagggctcca 6900
   ggacagggtg gcgggaacgg cggtggccaa caaaaagcta tgcattgaga ttctccagaa 6960
   taaagacggt ttttaaaggg atagccccac gctaatgggg gtctttaaaa atctcatctt 7020
   acggggacgc tctgcccctg ggaaaccacc gttgcaatac ttaacaaatt ttcgttttta 7080
   gcttggcaaa tgtctttggc aaaattggtt gatctggctt aaatcgtcag ttatttgccc 7140
   tggaatagtc tggggacggg caattctgat cagatttacc cccaacgctt ccgccacttt 7200
   ttgcttaacc aattctcccc cctgggcacc ggaggcttta gttaccaccc cttgaatttg 7260
   ccattgttgc cacagggctt tttccaatgg ttcggctacg ggggggcgca aagcaatgat 7320
   acggtcggaa gtaaacccag cggcgatcgc ctgggctagg gcttggggat agggcagaat 7380
   acgggcaaat agggcccagc ttggcgtaat catggtcata gctgtttcct gtgtgaaatt 7440
   gttatccgct cacaattcca cacaacatac gagccggaag cataaagtgt aaagcctggg 7500
   gtgcctaatg agtgagctaa ctcacattaa ttgcgttgcg ctcactgccc gctttccagt 7560
   cgggaaacct gtcgtgccag ctgcattaat gaatcggcca acgcgcgggg agaggcggtt 7620
   tgcgtattgg gcgctcttcc gcttcctcgc tcactgactc gctgcgctcg gtcgttcggc 7680
   tgcggcgagc ggtatcagct cactcaaagg cggtaatacg gttatccaca gaatcagggg 7740
   ataacgcagg aaagaacatg tgagcaaaag gccagcaaaa ggccaggaac cgtaaaaagg 7800
   ccgcgttgct ggcgtttttc cataggctcc gcccccctga cgagcatcac aaaaatcgac 7860
   gctcaagtca gaggtggcga aacccgacag gactataaag ataccaggcg tttccccctg 7920
   gaagctccct cgtgcgctct cctgttccga ccctgccgct taccggatac ctgtccgcct 7980
   ttctcccttc gggaagcgtg gcgctttctc atagctcacg ctgtaggtat ctcagttcgg 8040
   tgtaggtcgt tcgctccaag ctgggctgtg tgcacgaacc ccccgttcag cccgaccgct 8100
   gcgccttatc cggtaactat cgtcttgagt ccaacccggt aagacacgac ttatcgccac 8160
   tggcagcagc cactggtaac aggattagca gagcgaggta tgtaggcggt gctacagagt 8220
   tcttgaagtg gtggcctaac tacggctaca ctagaaggac agtatttggt atctgcgctc 8280
   tgctgaagcc agttaccttc ggaaaaagag ttggtagctc ttgatccggc aaacaaacca 8340
   ccgctggtag cggtggtttt tttgtttgca agcagcagat tacgcgcaga aaaaaaggat 8400
   ctcaagaaga tcctttgatc ttttctacgg ggtctgacgc tcagtggaac gaaaactcac 8460
   gttaagggat tttggtcatg agattatcaa aaaggatctt cacctagatc cttttaaatt 8520
   aaaaatgaag ttttaaatca atctaaagta tatatgagta aacttggtct ga 8572
<210> 32
   <211> 1032
   <212> DNA
   <213> Synechocystis PCC6803
<400> 32
   tatttgcccg tattctgccc tatccccaag ccctagccca ggcgatcgcc gctgggttta 60
   cttccgaccg tatcattgct ttgcgccccc ccgtagccga accattggaa aaagccctgt 120
   ggcaacaatg gcaaattcaa ggggtggtaa ctaaagcctc cggtgcccag gggggagaat 180
   tggttaagca aaaagtggcg gaagcgttgg gggtaaatct gatcagaatt gcccgtcccc 240
   agactattcc agggcaaata actgacgatt taagccagat caaccaattt tgccaaagac 300
   atttgccaag ctaaaaacga aaatttgtta agtattgcaa cggtggtttc ccaggggcag 360
   agcgtccccg taagatgaga tttttaaaga cccccattag cgtggggcta tccctttaaa 420
   aaccgtcttt attctggaga atctcaatgc atagcttttt gttggccacc gccgttcccg 480
   ccaccctgtc ctggagccct aaagttgctg gggtgatgat tgcttgcaac attttggcga 540
   tcgcctttgg taaattgacc atcaaacaac aaaatgtggg cacccccatg ccttcctcta 600
   acttctttgg cggctttggt ttaggggctg tgctgggcac cgctagcttt ggccacatcc 660
   tcggcgctgg agtaattctg gggctagcca atatgggagt actttaaggc tcgattctga 720
   atggactagc ttttatcctt tgggaaaata tcaaaggcga tcgggcaatt gaaagaaaag 780
   cctggtcgct tttttgttag ggattaggga aaatgccaaa acgcaccaag gtggtaatta 840
   tggctccgat gacggcaaga atcaacgccc aaatttgagc attagcccgc cctttgacat 900
   ctttaacatc atccttgact gtacctatct ccatcctgac cgcagataac tcggttttca 960
   ccgttgccat atcgatctta agagaagtta catctttttg gaggtcatcg agtttggtct 1020
   taatttcccc ca 1032
<210> 33
   <211> 824
   <212> DNA
   <213> Synechocystis PCC6803
<400> 33
   cctttaaatc ggtttctata gttacactca ttggcttttg cctgcaaagc aatatttcct 60
   gataccccta gggtaaatca tgggaaatgg cgatcgccgg agtttctcct gtttgctgga 120
   gggctgtctg caacatcttg gtgctgacca cggaatcggt ggcgaggtta aagaggggat 180
   tagccagaat acctgccagc gaggtagcaa ccaaagtagc gacaatgccc acctgtaggg 240
   gacgcatgcc gggtaaattc catttgatgg ccgggtaatt tttgattact tcggacattt 300
   cctggggctc cttcaccacc atcattttca ccacccggat gtagtagtag atggaaacta 360
   cactggtaac cagaccaagt aggactaggc catacaatcc cgattgccaa ccggcccaga 420
   agatgtaaat tttgccgaaa aagcccgcca gaggaggaat gccccccaag gataataaac 480
   aaatgctcaa gcccaaggtt aacaaggggt ctttgtggta cagaccagcg taatcactaa 540
   tttggtcact gccagtgcgg agggtgaaga gaataatgca actaaacgcc cccaggttca 600
   taaacagata gatgagcatg tagaaaacca tgctggcgta accatcttca ctgccggcca 660
   ctaggccaat catcacaaag cctgcttgac cgatggaaga gtaggccaac atccgtttca 720
   tgctggtttg ggctaaagcc accacgttgc ccagcaccat gctcaacacg gccagagcgg 780
   tgaaaataac gtgccactca tcggtaatac caccaaaggc agtc 824
<210> 34
   <211> 31
   <212> DNA
   <213> Artificial Sequence
<220>
   <223> Primer NB393
<400> 34
   ttctagatat ctgcaggcct aagctttatg c 31
<210> 35
   <211> 34
   <212> DNA
   <213> Artificial Sequence
<220>
   <223> Primer NB394
<400> 35
   tttttttcct ccttagtgtg aaattgttat ccgc 34
<210> 36
   <211> 6414
   <212> DNA
   <213> Artificial Sequence
<220>
   <223> pSGI-NB158 vector
<400> 36
   cctgaggctg aaatgagctg ttgacaatta atcatccggc tcgtataatg tgtggaattg 60
   tgagcggata acaatttcac actaaggagg aaaaaaacca tggcgaacgg tagcgctgtc 120
   tctctgaaga gcggctcctt gaatacgcaa gaggacactt cttcttcccc accgccacgc 180
   gcgttcatca accaattacc cgactggtcc atgttattga cggcgattac cactgtcttt 240
   gttgccgcag agaaacagtg gactatgtta gaccgcaaga gcaagcgctc cgatatgtta 300
   gtggattctt ttggcatgga acgcattgtg caggatggct tagtgtttcg tcaatctttt 360
   agcattcgtt cttatgaaat cggtgcagat cgtcgtgcat ccattgaaac cttaatgaac 420
   catctgcagg aaactagctt gaatcattgc aaatccattc gcttgttgaa tgagggtttt 480
   ggtcgcaccc ccgagatgtg caaacgtgac ttgatctggg tggttacccg catgcacatc 540
   atggtcaacc gctaccctac ctggggtgat accgttgaga ttaacacttg ggtttcccaa 600
   agcggcaaga atggtatggg tcgtgattgg ctgatttccg actgtaatac cggcgaaatc 660
   ctgatccgcg cgacgtctgc atgggcgatg atgaaccaaa agacccgtcg tctgtctaaa 720
   ctgccttacg aagtcagcca agagattgct ccgcacttcg tcgacagccc tcccgtgatc 780
   gaggacggcg accgtaagtt acacaagttc gatgtgaaaa ccggcgacag catccgtaaa 840
   ggtttgactc cgcgttggaa tgacttagat gttaatcagc acgttaacaa cgttaagtat 900
   atcggctgga tcttagagag catgccgacc gaggtcttgg aaactcatga actgtgtttc 960
   ttaactctgg agtatcgtcg cgagtgcggt cgcgatagcg tgctggaatc tgtgaccgcg 1020
   atggatcctt ctaatgaagg tggtcgctcc cactaccagc atttactgcg cttggaggac 1080
   ggtactgaca tcgttaaggg ccgcactgag tggcgtccaa agaatgcccg gaatattggt 1140
   gccattagta ccggtaaaac cagtaatggt aatcccgcca gttaataacc tttcaagctt 1200
   cagatcaatt cgcgctaact tacattaatt gcgttgcgct cactgcccgc tttccagtcg 1260
   ggaaacctgt cgtgccagct gcattaatga atcggccaac gcgcggggag aggcggtttg 1320
   cgtattgggc gccagggtgg tttttctttt caccagtgag acgggcaaca gctgattgcc 1380
   cttcaccgcc tggccctgag agagttgcag caagcggtcc acgctggttt gccccagcag 1440
   gcgaaaatcc tgtttgatgg tggttaacgg cgggatataa catgagctgt cttcggtatc 1500
   gtcgtatccc actaccgaga tatccgcacc aacgcgcagc ccggactcgg taatggcgcg 1560
   cattgcgccc agcgccatct gatcgttggc aaccagcatc gcagtgggaa cgatgccctc 1620
   attcagcatt tgcatggttt gttgaaaacc ggacatggca ctccagtcgc cttcccgttc 1680
   cgctatcggc tgaatttgat tgcgagtgag atatttatgc cagccagcca gacgcagacg 1740
   cgccgagaca gaacttaatg ggcccgctaa cagcgcgatt tgctggtgac ccaatgcgac 1800
   cagatgctcc acgcccagtc gcgtaccgtc ttcatgggag aaaataatac tgttgatggg 1860
   tgtctggtca gagacatcaa gaaataacgc cggaacatta gtgcaggcag cttccacagc 1920
   aatggcatcc tggtcatcca gcggatagtt aatgatcagc ccactgacgc gttgcgcgag 1980
   aagattgtgc accgccgctt tacaggcttc gacgccgctt cgttctacca tcgacaccac 2040
   cacgctggca cccagttgat cggcgcgaga tttaatcgcc gcgacaattt gcgacggcgc 2100
   gtgcagggcc agactggagg tggcaacgcc aatcagcaac gactgtttgc ccgccagttg 2160
   ttgtgccacg cggttgggaa tgtaattcag ctccgccatc gccgcttcca ctttttcccg 2220
   cgttttcgca gaaacgtggc tggcctggtt caccacgcgg gaaacggtct gataagagac 2280
   accggcatac tctgcgacat cgtataacgt tactggtttc acattcacca ccctgaattg 2340
   actctcttcc gggcgctatc atgccatacc gcgaaaggtt ttgcaccatt cgatggtgtc 2400
   aacgtaaatg catgccgctt cgccttcgcg caagcttaga agggcgaatt ccggaccaca 2460
   agataaaaat atatcatcat gaacaataaa actgtctgct tacataaaca gtaatacaag 2520
   gggtgttatg agccatattc aacgggaaac gtcttgctcg aggccgcgat taaattccaa 2580
   catggatgct gatttatatg ggtataaatg ggctcgcgat aatgtcgggc aatcaggtgc 2640
   gacaatctat cgattgtatg ggaagcccga tgcgccagag ttgtttctga aacatggcaa 2700
   aggtagcgtt gccaatgatg ttacagatga gatggtcaga ctaaactggc tgacggaatt 2760
   tatgcctctt ccgaccatca agcattttat ccgtactcct gatgatgcat ggttactcac 2820
   cactgcgatc cccgggaaaa cagcattcca ggtattagaa gaatatcctg attcaggtga 2880
   aaatattgtt gatgcgctgg cagtgttcct gcgccggttg cattcgattc ctgtttgtaa 2940
   ttgtcctttt aacagcgatc gcgtatttcg tctcgctcag gcgcaatcac gaatgaataa 3000
   cggtttggtt gatgcgagtg attttgatga cgagcgtaat ggctggcctg ttgaacaagt 3060
   ctggaaagaa atgcataagc ttttgccatt ctcaccggat tcagtcgtca ctcatggtga 3120
   tttctcactt gataacctta tttttgacga ggggaaatta ataggttgta ttgatgttgg 3180
   acgagtcgga atcgcagacc gataccagga tcttgccatc ctatggaact gcctcggtga 3240
   gttttctcct tcattacaga aacggctttt tcaaaaatat ggtattgata atcctgatat 3300
   gaataaattg cagtttcatt tgatgctcga tgagtttttc taatcagaat tggttaattg 3360
   gttgtaacac tggcagagca ttacgctgac ttgacgggac ggcggcccgg tatggatggc 3420
   accgatgcgg aatcccaaca gattgccttt gacaacaatg tggcctggaa taacctgggg 3480
   gatttgtcca ccaccaccca acgggcctac acttcggcta ttagcacaga cacagtgcag 3540
   agtgtttatg gcgttaatct ggaaaaaaac gataacattc ccattgtttt tgcgtggccc 3600
   atttttccca ccacccttaa tcccacagat tttcaggtaa tgcttaacac gggggaaatt 3660
   gtcaccccgg tgatcgcctc tttgattccc aacagtgaat acaacgaacg gcaaacggta 3720
   gtaattacgg gcaattttgg taatcgttta accccaggca cggagggagc gatttatccc 3780
   gtttccgtag gcacagtgtt ggacagtact cctttggaaa tggtgggacc caacggcccg 3840
   gtcagtgcgg tgggtattac cattgatagt ctcaacccct acgtggccgg caatggtccc 3900
   aaaattgtcg ccgctaagtt agaccgcttc agtgacctgg gggaaggggc tcccctctgg 3960
   ttagccacca atcaaaataa cagtggcggg gatttatatg gagaccaagc ccaatttcgt 4020
   ttgcgaattt acaccagcgc cggtttttcc cccgatggca ttgccagttt actacccaca 4080
   gaatttgaac ggtattttca actccaagcg gaagatatta cgggacggac agttatccta 4140
   acccaaactg gtgttgatta tgaaattccc ggctttggtc tggtgcaggt gttggggctg 4200
   gcggatttgg ccggggttca ggacagctat gacctgactt acatcgaaga tcatgacaac 4260
   tattacgaca ttatcctcaa aggggacgaa gccgcagttc gccaaattaa gagggttgct 4320
   ttgccctccg aaggggatta ttcggcggtt tataatcccg gtggccccgg caatgatcca 4380
   gagaatggtc ccccatgttc agctactgac ggggtggtgc gtaacggcaa aagcaccgcc 4440
   ggacatcagc gctagcggag tgtatactgg cttactatgt tggcactgat gagggtgtca 4500
   gtgaagtgct tcatgtggca ggagaaaaaa ggctgcaccg gtgcgtcagc agaatatgtg 4560
   atacaggata tattccgctt cctcgctcac tgactcgcta cgctcggtcg ttcgactgcg 4620
   gcgagcggaa atggcttacg aacggggcgg agatttcctg gaagatgcca ggaagatact 4680
   taacagggaa gtgagagggc cgcggcaaag ccgtttttcc ataggctccg cccccctgac 4740
   aagcatcacg aaatctgacg ctcaaatcag tggtggcgaa acccgacagg actataaaga 4800
   taccaggcgt ttccccctgg cggctccctc gtgcgctctc ctgttcctgc ctttcggttt 4860
   accggtgtca ttccgctgtt atggccgcgt ttgtctcatt ccacgcctga cactcagttc 4920
   cgggtaggca gttcgctcca agctggactg tatgcacgaa ccccccgttc agtccgaccg 4980
   ctgcgcctta tccggtaact atcgtcttga gtccaacccg gaaagacatg caaaagcacc 5040
   actggcagca gccactggta attgatttag aggagttagt cttgaagtca tgcgccggtt 5100
   aaggctaaac tgaaaggaca agttttggtg actgcgctcc tccaagccag ttacctcggt 5160
   tcaaagagtt ggtagctcag agaaccttcg aaaaaccgcc ctgcaaggcg gttttttcgt 5220
   tttcagagca agagattacg cgcagaccaa aacgatctca agaagatcat cttattaatc 5280
   agataaaata tttctagatt tcagtgcaat ttatctcttc aaatgtagca cctgaagtca 5340
   gccccatacg atataagttg taattctcat gtttgacagc ttatcatcga taagctttaa 5400
   tgcggtagtt tatcacagtt aaattattgc tgaagcggaa tccctggtta atgccgccgc 5460
   cgatgccaat tgcattctcc aagtggggca cattgaacgc ttcaacccgg catttttaga 5520
   gctaaccaaa attctcaaaa cggaagagtt attggcgatc gaagcccatc gcatgagtcc 5580
   ctattcccag cgggccaatg atgtctccgt ggtattggat ttgatgatcc atgacattga 5640
   cctgttgctg gaattggtgg gttcggaagt ggttaaactg tccgccagtg gcagtcgggc 5700
   ttctgggtca ggatatttgg attatgtcac cgctacgtta ggcttctcct ccggcattgt 5760
   ggccaccctc accgccagta aggtcaccca tcgtaaaatt cgttccatcg ccgcccactg 5820
   caaaaattcc ctcaccgaag cggattttct caataacgaa attttgatcc atcgccaaac 5880
   caccgctgat tggagcgcgg actatggcca ggtattgtat cgccaggatg gtctaatcga 5940
   aaaggtttac accagtaata ttgaacctct ccacgctgaa ttagaacatt ttattcattg      6000
   tgttagggga ggtgatcaac cctcagtggg gggagaacag gccctcaagg ccctgaagtt      6060
   agccagttta attgaagaaa tggccctgga cagtcaggaa tggcatgggg gggaagttgt 6120
   gacagaatat caagatgcca ccctggccct cagtgcgagt gtttaaatca acttaattaa 6180
   tgcaattatt gcgagttcaa actcgataac tttgtgaaat attactgttg aattaatcta 6240
   tgactattca atacaccccc ctagccgatc gcctgttggc ctacctcgcc gccgatcgcc 6300
   taaatctcag cgccaagagt agttccctca acaccagtat tctgctcagc agtgacctat 6360
   tcaatcagga agggggaatt gtaacagcca actatggctt tgatggttat atgg            6414
<210> 37
   <211> 37
   <212> DNA
   <213> Artificial Sequence
<220>
   <223> Primer NB470
<400> 37
   agagaatggt cccccatgtt cagctactga cggggtg 37
<210> 38
   <211> 56
   <212> DNA
   <213> Artificial Sequence
<220>
   <223> Primer NB471
<400> 38
   ggattccgct tcagcaataa tttaactgtg ataaactacc gcattaaagc ttatcg 56
<210> 39
   <211> 57
   <212> DNA
   <213> Artificial Sequence
<220>
   <223> Primer NB466
<400> 39
   gggcgaattc cggaccacaa gataaaaata tatcatcatg aacaataaaa ctgtctg 57
<210> 40
   <211> 34
   <212> DNA
   <213> Artificial Sequence
<220>
   <223> Primer NB467
<400> 40
   ggtgccatcc ataccgggcc gccgtcccgt caag 34
<210> 41
   <211> 989
   <212> DNA
   <213> Synechocystis PCC6803
<400> 41
   attgctgaag cggaatccct ggttaatgcc gccgccgatg ccaattgcat tctccaagtg       60
   gggcacattg aacgcttcaa cccggcattt ttagagctaa ccaaaattct caaaacggaa      120
   gagttattgg cgatcgaagc ccatcgcatg agtccctatt cccagcgggc caatgatgtc      180
   tccgtggtat tggatttgat gatccatgac attgacctgt tgctggaatt ggtgggttcg      240
   gaagtggtta aactgtccgc cagtggcagt cgggcttctg ggtcaggata tttggattat      300
   gtcaccgcta cgttaggctt ctcctccggc attgtggcca ccctcaccgc cagtaaggtc      360
   acccatcgta aaattcgttc catcgccgcc cactgcaaaa attccctcac cgaagcggat      420
   tttctcaata acgaaatttt gatccatcgc caaaccaccg ctgattggag cgcggactat      480
   ggccaggtat tgtatcgcca ggatggtcta atcgaaaagg tttacaccag taatattgaa      540
   cctctccacg ctgaattaga acattttatt cattgtgtta ggggaggtga tcaaccctca      600
   gtggggggag aacaggccct caaggccctg aagttagcca gtttaattga agaaatggcc      660
   ctggacagtc aggaatggca tgggggggaa gttgtgacag aatatcaaga tgccaccctg      720
   gccctcagtg cgagtgttta aatcaactta attaatgcaa ttattgcgag ttcaaactcg      780
   ataactttgt gaaatattac tgttgaatta atctatgact attcaataca cccccctagc      840
   cgatcgcctg ttggcctacc tcgccgccga tcgcctaaat ctcagcgcca agagtagttc      900
   cctcaacacc agtattctgc tcagcagtga cctattcaat caggaagggg gaattgtaac      960
   agccaactat ggctttgatg gttatatgg                                        989
<210> 42
   <211> 989
   <212> DNA
   <213> Synechocystis PCC6803
<400> 42
   ccggtatgga tggcaccgat gcggaatccc aacagattgc ctttgacaac aatgtggcct      60
   ggaataacct gggggatttg tccaccacca cccaacgggc ctacacttcg gctattagca     120
   cagacacagt gcagagtgtt tatggcgtta atctggaaaa aaacgataac attcccattg     180
   tttttgcgtg gcccattttt cccaccaccc ttaatcccac agattttcag gtaatgctta     240
   acacggggga aattgtcacc ccggtgatcg cctctttgat tcccaacagt gaatacaacg     300
   aacggcaaac ggtagtaatt acgggcaatt ttggtaatcg tttaacccca ggcacggagg     360
   gagcgattta tcccgtttcc gtaggcacag tgttggacag tactcctttg gaaatggtgg     420
   gacccaacgg cccggtcagt gcggtgggta ttaccattga tagtctcaac ccctacgtgg     480
   ccggcaatgg tcccaaaatt gtcgccgcta agttagaccg cttcagtgac ctgggggaag     540
   gggctcccct ctggttagcc accaatcaaa ataacagtgg cggggattta tatggagacc     600
   aagcccaatt tcgtttgcga atttacacca gcgccggttt ttcccccgat ggcattgcca     660
   gtttactacc cacagaattt gaacggtatt ttcaactcca agcggaagat attacgggac     720
   ggacagttat cctaacccaa actggtgttg attatgaaat tcccggcttt ggtctggtgc     780
   aggtgttggg gctggcggat ttggccgggg ttcaggacag ctatgacctg acttacatcg     840
   aagatcatga caactattac gacattatcc tcaaagggga cgaagccgca gttcgccaaa     900
   ttaagagggt tgctttgccc tccgaagggg attattcggc ggtttataat cccggtggcc     960
   ccggcaatga tccagagaat ggtccccca                                      989
<210> 43
   <211> 1089
   <212> DNA
   <213> Cuphea carthagenensis
<400> 43
   atggcgaacg gtagcgctgt ctctctgaag agcggctcct tgaatacgca agaggacact 60
   tcttcttccc caccgccacg cgcgttcatc aaccaattac ccgactggtc catgttattg 120
   acggcgatta ccactgtctt tgttgccgca gagaaacagt ggactatgtt agaccgcaag 180
   agcaagcgct ccgatatgtt agtggattct tttggcatgg aacgcattgt gcaggatggc 240
   ttagtgtttc gtcaatcttt tagcattcgt tcttatgaaa tcggtgcaga tcgtcgtgca 300
   tccattgaaa ccttaatgaa ccatctgcag gaaactagct tgaatcattg caaatccatt 360
   cgcttgttga atgagggttt tggtcgcacc cccgagatgt gcaaacgtga cttgatctgg 420
   gtggttaccc gcatgcacat catggtcaac cgctacccta cctggggtga taccgttgag 480
   attaacactt gggtttccca aagcggcaag aatggtatgg gtcgtgattg gctgatttcc 540
   gactgtaata ccggcgaaat cctgatccgc gcgacgtctg catgggcgat gatgaaccaa 600
   aagacccgtc gtctgtctaa actgccttac gaagtcagcc aagagattgc tccgcacttc 660
   gtcgacagcc ctcccgtgat cgaggacggc gaccgtaagt tacacaagtt cgatgtgaaa 720
   accggcgaca gcatccgtaa aggtttgact ccgcgttgga atgacttaga tgttaatcag 780
   cacgttaaca acgttaagta tatcggctgg atcttagaga gcatgccgac cgaggtcttg 840
   gaaactcatg aactgtgttt cttaactctg gagtatcgtc gcgagtgcgg tcgcgatagc 900
   gtgctggaat ctgtgaccgc gatggatcct tctaatgaag gtggtcgctc ccactaccag 960
   catttactgc gcttggagga cggtactgac atcgttaagg gccgcactga gtggcgtcca 1020
   aagaatgccc ggaatattgg tgccattagt accggtaaaa ccagtaatgg taatcccgcc 1080
   agttaataa 1089
<210> 44
   <211> 361
   <212> PRT
   <213> Cuphea carthagenensis
<400> 44
   Met Ala Asn Gly Ser Ala Val Ser Leu Lys Ser Gly Ser Leu Asn Thr
   1               5                   10                  15
   Gln Glu Asp Thr Ser Ser Ser Pro Pro Pro Arg Ala Phe Ile Asn Gln
               20                  25                  30
   Leu Pro Asp Trp Ser Met Leu Leu Thr Ala Ile Thr Thr Val Phe Val
           35                  40                  45
   Ala Ala Glu Lys Gln Trp Thr Met Leu Asp Arg Lys Ser Lys Arg Ser
       50                  55                  60
   Asp Met Leu Val Asp Ser Phe Gly Met Glu Arg Ile Val Gln Asp Gly
   65                  70                  75                  80
   Leu Val Phe Arg Gln Ser Phe Ser Ile Arg Ser Tyr Glu Ile Gly Ala
                   85                  90                  95
   Asp Arg Arg Ala Ser Ile Glu Thr Leu Met Asn His Leu Gln Glu Thr
               100                 105                 110
   Ser Leu Asn His Cys Lys Ser Ile Arg Leu Leu Asn Glu Gly Phe Gly
           115                 120                 125
   Arg Thr Pro Glu Met Cys Lys Arg Asp Leu Ile Trp Val Val Thr Arg
       130                 135                 140
   Met His Ile Met Val Asn Arg Tyr Pro Thr Trp Gly Asp Thr Val Glu
   145                 150                 155                 160
   Ile Asn Thr Trp Val Ser Gln Ser Gly Lys Asn Gly Met Gly Arg Asp
                   165                 170                 175
   Trp Leu Ile Ser Asp Cys Asn Thr Gly Glu Ile Leu Ile Arg Ala Thr
               180                 185                 190
   Ser Ala Trp Ala Met Met Asn Gln Lys Thr Arg Arg Leu Ser Lys Leu
           195                 200                 205
   Pro Tyr Glu Val Ser Gln Glu Ile Ala Pro His Phe Val Asp Ser Pro
       210                 215                 220
   Pro Val Ile Glu Asp Gly Asp Arg Lys Leu His Lys Phe Asp Val Lys
   225                 230                 235                 240
   Thr Gly Asp Ser Ile Arg Lys Gly Leu Thr Pro Arg Trp Asn Asp Leu
                   245                 250                 255
   Asp Val Asn Gln His Val Asn Asn Val Lys Tyr Ile Gly Trp Ile Leu
               260                 265                 270
   Glu Ser Met Pro Thr Glu Val Leu Glu Thr His Glu Leu Cys Phe Leu
           275                 280                 285
   Thr Leu Glu Tyr Arg Arg Glu Cys Gly Arg Asp Ser Val Leu Glu Ser
       290                 295                 300
   Val Thr Ala Met Asp Pro Ser Asn Glu Gly Gly Arg Ser His Tyr Gln
   305                 310                 315                 320
   His Leu Leu Arg Leu Glu Asp Gly Thr Asp Ile Val Lys Gly Arg Thr
                   325                 330                 335
   Glu Trp Arg Pro Lys Asn Ala Arg Asn Ile Gly Ala Ile Ser Thr Gly
               340                 345                 350
   Lys Thr Ser Asn Gly Asn Pro Ala Ser
           355                 360
<210> 45
   <211> 42
   <212> DNA
   <213> Artificial Sequence
<220>
   <223> Primer NB462
<400> 45
   ctttgatggt tatatggcct gaggctgaaa tgagctgttg ac 42
<210> 46
   <211> 47
   <212> DNA
   <213> Artificial Sequence
<220>
   <223> Primer NB463
<400> 46
   gatctgaagc ttgaaaggtt attaactggc gggattacca ttactgg 47
<210> 47
   <211> 47
   <212> PRT
   <213> Artificial Sequence
<220>
   <223> Primer NB464
<400> 47
   Gly Thr Ala Ala Thr Cys Cys Cys Gly Cys Cys Ala Gly Thr Thr Ala
   1               5                   10                  15
   Ala Thr Ala Ala Cys Cys Thr Thr Thr Cys Ala Ala Gly Cys Thr Thr
               20                  25                  30
   Cys Ala Gly Ala Thr Cys Ala Ala Thr Thr Cys Gly Cys Gly Cys
           35                  40                  45
<210> 48
   <211> 39
   <212> DNA
   <213> Artificial Sequence
<220>
   <223> Primer NB465
<400> 48
   atatttttat cttgtggtcc ggaattcgcc cttctaagc 39
<210> 49
   <211> 57
   <212> DNA
   <213> Artificial Sequence
<220>
   <223> Primer NB466
<400> 49
   gggcgaattc cggaccacaa gataaaaata tatcatcatg aacaataaaa ctgtctg 57
<210> 50
   <211> 34
   <212> DNA
   <213> Artificial Sequence
<220>
   <223> Primer NB467
<400> 50
   ggtgccatcc ataccgggcc gccgtcccgt caag 34
<210> 51
   <211> 6663
   <212> DNA
   <213> Artificial Sequence
<220>
   <223> pSGI-NB19 vector
<400> 51
   cccccgtgct atgactagcg gcgatcgcca taccggccac gaccatttgc attggatccc 60
   caacggcggc cacaacttcc atggcattga gatgcgggga atgatgttct agactctgac 120
   gcaccaaagc caatttttgt tgatggttgc aatggggatg actactgttc actttgcccc 180
   cagcgtcaat gcctagacct agcagtaccc ccagggctgt ggtagtgccc cccaccacgc 240
   attcgcttag cactaagtaa ctttcggcat gttcctgggc taactgtgcg ccccactgca 300
   aaccctgctg aaaaagatgc tccaccaggg ccaacggtaa cgcttgccct gtggaaagac 360
   agcgggcggg ttgtccgtct agattgatga ctggcaccgc tgggggaatg ggtaaaccag 420
   agttaaataa ataaaccgga gtatggaggg catccaccaa cgctttggtg atgaacactg 480
   gggaaacccc agaaatgagg ggaggtaagg gataggttgc ccctgccgta gttcccttga 540
   ttaaaaattc cgcatcggcg atcgccgtca attttcgatc agcgggggtt ttacccgccg 600
   cagaaatgcc cggaattaaa ccagtttccg taaagcccaa cacacagaca aacaccggtg 660
   gacagtggcc atggcgctca atccaggata aagcttggtc agactgggta taaactgtca 720
   acatatttct gcaagagtgg gcccatgcac ccctatttgt ttatttttct aaatacattc 780
   aaatatgtat ccgctcatga gacaataacc ctgataaatg cttcaataat acgaggagga 840
   aaaaaaaatg cgctcacgca actggtccag aaccttgacc gaacgcagcg gtggtaacgg 900
   cgcagtggcg gttttcatgg cttgttatga ctgttttttt gtacagtcta tgcctcgggc 960
   atccaagcag caagcgcgtt acgccgtggg tcgatgtttg atgttatgga gcagcaacga 1020
   tgttacgcag cagggcagtc gccctaaaac aaagttaggt ggctcaagta tgggcatcat 1080
   tcgcacatgt aggctcggcc ctgaccaagt caaatccatg cgggctgctc ttgatctttt 1140
   cggtcgtgag ttcggtgacg tagccaccta ctcccaacat cagccggact ccgattacct 1200
   cgggaacttg ctccgtagta agacattcat cgcgcttgct gccttcgacc aagaagcggt 1260
   tgttggcgct ctcgcggctt acgttctgcc caagtttgag cagccgcgta gtgagatcta 1320
   tatctatgat ctcgcagtct ccggcgagca ccggaggcag ggcattgcca ccgcgctcat 1380
   caatctcctc aagcatgagg ccaacgcgct tggtgcttat gtgatctacg tgcaagcaga 1440
   ttacggtgac gatcccgcag tggctctcta tacaaagttg ggcatacggg aagaagtgat 1500
   gcactttgat atcgacccaa gtaccgccac ctaagcagag cttacgctga gcgcgcagat 1560
   cagttggaag aatttgtcca ctacgtgaaa ggcgagatca ccaaggtagt cggcaaataa 1620
   tgtctaacaa ttcgttcaag ccgacgccgc ttcgcggcgc ggcttaactc aagcgttaga 1680
   tgcactaagc acataattgc tcacagccaa actatcaggt caagtctgct tttattattt 1740
   ttaagcgtgc ataataagcc ctacacaaat tgggagatat atcatgaaag gctggctttt 1800
   tcttgttatc gcaatagttg gcgaagtaat cgcaacatcc gcattaaaat ctagcgaggg 1860
   ctttactaag ctgatccggt ggatgacctt ttgaatgacc tttaatagat tatattacta 1920
   attaattggg gaccctagag gtcccctttt ttattttaaa aattttttca caaaacggtt 1980
   tacaagcata aagcttaggc cgtttaaacc aattgcggga agaaattaca gcttttgccg 2040
   ctggcctaca gagtttagga gttacccccc atcaacacct ggccattttc gccgacaaca 2100
   gcccccggtg gtttatcgcc gatcaaggca gtatgttggc tggagccgtc aacgccgtcc 2160
   gttctgccca agcagagcgc caggaattac tctacatcct agaagacagc aacagccgta 2220
   ctttaatcgc agaaaatcgg caaaccctaa gcaaattggc cctagatggc gaaaccattg 2280
   acctgaaact aatcatcctc ctcaccgatg aagaagtggc agaggacagc gccattcccc 2340
   aatataactt tgcccaggtc atggccctag gggccggcaa aatccccact cccgttcccc 2400
   gccaggaaga agatttagcc accctgatct acacctccgg caccacagga caacccaaag 2460
   gggtgatgct cagccacggt aatttattgc accaagtacg ggaattggat tcggtgatta 2520
   ttccccgccc cggcgatcag gtgttgagca ttttgccctg ttggcactcc ctagaaagaa 2580
   gcgccgaata ttttcttctt tcccggggct gcacgatgaa ctacaccagc attcgccatt 2640
   tcaaggggga tgtgaaggac attaaacccc atcacattgt cggtgtgccc cggctgtggg 2700
   aatccctcta cgaaggggta caaaaaacgt tccgagggcg aattctgcag atatccatca 2760
   cactggcggc cgctcgagca tgcatctaga gggcccaatt cgccctatag tgagtcgtat 2820
   tacaattcac tggccgtcgt tttacaacgt cgtgactggg aaaaccctgg cgttacccaa 2880
   cttaatcgcc ttgcagcaca tccccctttc gccagctggc gtaatagcga agaggcccgc 2940
   accgatcgcc cttcccaaca gttgcgcagc ctgaatggcg aatggacgcg ccctgtagcg 3000
   gcgcattaag cgcggcgggt gtggtggtta cgcgcagcgt gaccgctaca cttgccagcg 3060
   ccctagcgcc cgctcctttc gctttcttcc cttcctttct cgccacgttc gccggctttc 3120
   cccgtcaagc tctaaatcgg gggctccctt tagggttccg atttagtgct ttacggcacc 3180
   tcgaccccaa aaaacttgat tagggtgatg gttcacgtag tgggccatcg ccctgataga 3240
   cggtttttcg ccctttgacg ttggagtcca cgttctttaa tagtggactc ttgttccaaa 3300
   ctggaacaac actcaaccct atctcggtct attcttttga tttataaggg attttgccga 3360
   tttcggccta ttggttaaaa aatgagctga tttaacaaaa atttaacgcg aattttaaca 3420
   aaattcaggg cgcaagggct gctaaaggaa gcggaacacg tagaaagcca gtccgcagaa 3480
   acggtgctga ccccggatga atgtcagcta ctgggctatc tggacaaggg aaaacgcaag 3540
   cgcaaagaga aagcaggtag cttgcagtgg gcttacatgg cgatagctag actgggcggt 3600
   tttatggaca gcaagcgaac cggaattgcc agctggggcg ccctctggta aggttgggaa 3660
   gccctgcaaa gtaaactgga tggctttctt gccgccaagg atctgatggc gcaggggatc 3720
   aagatctgat caagagacag gatgaggatc gtttcgcatg attgaacaag atggattgca 3780
   cgcaggttct ccggccgctt gggtggagag gctattcggc tatgactggg cacaacagac 3840
   aatcggctgc tctgatgccg ccgtgttccg gctgtcagcg caggggcgcc cggttctttt 3900
   tgtcaagacc gacctgtccg gtgccctgaa tgaactgcag gacgaggcag cgcggctatc 3960
   gtggctggcc acgacgggcg ttccttgcgc agctgtgctc gacgttgtca ctgaagcggg 4020
   aagggactgg ctgctattgg gcgaagtgcc ggggcaggat ctcctgtcat cccaccttgc 4080
   tcctgccgag aaagtatcca tcatggctga tgcaatgcgg cggctgcata cgcttgatcc 4140
   ggctacctgc ccattcgacc accaagcgaa acatcgcatc gagcgagcac gtactcggat 4200
   ggaagccggt cttgtcgatc aggatgatct ggacgaagag catcaggggc tcgcgccagc 4260
   cgaactgttc gccaggctca aggcgcgcat gcccgacggc gaggatctcg tcgtgaccca 4320
   tggcgatgcc tgcttgccga atatcatggt ggaaaatggc cgcttttctg gattcatcga 4380
   ctgtggccgg ctgggtgtgg cggaccgcta tcaggacata gcgttggcta cccgtgatat 4440
   tgctgaagag cttggcggcg aatgggctga ccgcttcctc gtgctttacg gtatcgccgc 4500
   tcccgattcg cagcgcatcg ccttctatcg ccttcttgac gagttcttct gaattgaaaa 4560
   aggaagagta tgagtattca acatttccgt gtcgccctta ttcccttttt tgcggcattt 4620
   tgccttcctg tttttgctca cccagaaacg ctggtgaaag taaaagatgc tgaagatcag 4680
   ttgggtgcac gagtgggtta catcgaactg gatctcaaca gcggtaagat ccttgagagt 4740
   tttcgccccg aagaacgttt tccaatgatg agcactttta aagttctgct atgtggcgcg 4800
   gtattatccc gtattgacgc cgggcaagag caactcggtc gccgcataca ctattctcag 4860
   aatgacttgg ttgagtactc accagtcaca gaaaagcatc ttacggatgg catgacagta 4920
   agagaattat gcagtgctgc cataaccatg agtgataaca ctgcggccaa cttacttctg 4980
   acaacgatcg gaggaccgaa ggagctaacc gcttttttgc acaacatggg ggatcatgta 5040
   actcgccttg atcgttggga accggagctg aatgaagcca taccaaacga cgagcgtgac 5100
   accacgatgc ctgtagcaat ggcaacaacg ttgcgcaaac tattaactgg cgaactactt 5160
   actctagctt cccggcaaca attaatagac tggatggagg cggataaagt tgcaggacca 5220
   cttctgcgct cggcccttcc ggctggctgg tttattgctg ataaatctgg agccggtgag 5280
   cgtgggtctc gcggtatcat tgcagcactg gggccagatg gtaagccctc ccgtatcgta 5340
   gttatctaca cgacggggag tcaggcaact atggatgaac gaaatagaca gatcgctgag 5400
   ataggtgcct cactgattaa gcattggtaa ctgtcagacc aagtttactc atatatactt 5460
   tagattgatt taaaacttca tttttaattt aaaaggatct aggtgaagat cctttttgat 5520
   aatctcatga ccaaaatccc ttaacgtgag ttttcgttcc actgagcgtc agaccccgta 5580
   gaaaagatca aaggatcttc ttgagatcct ttttttctgc gcgtaatctg ctgcttgcaa 5640
   acaaaaaaac caccgctacc agcggtggtt tgtttgccgg atcaagagct accaactctt 5700
   tttccgaagg taactggctt cagcagagcg cagataccaa atactgttct tctagtgtag 5760
   ccgtagttag gccaccactt caagaactct gtagcaccgc ctacatacct cgctctgcta 5820
   atcctgttac cagtggctgc tgccagtggc gataagtcgt gtcttaccgg gttggactca 5880
   agacgatagt taccggataa ggcgcagcgg tcgggctgaa cggggggttc gtgcacacag 5940
   cccagcttgg agcgaacgac ctacaccgaa ctgagatacc tacagcgtga gctatgagaa 6000
   agcgccacgc ttcccgaagg gagaaaggcg gacaggtatc cggtaagcgg cagggtcgga 6060
   acaggagagc gcacgaggga gcttccaggg ggaaacgcct ggtatcttta tagtcctgtc 6120
   gggtttcgcc acctctgact tgagcgtcga tttttgtgat gctcgtcagg ggggcggagc 6180
   ctatggaaaa acgccagcaa cgcggccttt ttacggttcc tggccttttg ctggcctttt 6240
   gctcacatgt tctttcctgc gttatcccct gattctgtgg ataaccgtat taccgccttt 6300
   gagtgagctg ataccgctcg ccgcagccga acgaccgagc gcagcgagtc agtgagcgag 6360
   gaagcggaag agcgcccaat acgcaaaccg cctctccccg cgcgttggcc gattcattaa 6420
   tgcagctggc acgacaggtt tcccgactgg aaagcgggca gtgagcgcaa cgcaattaat 6480
   gtgagttagc tcactcatta ggcaccccag gctttacact ttatgcttcc ggctcgtatg 6540
   ttgtgtggaa ttgtgagcgg ataacaattt cacacaggaa acagctatga ccatgattac 6600
   gccaagcttg gtaccgagct cggatccact agtaacggcc gccagtgtgc tggaattcgc 6660
   cct 6663
<210> 52
   <211> 25
   <212> DNA
   <213> Artificial Sequence
<220>
   <223> Primer NB1
<400> 52
   ctcgagcccc cgtgctatga ctagc 25
<210> 53
   <211> 28
   <212> DNA
   <213> Artificial Sequence
<220>
   <223> Primer NB9
<400> 53
   ctcgagcccg gaacgttttt tgtacccc 28
<210> 54
   <211> 362
   <212> PRT
   <213> Cuphea decandra
<400> 54
   Met Gly Ile Asn Gly Ser Ser Val Gly Leu Lys Ser Gly Ser Leu Lys
   1               5                   10                  15
   Thr Gln Glu Asp Thr Pro Ser Ser Pro Pro Pro Arg Thr Phe Ile Asn
               20                  25                  30
   Gln Leu Pro Asp Trp Ser Met Leu Leu Ala Ala Ile Thr Thr Val Phe
           35                  40                  45
   Leu Ala Ala Glu Lys Gln Trp Met Met Leu Asp Trp Lys Pro Lys Arg
       50                  55                  60
   Pro Asp Met Leu Val Asp Pro Phe Gly Leu Gly Arg Ile Val Gln Asp
   65                  70                  75                  80
   Gly Leu Val Phe Arg Gln Asn Phe Ser Ile Arg Ser Tyr Glu Ile Gly
                   85                  90                  95
   Ala Asp Arg Thr Ala Ser Ile Glu Thr Leu Met Asn His Leu Gln Glu
               100                 105                 110
   Thr Ala Leu Asn His Val Lys Ser Ala Gly Leu Leu Asn Asp Gly Phe
           115                 120                 125
   Gly Arg Thr Pro Glu Met Tyr Lys Arg Asp Leu Ile Trp Val Val Ala
       130                 135                 140
   Lys Met Gln Val Met Val Asn Arg Tyr Pro Thr Trp Gly Asp Thr Val
   145                 150                 155                 160
   Glu Val Asn Thr Trp Val Ala Lys Ser Gly Lys Asn Gly Met Arg Arg
   165 170 175
   Asp Trp Leu Ile Ser Asp Cys Asn Thr Gly Glu Ile Leu Thr Arg Ala
   180 185 190
   Ser Ser Val Trp Val Met Met Asn Gln Lys Thr Arg Arg Leu Ser Lys
   195 200 205
   Ile Pro Asp Glu Val Arg His Glu Ile Glu Pro His Phe Val Asp Ser
   210 215 220
   Pro Pro Val Ile Glu Asp Asp Asp Arg Lys Leu Pro Lys Leu Asp Glu
   225 230 235 240
   Lys Thr Ala Asp Ser Ile Arg Lys Gly Leu Thr Pro Arg Trp Asn Asp
   245 250 255
   Leu Asp Val Asn Gln His Val Asn Asn Val Lys Tyr Ile Gly Trp Ile
   260 265 270
   Leu Glu Ser Thr Pro Gln Glu Val Leu Glu Thr Gln Glu Leu Cys Ser
   275 280 285
   Leu Thr Leu Glu Tyr Arg Arg Glu Cys Gly Arg Asp Ser Val Leu Glu
   290 295 300
   Ser Leu Thr Ala Val Asp His Ser Gly Lys Gly Ser Gly Ser Asn Phe
   305 310 315 320
   Gln His Leu Leu Arg Leu Glu Asp Gly Gly Glu Ile Val Lys Gly Arg
   325 330 335
   Thr Glu Trp Arg Pro Lys Asn Ala Val Ile Asn Gly Ala Val Ala Pro
   340 345 350
   Gly Glu Thr Ser Pro Gly Asn Ser Val Ser
   355 360
<210> 55
   <211> 356
   <212> PRT
   <213> Cuphea paucipetela
<400> 55
   Met Ala Asn Gly Ser Ala Val Ser Leu Lys Asp Gly Ser Leu Glu Thr
   1 5 10 15
   Gln Glu Gly Thr Ser Ser Ser Ser His Pro Pro Arg Thr Phe Ile Asn
               20                  25                  30
   Gln Leu Pro Asp Trp Ser Met Leu Leu Ser Ala Ile Thr Thr Val Phe
           35                  40                  45
   Val Ala Ala Glu Lys Gln Trp Thr Met Leu Asp Arg Lys Ser Lys Arg
       50                  55                  60
   Pro Asp Met Leu Val Glu Pro Phe Val Gln Asp Gly Val Ser Phe Arg
   65                  70                  75                  80
   Gln Ser Phe Ser Ile Arg Ser Tyr Glu Ile Gly Ala Asp Arg Thr Ala
                   85                  90                  95
   Ser Ile Glu Thr Leu Met Asn Ile Phe Gln Glu Thr Ser Leu Asn His
               100                 105                 110
   Cys Lys Ser Leu Gly Leu Leu Asn Asp Gly Phe Gly Arg Thr Pro Glu
           115                 120                 125
   Met Cys Lys Arg Asp Leu Ile Trp Val Val Thr Lys Met Gln Ile Glu
       130                 135                 140
   Val Asn Arg Tyr Pro Thr Trp Gly Asp Thr Ile Glu Val Thr Thr Trp
   145                 150                 155                 160
   Val Ser Glu Ser Gly Lys Asn Gly Met Ser Arg Asp Trp Leu Ile Ser
                   165                 170                 175
   Asp Cys His Thr Gly Glu Ile Leu Ile Arg Ala Thr Ser Val Trp Ala
               180                 185                 190
   Met Met Asn Gln Lys Thr Arg Arg Leu Ser Lys Ile Pro Asp Glu Val
           195                 200                 205
   Arg Gln Glu Ile Val Pro Tyr Phe Val Asp Ser Ala Pro Val Ile Glu
       210                 215                 220
   Asp Asp Arg Lys Leu His Lys Leu Asp Val Lys Thr Gly Asp Ser Ile
   225                 230                 235                 240
   Arg Asn Gly Leu Thr Pro Arg Trp Asn Asp Leu Asp Val Asn Gln His
                   245                 250                 255
   Val Asn Asn Val Lys Tyr Ile Gly Trp Ile Leu Lys Ser Val Pro Thr
               260                 265                 270
   Glu Val Phe Val Thr Gln Glu Leu Cys Gly Leu Thr Leu Glu Tyr Arg
           275                 280                 285
   Arg Glu Cys Arg Arg Asp Ser Val Leu Glu Ser Val Thr Ala Met Asp
       290                 295                 300
   Pro Ser Lys Glu Gly Asp Arg Ser Leu Tyr Gln His Leu Leu Arg Leu
   305                 310                 315                 320
   Glu Asn Gly Ala Asp Ile Ala Leu Gly Arg Thr Glu Trp Arg Pro Lys
                   325                 330                 335
   Asn Ala Gly Thr Asn Gly Ala Ile Ser Thr Thr Lys Thr Ser Pro Gly
               340                 345                 350
   Asn Ser Val Ser
           355
<210> 56
   <211> 267
   <212> PRT
   <213> Elaeis oleifera
<400> 56
   Asn Ser Ile Pro Asp Trp Ser Met Leu Leu Ala Ala Val Thr Thr Ile
   1               5                   10                  15
   Phe Leu Ala Ala Glu Lys Gln Trp Thr Leu Leu Asp Trp Lys Pro Arg
               20                  25                  30
   Arg Pro Asp Met Leu Thr Asp Ala Phe Ser Leu Gly Glu Ile Val Gln
           35                  40                  45
   Asp Gly Leu Val Phe Arg Gln Asn Phe Ser Ile Arg Ser Tyr Glu Ile
       50                  55                  60
   Gly Ala Asp Arg Thr Ala Ser Ile Glu Thr Leu Met Asn His Leu Gln
   65                  70                  75                  80
   Glu Thr Val Leu Asn His Val Arg Asn Ala Gly Leu Leu Gly Asp Gly
                   85                  90                  95
   Phe Gly Ala Thr Pro Glu Met Ser Lys Arg Asn Leu Ile Trp Val Val
               100                 105                 110
   Thr Lys Met Gln Val Leu Ile Glu His Tyr Pro Ser Trp Gly Asp Val
               115                 120                 125
   Val Glu Val Asp Thr Trp Val Gly Ala Ser Gly Lys Asn Gly Met Arg
       130                 135                 140
   Arg Asp Trp His Val Arg Asp Tyr Arg Thr Gly Gln Thr Ile Pro Arg
   145                 150                 155                 160
   Ala Thr Ser Ile Trp Val Met Met Asn Lys His Thr Arg Lys Leu Ser
                   165                 170                 175
   Lys Met Pro Glu Glu Val Arg Ala Glu Ile Gly Pro Tyr Phe Met Glu
               180                 185                 190
   His Ala Thr Ile Val Asp Glu Asp Ser Gly Lys Leu Pro Lys Leu Asp
           195                 200                 205
   Asp Asp Thr Ala Asp Tyr Ile Lys Trp Gly Leu Thr Pro Arg Trp Ser
       210                 215                 220
   Asp Leu Asp Val Asn Gln His Val Asn His Val Lys Tyr Ile Gly Trp
   225                 230                 235                 240
   Ile Leu Glu Ser Ala Pro Ile Ser Ile Leu Glu Asn His Asn Trp Ala
                   245                 250                 255
   Ser Leu Ile Leu Asp Thr Gly Arg Asn Trp Asn
               260                 265
<210> 57
   <211> 417
   <212> PRT
   <213> Elaeis guineensis
<400> 57
   Met Val Ala Ser Ile Val Ala Trp Ala Phe Phe Pro Thr Pro Ser Phe
   1               5                   10                  15
   Ser Pro Thr Ala Ser Ala Lys Ala Ser Lys Thr Ile Gly Glu Gly Ser
               20                  25                  30
   Glu Asn Leu Asn Val Arg Gly Ile Ile Ala Lys Pro Thr Ser Ser Ser
           35                  40                  45
   Ala Ala Lys Gln Gly Lys Val Met Ala Gln Ala Val Pro Lys Ile Asn
       50                  55                  60
   Gly Ala Lys Val Gly Leu Lys Ala Glu Ser Gln Lys Ala Glu Glu Asp
   65                  70                  75                  80
   Ala Ala Pro Ser Ser Ala Pro Arg Thr Phe Tyr Asn Gln Leu Pro Asp
                   85                  90                  95
   Trp Ser Val Leu Leu Ala Ala Val Thr Thr Ile Phe Leu Ala Ala Glu
               100                 105                 110
   Lys Gln Trp Thr Leu Leu Asp Trp Lys Pro Arg Arg Pro Asp Met Leu
           115                 120                 125
   Thr Gly Ala Phe Ser Leu Gly Lys Ile Val Gln Asp Gly Leu Val Phe
       130                 135                 140
   Arg Gln Asn Phe Ser Ile Arg Ser Tyr Glu Ile Gly Ala Asp Arg Thr
   145                 150                 155                 160
   Ala Ser Ile Glu Thr Leu Met Asn His Leu Gln Glu Thr Ala Leu Asn
                   165                 170                 175
   His Val Arg Asn Ala Gly Leu Leu Gly Asp Gly Phe Gly Ala Thr Pro
               180                 185                 190
   Glu Met Ser Lys Arg Asn Leu Ile Trp Val Val Thr Lys Met Gln Val
           195                 200                 205
   Leu Ile Glu His Tyr Pro Ser Trp Gly Asp Val Val Glu Val Asp Thr
       210                 215                 220
   Trp Val Gly Ala Ser Gly Lys Asn Gly Met Arg Arg Asp Trp His Val
   225                 230                 235                 240
   Arg Asp Tyr Arg Thr Gly Gln Thr Ile Leu Arg Ala Thr Ser Ile Trp
                   245                 250                 255
   Val Met Met Asp Lys His Thr Arg Lys Leu Ser Lys Met Pro Glu Glu
               260                 265                 270
   Val Arg Ala Glu Ile Gly Pro Tyr Phe Met Glu His Ala Ala Ile Val
           275                 280                 285
   Asp Glu Asp Ser Arg Lys Leu Pro Lys Leu Asp Asp Asp Thr Ala Asp
       290                 295                 300
   Tyr Ile Lys Trp Gly Leu Thr Pro Arg Trp Ser Asp Leu Asp Val Asn
   305                 310                 315                 320
   Gln His Val Asn Asn Val Lys Tyr Ile Gly Trp Ile Leu Glu Ser Ala
                   325                 330                 335
   Pro Ile Ser Ile Leu Glu Asn His Glu Leu Ala Ser Met Thr Leu Glu
               340                 345                 350
   Tyr Arg Arg Glu Cys Gly Arg Asp Ser Val Leu Gln Ser Leu Thr Ala
           355                 360                 365
   Val Ala Asn Asp Cys Thr Gly Gly Leu Pro Glu Ala Ser Ile Glu Cys
       370                 375                 380
   Gln His Leu Leu Gln Leu Glu Cys Gly Ala Glu Ile Val Arg Gly Arg
   385                 390                 395                 400
   Thr Gln Trp Arg Pro Arg Arg Ala Ser Gly Pro Thr Ser Ala Gly Ser
                   405                 410                 415
   Ala